# EUROPÄISCHE PATENTSCHRIFT

(11) **EP 1 618 101 B1**
(45) Veröffentlichungstag und Bekanntmachung des Hinweises auf die Patenterteilung: **20.12.2017**
(21) Anmeldenummer: 04705793.0
(22) Anmeldetag: 28.01.2004
(51) Int. Cl.: C07D 311/80, C09K 19/34

(54) **BENZOCHROMENDERIVATE**
BENZOCHROMENE DERIVATIVES
DERIVES DE BENZOCHROMENE

(30) Priorität: 25.02.2003 DE 10308266
(43) Veröffentlichungstag der Anmeldung: 25.01.2006
(73) Patentinhaber: Merck Patent GmbH, 64293 Darmstadt (DE)
(72) Erfinder: TAUGERBECK, Andreas, 64285 Darmstadt (DE); KLASEN-MEMMER, Melanie, 67259 Heuchelheim (DE)
(86) Internationale Anmeldenummer: PCT/EP2004/000731
(87) Internationale Veröffentlichungsnummer: WO 2004/076438

(56) Entgegenhaltungen:
- FR-E- 95 364
- US-A- 5 648 021
- FEDOROV A Y ET AL: "Aryllead triacetates in the synthesis of oxaphenanthrene derivatives" TETRAHEDRON LETTERS, ELSEVIER SCIENCE PUBLISHERS, AMSTERDAM, NL, Bd. 42, Nr. 34, 20. August 2001 (2001-08-20), Seiten 5875-5877, XP004295946 ISSN: 0040-4039
- HARROWVEN D C ET AL: "A new cascade radical reaction for the synthesis of biaryls and triaryls from benzyl iodoaryl ethers" TETRAHEDRON LETTERS, ELSEVIER SCIENCE PUBLISHERS, AMSTERDAM, NL, Bd. 42, Nr. 5, 29. Januar 2001 (2001-01-29), Seiten 961-964, XP004314727 ISSN: 0040-4039
- NEUMEISTER, JOACHIM ET AL: "Abnormal products in the exhaustive ozone oxidation of phenanthrene in hydrochloric acid/methanol" CHEMISCHE BERICHTE , 117(4), 1640-2 CODEN: CHBEAM; ISSN: 0009-2940, 1984, XP001194528
- SHISHIDO, KOZO ET AL: "The competition between electrocyclic reaction and [1,5]sigmatropic reaction in the thermolysis of 1,1-disubstituted benzocyclobutenes" CHEMISTRY LETTERS , (11), 1943-6 CODEN: CMLTAG; ISSN: 0366-7022, 1984, XP001194529
- PATENT ABSTRACTS OF JAPAN Bd. 2000, Nr. 16, 8. Mai 2001 (2001-05-08) -& JP 2001 026587 A (CHISSO CORP), 30. Januar 2001 (2001-01-30) in der Anmeldung erwähnt
- PATENT ABSTRACTS OF JAPAN Bd. 1998, Nr. 14, 31. Dezember 1998 (1998-12-31) -& JP 10 236992 A (CHISSO CORP), 8. September 1998 (1998-09-08)

## Beschreibung

Die vorliegende Erfindung betrifft Benzochromenderivate, bevorzugt mesogene Benzochromenderivate, insbesondere flüssigkristalline Benzochromenderivate, sowie diese Benzochromenderivate enthaltende flüssigkristalline Medien. Ferner betrifft die vorliegende Erfindung Flüssigkristallanzeigen, insbesondere mittels einer aktiven Matrix angesteuerte Flüssigkristallanzeigen (AMDs oder AM LCDs nach Englisch "active matrix addressed liquid crystal displays") und ganz insbesonders sogenannte VAN (Englisch "vertically aligned nematics") Flüssigkristallanzeigen, einer Ausführungsform von ECB (von Englisch "electrically controlled birefringence") Flüssigkristallanzeigen, bei denen nematische Flüssigkristalle mit einer negativen dielektrischen Anisotropie (Δε) verwendet werden.

In derartigen Flüssigkristallanzeigen werden die Flüssigkristalle als Dielektrika verwendet, deren optische Eigenschaften sich bei Anlegen einer elektrischen Spannung reversibel ändern. Elektrooptische Anzeigen die Flüssigkristalle als Medien verwenden sind dem Fachmann bekannt. Diese Flüssigkristallanzeigen verwenden verschiedene elektrooptische Effekte. Die gebräuchlichsten hiervon sind der TN-Effekt (Englisch "twisted nematic"), mit einer homogenen, nahezu planaren Ausgangsorientierung des Flüssigkristalldirektors und einer um ca. 90° verdrillten nematischen Struktur), der STN-Effekt (Englisch "super-twisted nematic") und der SBE-Effekt (Englisch "supertwisted birefringence effect" mit einer 180° oder mehr verdrillten nematischen Struktur). Bei diesen und ähnlichen elektrooptischen Effekten werden flüssigkristalline Medien mit positiver dielektrischer Anisotropie (Δε) verwendet.

Neben den genannten elektrooptischen Effekten, welche Flüssigkristallmedien mit positiver dielektrischer Anisotropie benötigen, gibt es andere elektrooptische Effekte welche Flüssigkristallmedien mit negativer dielektrischer Anisotropie verwenden, wie z.B. der ECB-Effekt und seine Unterformen DAP (Englisch "deformation of aligned phases"), VAN und CSH (Englisch "color super homeotropics").

Ein elektrooptischer Effekt mit hervorragender, kleiner Blickwinkelabhängigkeit des Kontrasts verwendet axial symmetrische Micropixel (ASM von Englisch "axially symmetric micro pixel"). Bei diesem Effekt ist der Flüssigkristall jedes Pixels zylinderförmig von einem Polymermaterial umgeben. Dieser Mode eignet sich besonders zur Kombination mit der Adressierung durch Plasmakanäle. So lassen sich insbesondere großflächige PA (Englisch "plasma addressed") LCDs mit guter Blickwinkelabhängigkeit des Kontrasts realisieren.

Der in letzter Zeit verstärkt eingesetzte IPS-Effekt (Englisch "in plane switching") kann sowohl dielektrisch positive wie auch dielektrisch negative Flüssigkristallmedien verwenden, ähnlich wie auch "guest/host"-Anzeigen also Gast/Wirt-Anzeigen, die Farbstoffe je nach verwandtem Anzeigemodus entweder in dielektrisch positiven oder in dielektrisch negativen Medien einsetzen können.

Da bei Flüssigkristallanzeigen im allgemeinen, also auch bei Anzeigen nach diesen Effekten, die Betriebsspannung möglichst gering sein soll, werden Flüssigkristallmedien mit einem großen Absolutwert der dielektrischen Anisotropie eingesetzt, die in der Regel überwiegend und meist sogar weitestgehend aus Flüssigkristallverbindungen mit einer dielektrischen Anisotropie mit dem entsprechenden Vorzeichen bestehen. Also, bei dielektrisch positiven Medien aus Verbindungen mit positiver dielektrischer Anisotropie und bei dielektrisch negativen Medien aus Verbindungen mit negativer dielektrischer Anisotropie. Bei den jeweiligen Arten von Medien (dielektrisch positiv bzw. dielektrisch negativ) werden typischer Weise allenfalls nennenswerte Mengen an dielektrisch neutralen Flüssigkristallverbindungen eingesetzt. Flüssigkristallverbindungen mit dem der dielektrischen Anisotropie des Medium entgegengesetzten Vorzeichen der dielektrischen Anisotropie werden in der Regel äußerst sparsam oder gar nicht eingesetzt.

Eine Ausnahme bilden hier flüssigkristalline Medien für MIM-Anzeigen (Englisch "metal insultator metal", Simmons, J.G., Phys. Rev. 155 Nr. 3, S. 657-660 und Niwa, J.G. et al., SID 84 Digest, S. 304-307, Juni 1984) bei denen die Flüssigkristallmedien mit einer aktiven Matrix aus Dünnfilmtransistoren angesteuert werden. Bei dieser Art von Ansteuerung, welche die nicht lineare Kennlinie der Diodenschaltung ausnutzt, kann im Gegensatz zu TFT-Anzeigen kein Speicherkondensator gemeinsam mit den Elektroden der Flüssigkristallanzeigeelemente (Pixeln) aufgeladen werden. Somit ist zur Verminderung des Effekts des Spannungsabfalls während des Ansteuerzyklus ein möglichst großer Grundwert der Dielektrizitätskonstante erforderlich. Bei dielektrisch positiven Medien, wie sie z.B. bei MIM-TN-Anzeigen eingesetzt werden, muß also die Dielektrizitätskonstante senkrecht zur Molekülachse (ε⊥) möglichst groß sein, da sie die Grundkapazität des Pixels bestimmt. Hierzu werden, wie z.B. in WO 93/01253, EP 0 663 502 und DE 195 21 483 beschrieben, in den dielektrisch positiven Flüssigkristallmedien, neben dielektrisch positiven Verbindungen, gleichzeitig auch Verbindungen mit negativer dielektrischer Anisotropie eingesetzt.

Eine weitere Ausnahme bilden STN-Anzeigen in denen z.B. nach DE 41 00 287 dielektrisch positive Flüssigkristallmedien mit dielektrisch negativen Flüssigkristallverbindungen eingesetzt werden um die Steilheit der elektrooptischen Kennlinie zu erhöhen.

Die Bildpunkte der Flüssigkristallanzeigen können direkt angesteuert werden, zeitsequentiell, also im Zeitmultiplexverfahren oder mittels einer Matrix von aktiven Elementen mit nichtlinearen elektrischen Kennlinien angesteuert werden.

Die bislang gebräuchlichsten AMDs verwenden diskrete aktive elektronische Schaltelemente, wie z.B. dreipolige Schaltelemente wie MOS (Englisch "metal oxide silicon") Transistoren oder Dünnfilmtransistoren (TFTs von Englisch "thin film transistors) oder Varistoren oder 2-polige Schaltelemente wie z.B. MIM (Englisch "metal insulator metal") Dioden, Ringdioden oder "back to back"-Dioden. Bei den TFTs werden verschiedene Halbleitermaterialien, überwiegend Silizium, aber auch Cadmiumselenid, verwendet. Insbesondere wird amorphes Silizium oder polykristallines Silizium verwendet.

Gemäß der vorliegenden Anmeldung sind Flüssigkristallanzeigen mit zur Flüssigkristallschicht senkrechtem elektrischen Feld und Flüssigkristallmedien mit negativer dielektrischer Anisotropie (Δε < 0) bevorzugt. Bei diesen Anzeigen ist die Randorientierung der Flüssigkristalle homeotrop. Im voll durchgeschalteten Zustand, also bei Anliegen einer entsprechend großen elektrischen Spannung, ist der Flüssigkristalldirektor parallel zur Schichtebene orientiert.

Cyclische Lactone der Formel 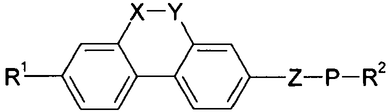 worin
- -X-Y-: -CO-O- oder -O-CO- bedeutet
sind aus JP 2001-026 587 (A) bekannt. Diese Verbindungen sind durch breite smektische Phasen gekennzeichnet und werden in JP 2001-026 587 (A) zur Verwendung in ferroelektrischen Flüssigkristallmischungen vorgeschlagen.

In der JPH10 236992 sind verschiedene in 3,3'-Position verbrückte Tetrafluorbiphenylderivate offengelegt, u.a. beispielsweise Verbindungen der Formel 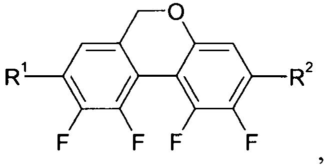 die in allgemeiner Form für die Verwendung in Flüssigkristallmischungen mit negativer dielektrischer Anisotropie vorgeschlagen werden.

Aus U.S.P. 5,648,021 sind fluorierte Phenanthrene der Formel 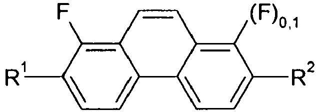 sowie fluorierte 9,10-Dihydrophenanthrene der Formel 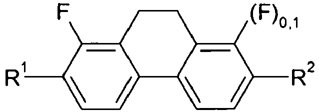 bekannt. Auch diese weisen breite smektische Phasen auf und sie werden ebenfalls zur Verwendung in ferroelektrischen Flüssigkristallmischungen vorgeschlagen.

In DE 100 64 995 werden fluorierte Phenanthrene der Formel 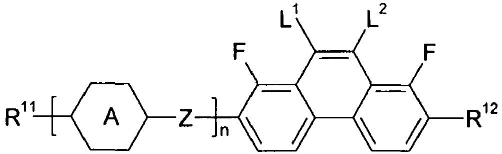 worin
L¹ und L², jeweils unabhängig voneinander, H oder F bedeuten
vorgestellt und zur Verwendung in nematischen Flüssigkristallmischungen insbesondere für ECB-Anzeigen vorgeschlagen. Die Beispielverbindungen bei denen L¹ und L² beide gleich H sind und die eine Alky- Endgruppe und eine Alkoxy-Endgruppe haben, besitzen ein nur schwach negatives Δε, wohingegen die Beispielverbindungen, bei denen L¹ und L² beide gleich F sind und die eine Alkoxy-Endgruppe aufweisen, zwar ein betragsmäßig größeres negatives Δε haben, jedoch in der Regel eine größere Rotationsviskosität haben, deutlich schlechter löslich und zudem meist nicht ausreichend UV-stabil sind.

Somit ist ersichtlich, dass sowohl ein Bedarf an weiteren mesogenen Verbindungen, als auch insbesondere ein Bedarf an Flüssigkristallmedien mit negativer dielektrischer Anisotropie, mit einem großen Absolutwert der dielektrischen Anisotropie, einem der jeweiligen Anwendung entsprechenden Wert der optischen Anisotropie (Δη), einer breiten nematischen Phase, guter Stabilität gegen UV, Wärme und elektrische Spannung und einer niedrigen Rotationsviskosität besteht.

Dies wird erreicht, durch Einsatz der erfindungsgemäßen mesogenen Verbindungen der Formel I-2 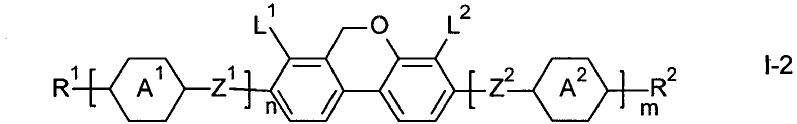 worin
- L¹ und L²: beide F, 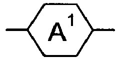
und 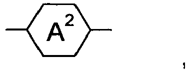 jeweils unabhängig voneinander, und wenn mehrfach vorhanden auch diese unabhängig voneinander,
(a) einen trans-1,4-Cyclohexylenrest, worin auch eine oder zwei nicht benachbarte CH₂-Gruppen durch -O- und/oder -S-ersetzt sein können,
(b) einen 1,4-Cyclohexenylenrest,
(c) einen 1,4-Phenylenrest, worin auch eine oder zwei nicht benachbarte CH-Gruppen durch N ersetzt sein können oder 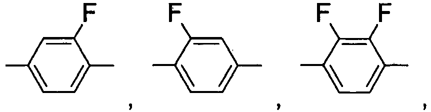 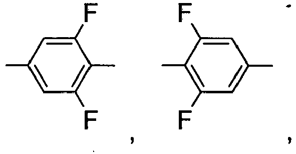 oder
(d) einen Rest ausgewählt aus der Gruppe 1,4-Bicyclo-[2,2,2]-octylen, 1,3-Bicyclo-[1,1,1]-pentylen, Spiro-[3,3]-heptan-2,4-diyl, Piperidin-1,4-diyl, Naphtalin-2,6-diyl, Decahydronaphthalin-2,6-diyl und 1,2,3,4-Tetrahydronaphthalin-2,6-diyl, bevorzugt 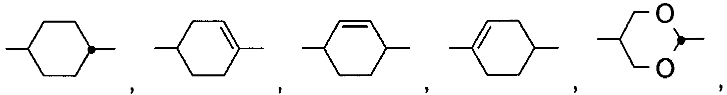 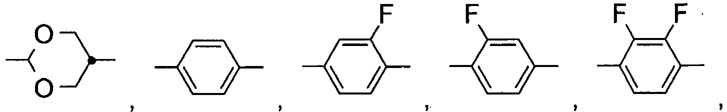 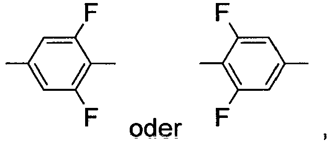
   - R¹ und R²: jeweils unabhängig voneinander, H, Halogen, -CN, -SCN, -SF₅, -CF₃, -CHF₂, -CH₂F, -OCF₃, -OCHF₂, eine einfach durch CN oder CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-, -CF=CH-, -CH=CF-, 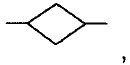 -CO-, -CO-O-, -O-CO- oder -O-CO-O- so ersetzt sein können, dass weder O- noch S-Atome direkt miteinander verknüpft sind,
   oder einer von
   - R¹ und R²: Alkyl und Alkoxy mit 1 bis 12 C-Atomen, Alkoxyalkyl, Alkenyl oder Alkenyloxy mit 2 bis 12 C-Atomen und der andere, unabhängig vom ersten, ebenfalls Alkyl und Alkoxy mit 1 bis 12 C-Atomen, Alkoxyalkyl, Alkenyl oder Alkenyloxy mit 2 bis 12 C-Atomen oder auch F, Cl, Br, -CN, -SCN, -SF₅, -CF₃, -CHF₂, -CH₂F, -OCF₃ oder -OCHF₂
   - Z¹ und Z²: , jeweils unabhängig voneinander, -CH₂-CH₂-, -CF₂-CF₂-, -CF₂-CH₂-,-CH₂-CF₂-, -CH=CH-, -CF=CF-, -CF=CH-, -CH=CF-, -C=C-, -COO-, -OCO-, -CH₂O-, -OCH₂-, -CF₂O-, -OCF₂-, oder eine Kombination von zweien dieser Gruppen, wobei keine zwei O-Atome miteinander verbunden sind, oder eine Einfachbindung,
   - bevorzugt: -(CH₂)₄-, -CH₂-CH₂-, -CF₂-CF₂-, -CH=CH-, -CF=CF-, -C≡C-, -CH₂O-, -CF₂O- oder eine Einfachbindung,
   besonders bevorzugt -CH₂O-, -CH₂-CH₂-, -CF₂-CF₂-, -CF=CF-, -CF₂O- oder eine Einfachbindung und
   - n und m: jeweils 0, 1 oder 2, wobei
   - n + m: 0, 1, 2 oder 3, bevorzugt 0, 1 oder 2, besonders bevorzugt 0 oder 1.
   bedeuten.

Bevorzugt sind Verbindungen der Formel I-2, bei denen
die Summe n + m 0 oder 1, bevorzugt 0, beträgt.

Eine bevorzugte Ausführungsform stellen die Verbindungen der Formel I-2, bei denen die Summe n + m 1 beträgt und bevorzugt
m oder n 1, 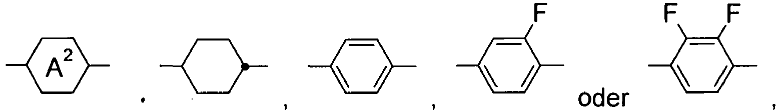
- Z²: bevorzugt -(CH₂)₄-, -CH₂-CH₂-, -CF₂-CF₂-, -CH=CH-, -CF=CF-, -C≡C-, -O-CH₂-, -O-CF₂- oder eine Einfachbindung, besonders bevorzugt -O-CH₂-, -CH₂-CH₂-, -CF₂-CF₂-, -CF=CF-, -O-CF₂- oder eine Einfachbindung
bedeuten und L¹, L², R¹ und R² die oben bei Formel I-2 gegebene Bedeutung haben..

Besonders bevorzugt sind Verbindungen der Formel I-2, bei denen
n und m beide 0 bedeuten und
L¹, L², R¹ und R² die oben bei der entsprechenden Formel gegebene Bedeutung haben.

Verbindungen der Formel I-2 mit verzweigten Flügelgruppen R¹ und/oder R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien. Verbindungen der Formel I mit S_{A}-Phasen eignen sich beispielsweise für thermisch adressierte Displays.

Falls R¹ und/oder R² einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl, bzw. Alkoxyalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R¹ und/oder R² einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ und/oder R² einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy,

Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R¹ und/oder R² einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl; 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R¹ und/oder R² einen einfach durch CN oder CF₃ substituierten Alkyl- oder Alkenylrest bedeutet, so ist dieser Rest vorzugsweise geradkettig. Die Substitution durch CN oder CF₃ ist in beliebiger Position.

Falls R¹ und/oder R² einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verzweigte Gruppen enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R¹ und/oder R² einen Alkylrest darstellt, in dem zwei oder mehr CH₂-Guppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Insbesondere bevorzugt sind Verbindungen der Formel I bei denen n = 0 oder 1 und m = 0 sowie R¹ Methyl, Ethyl, Propyl, Butyl, Pentyl, Vinyl, 1E-Propenyl, 1E-Butenyl oder 1E-Pentenyl bedeutet so wie diese Verbindungen enthaltende Medien. Insbesondere bevorzugt von diesen Verbindungen sind die durch Alkyl substituierten Verbindungen eingesetzt.

Die Verbindungen der Formel I-2 werden gemäß den folgenden Schemata (Schema I bis XX) hergestellt. Geeignete Vorstufen sind beispielsweise Lactone (Schema I).
Diese Lactone können durch intramolekulare Cyclisierung von in geeigneter Weise halogenierten Arylphenylestern durch Kupplungsreaktionen vom Ullmann-Typ hergestellt werden (Houben Weyl, Methoden der Organischen Chemie, New York, 1993). Sie können alternativ durch palladiumkatalysierte intramolekulare Kreuzkopplungen nach Schema II und Schema III erhalten werden.

Alternativ kann die Kreuzkopplung auch in einem ersten Schritt mit den entsprechend geschützten Phenolen und Carbonsäureestern durchgeführt werden, wie in Schema IIIa am Beispiel einer Negishi-Reaktion (Negishi, E. et al., J. Org. Chem. (1977) 42, S. 1821-1823) gezeigt ist. 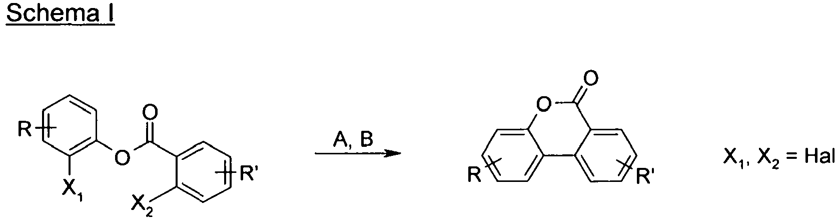 , worin, wie in den Schemata la bis Id, II, III, lila IV bis XX, wenn nicht explizit anders angegeben,
- R und R',: jeweils unabgängig voneinander, die oben bei Formel I für 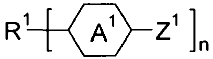 bzw. 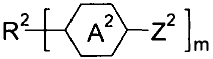 gegebene Bedeutung haben und
- Hal: Halogen, bevorzugt Br oder I bedeutet.

A: Hasan, I. et al., Chem. Rev. (2002), 102, S. 1359-1469,
B: Hennings, D.D. et al., Org. Lett. (1999), 1, S. 1205-1208.

Die Synthese der Estervorstufen erfolgt nach Standardreaktionen (Houben-Weyl) aus kommerziell erhältlichem 4-Brom-3-fluor-1-iodbenzol oder (im Fall R = Methyl aus 4-Brom-2-fluortoluol, oder aus 4-Brom-2-fluorphenol (Fa. ABCR, Karlsruhe, Deutschland)).

Alkylseitenketten werden vorteilhaft durch Sonogashira-Kupplung nach Schema la in die Vorstufen eingeführt.

Vorstufen mit Alkoxyseitenketten werden vorteilhaft gemäß Schema Ib erhalten.

Die beiden nach den Schemata la bzw. Ib erhaltenen Arten von Zwischenstufen können, wie in Schema Ic beispielhaft für die Alkylverbindungen gezeigt, wahlweise zum Phenol oder zur Carbonsäure umgesetzt und anschließend verestert werden.

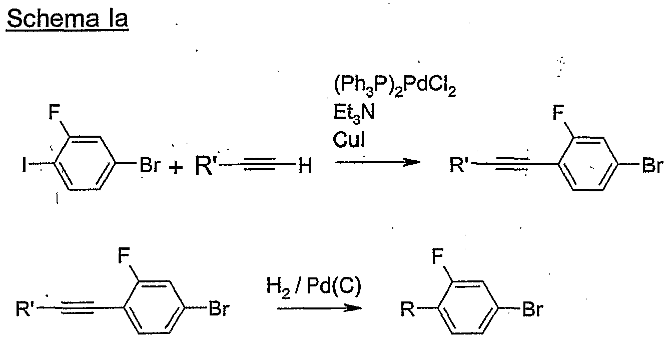

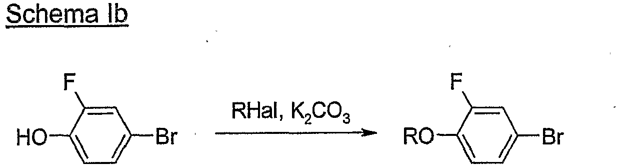

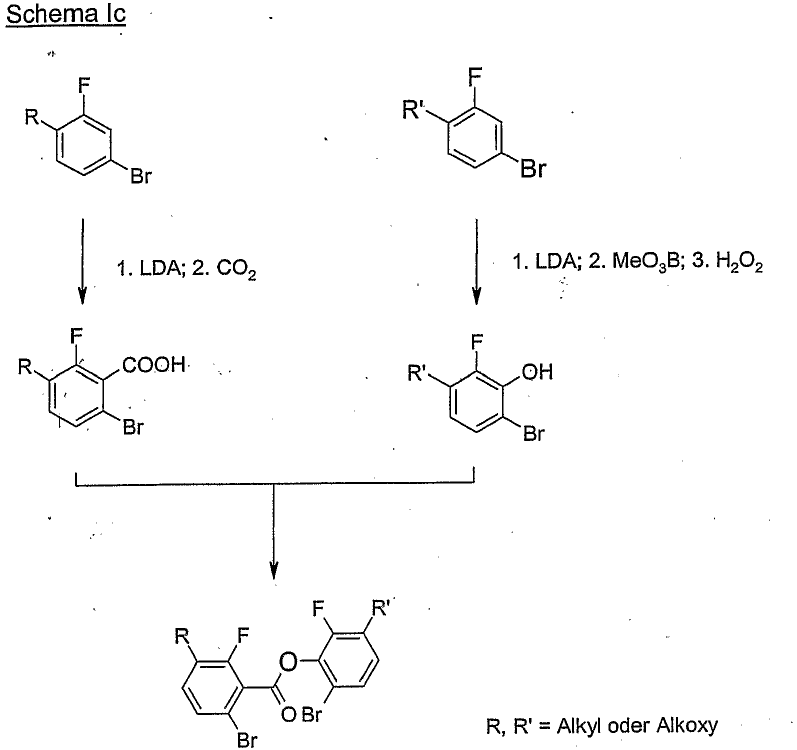

Das Bromphenol kann geschützt und nach Schema Id für nachfolgende Suzuki-Kupplungen zur Boronsäure umgesetzt werden.

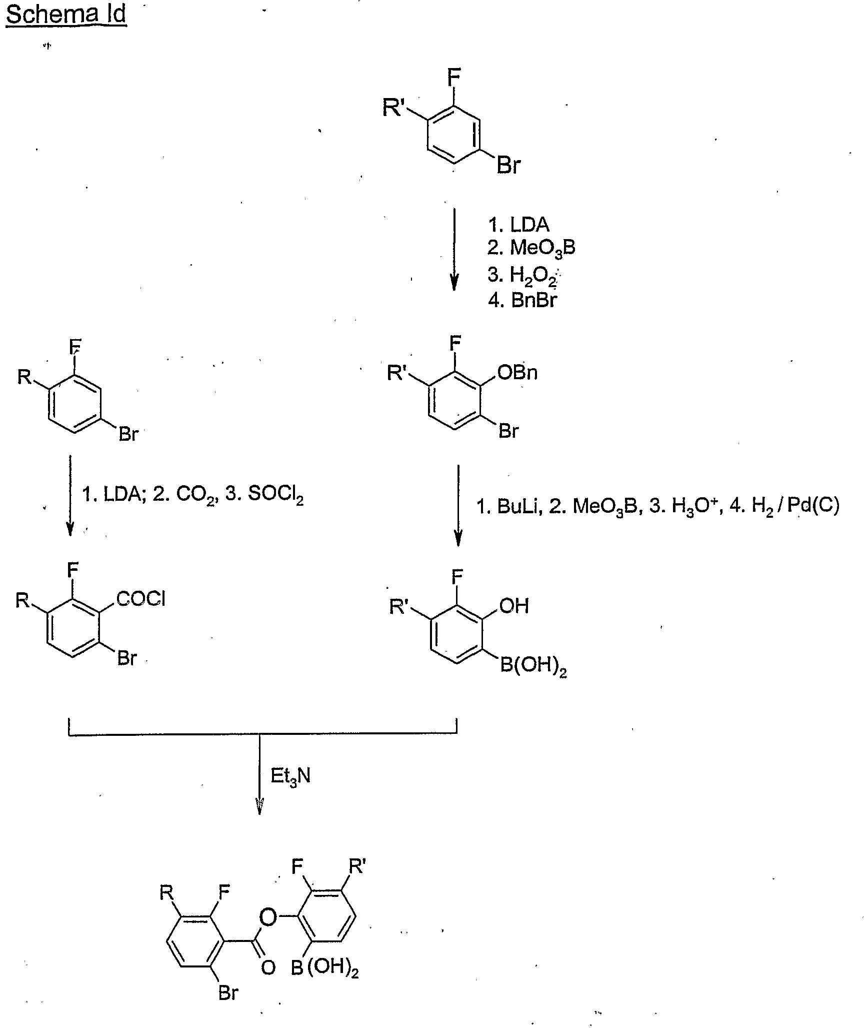

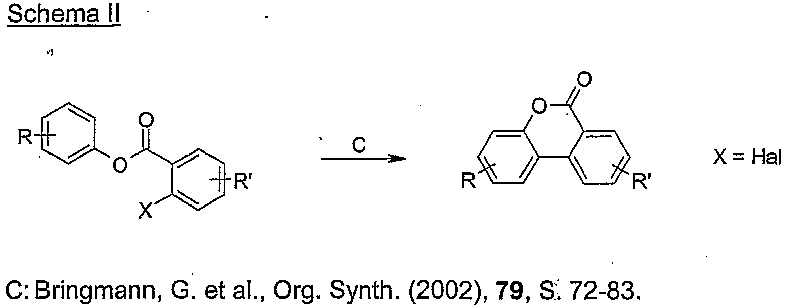

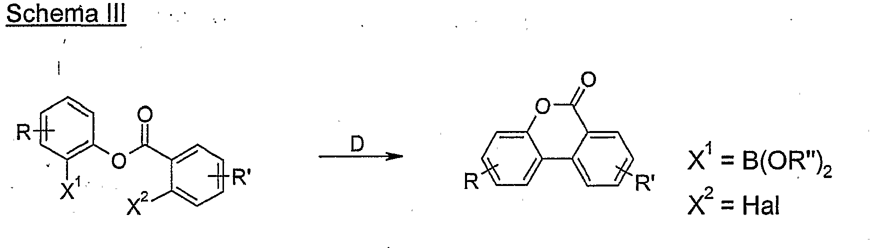

D: Alo, B.I. et al., J. Org. Chem. (1991), 56, S. 3763-3768.

Die Lactone (**1a**) können, wie in Schema IV gezeigt, analog zu EP 1 061 113, in die Benzochromene (**1b**) oder alternativ, mit Lawessons Reagenz und anschließende Reaktion mit DAST, in die Difluorbenzochromene (1c) überführt werden (Bunelle, W.H. et al., J. Org. Chem. (1990), 55, S. 768-770).

Aus den Lactonen (**1a**) können alternativ nach Schema V (Ringom, R. und Bennecke, T., Acta. Chem. Scand. (1999), 53, S. 41-47) durch Reaktion mit LiAlH₄ in THF und anschließende Umsetzung mit DAST die Fluorbenzochromene (**1d**) erhalten werden.

Die bereits erwähnte alternative Synthese bei der die Kreuzkopplung in einem ersten Schritt mit den entsprechend geschützten Phenolen und Carbonsäureestern durchgeführt wird, ist in Schema lila auf S. 16 am Beispiel einer Negishi-Reaktion gezeigt. 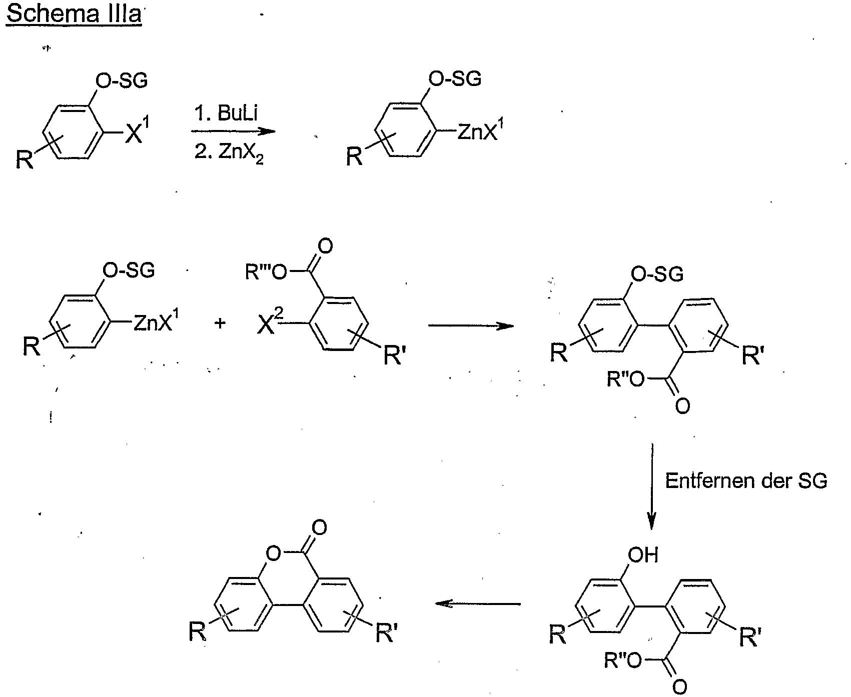 worin
SG eine Schutzgruppe,
X Halogen, bevorzugt Br,
X¹ und X² unabhängig voneinander Halogen,
X¹ bevorzugt Cl, Br, J, beesondes bevorzugt Br oder J, ganz besonders bevorzugt Br,
R" Alkyl, bevorzugt Methyl, bedeutet und
R und R', jeweils unabhängig voneinander, die oben bei Schema I gegebene Bedeutung haben.

Nach Abspaltung der Schutzgruppe können z.B. durch einfaches Erhitzen in einem inerten Lösungsmittel oder durch Behandeln mit Säure oder Base die Lactone erhalten werden.

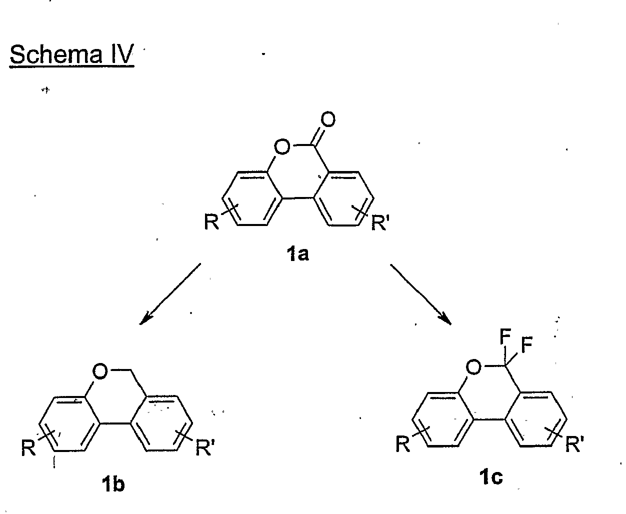

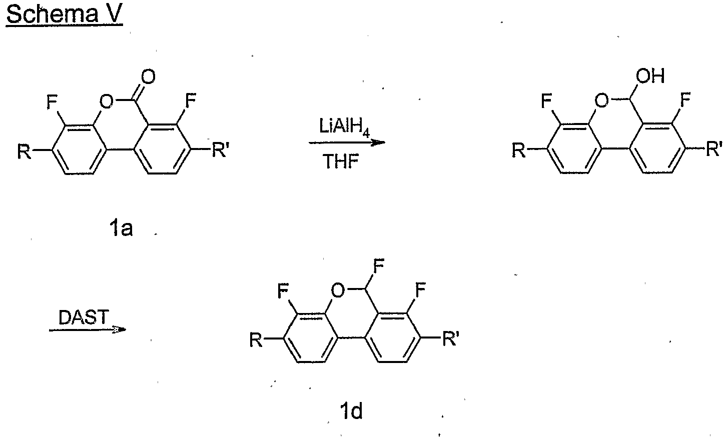

Verbindungen der Formel I in denen 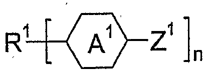 und 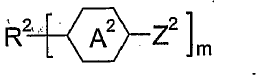 R¹, R², 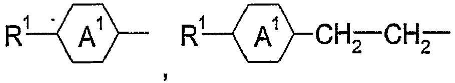 oder 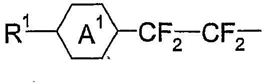 bedeuten, worin die Ringe 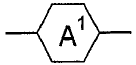 auch Heterocyclen sein können, können nach Schemata VI bis XX erhalten werden.

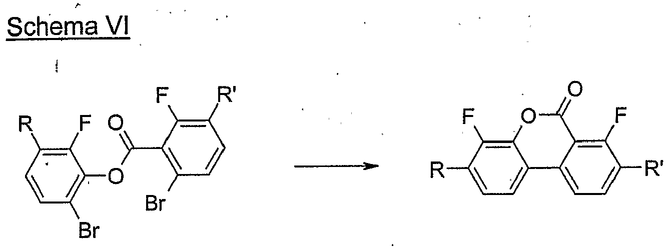

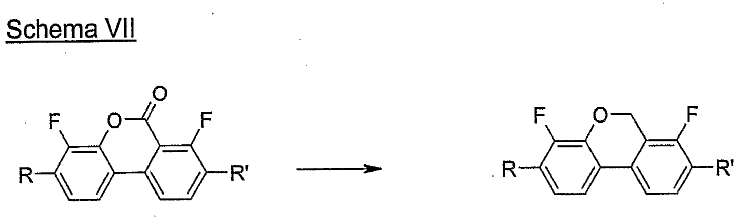

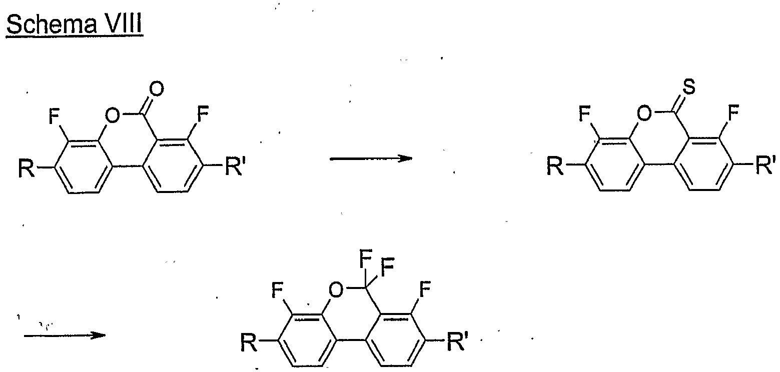

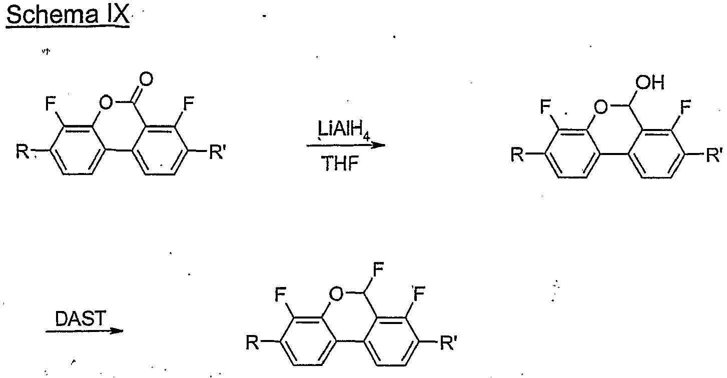

4-Brom-3-fluor-1-iodbenzol und 4-Brom-2-fluorphenol eignen sich ebenfalls als Synthesebausteine für eine Vielzahl von Derivaten, die analog den in den Schemata I-V dargestellten Reaktionsfolgen zu den entsprechenden Zielverbindungen umgesetzt werden können.

Cyclohexylderivate werden beispielsweise nach Schema X erhalten. Metallierung von 4-Brom-3-fluor-1-iodbenzol mit *n*-Butyllithium und Addition an Cyclohexanone liefert Phenylcyclohexanole, aus denen nach Wasserabspaltung und Hydrierung 4-Brom-2-fluor-1-cyclohexylbenzolderivate erhalten werden.

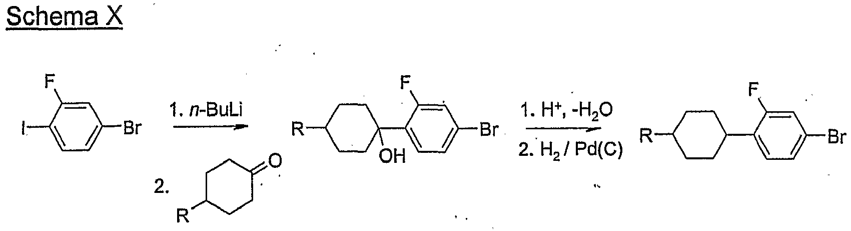

Arylderivate werden beispielsweise nach Schema XI erhalten. Aus 4-Brom-3-fluor-1-iodbenzol lassen sich direkt durch Suzuki-Kupplung mit den entsprechenden Boronsäuren 1-Aryl-4-brom-2-fluorderivate herstellen.

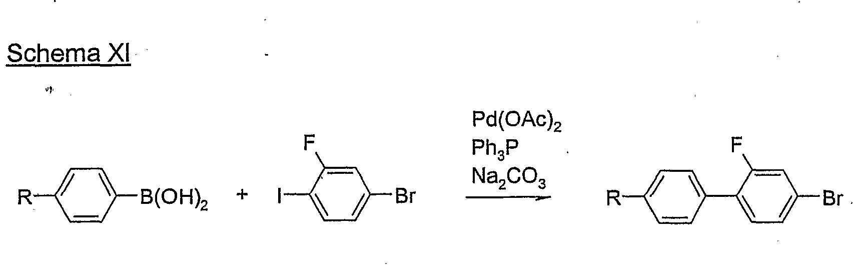

Sonogashirakupplung von 4-Brom-3-fluor-1-iodbenzol mit Phenylacetylenen liefert wie in Schema XII gezeigt 4-Brom-2-fluortolane. Diese können, wie in Schema XIII gezeigt, wahlweise zu ethylenverbrückten Verbindungen hydriert werden oder nach V .O. Rogatchov, V. D. Filimonov, M. S. Yusubov, Synthesis (2001), 7, 1001-1003, zu Diketonen umgesetzt werden, aus denen durch Reaktion mit Schwefeltetrafluorid tetrafluorethylenverbrückte Verbindungen erhalten werden.

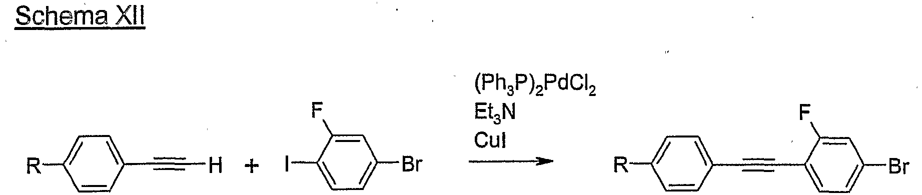

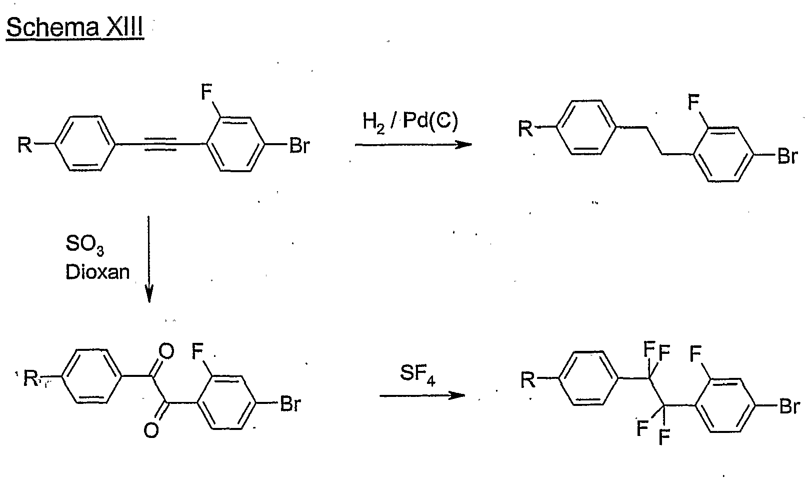

Aus 4-Brom-2-fluorphenol können nach Schema XIV Hydroxyverbindungen und Triflate als Synthesebausteine erhalten werden. Dabei wird 4-Brom-2-fluorphenol mit einer geeigneten Schutzgruppe "SG" (z. B. Benzyl) geschützt, nach entsprechender Umsetzung gemäß Schemata I bis V und antschließender Entfernung der Schutzgruppe (z.B durch Hydrierung für Benzyl als SG), lassen sich Phenole und daraus Triflate (Trifluormethansulfonate, TfO-) erhalten. 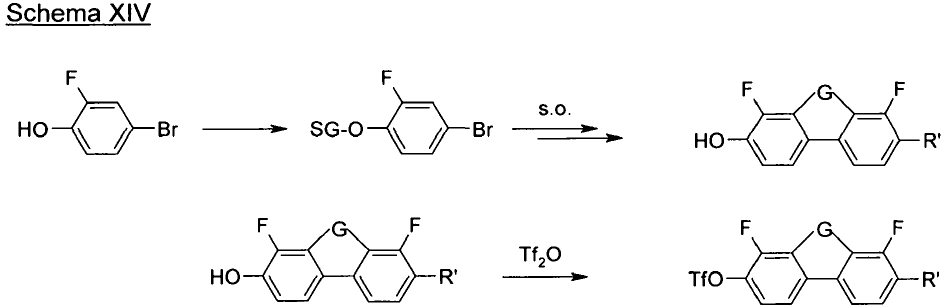 worin, wie in den Schemata XV bis XX,
- G: Y-O oder O-Y bedeutet und
- Y: -CH₂-, C=O oder -CF₂- bedeutet.

Die so erhaltenen Phenole können nach Schema XV zu Estern und Ethern umgesetzt werden. 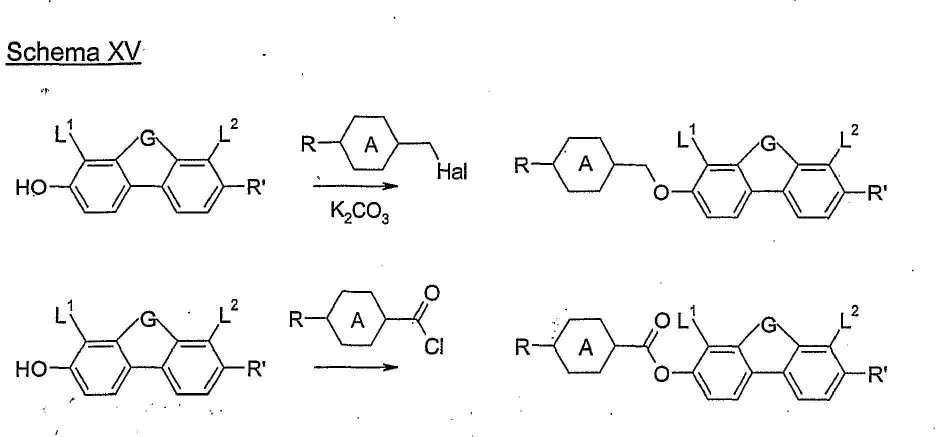 worin R, wie in Schemata XVI bis XX, die bei Formel I für R¹ gegebene Bedeutung besitzt.

CF₂O-verbrückte Verbindungen werden nach WO 02-48 073 und WO 01-64 667, wie in Schema XVI gezeigt, erhalten.

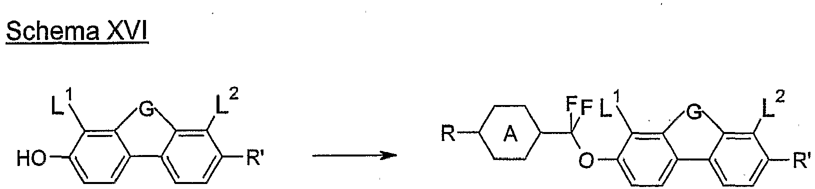

Aus den nach Schema XIV erhaltenen Triflaten können durch Suzuki-Kupplung mit Ethenboronsäuren Stilbene erhalten werden (Schema XVII).

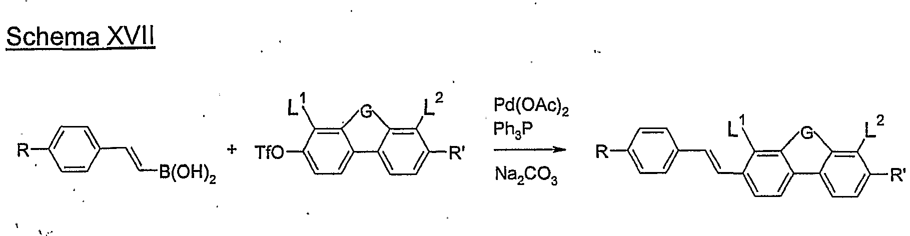

Analog sind durch Stille-Reaktion nach L. Lu, D. J. Burton, Tetrahedron Lett. 1997, 38, 7673-7676, Difluorstilbene zugänglich (Schema XVIII).

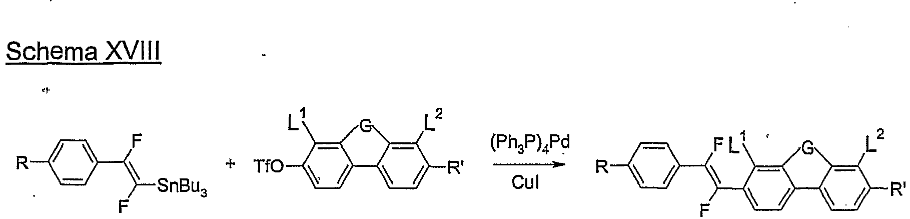

Carbonylierung von Triflaten nach S. Cacchi, P.G. Ciattini, E. Morera, G. Ortar, Tetrahedron Lett. (1986), 27, 3931-3934 liefert Carbonsäureester (Schema XIX). Nach Verseifung zu den entsprechenden Carbonsäuren können daraus durch Umsetzung mit Phenolen z. B. Phenylester erhalten werden.

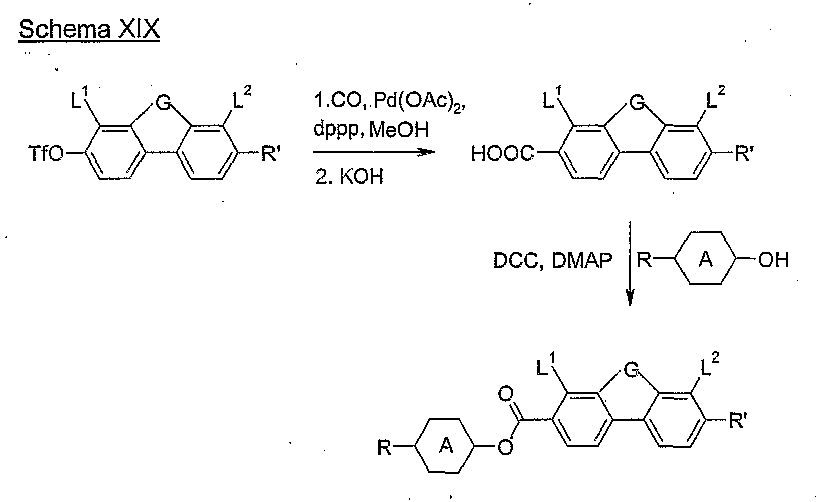

Die nach Schema XIX erhaltenen Carbonsäureester lassen sich nach WO 02-480 73 und WO 01-64 667, wie in Schema XX gezeigt, zu Difluorbenzylethern umsetzen.

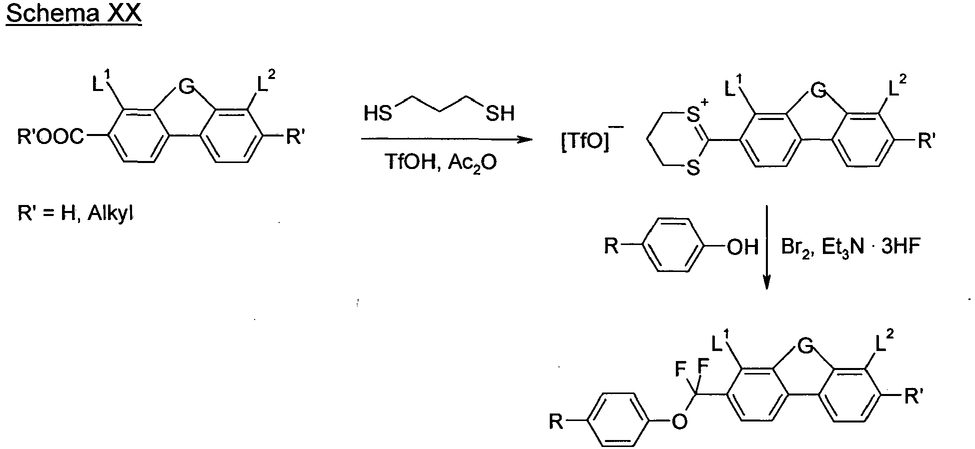

Auf den folgenden Seiten werden Beispiele für Strukturen bevorzugter Verbindungen der Formel I-2 und deren Lacton-Vorstufen gegeben, worin R und R' die jeweilige unter Formel I-2 für R¹ bzw. R² gegebene Bedeutung haben.

Die nachfolgend zunächst aufgeführten Benzo[c]chromen-6-onderivate sind als Lactone geeignete Vorstufen für die Herstellung von Verbindungen der Formel I-2, z.B. gemäß dem oben aufgeführten Schema IV:

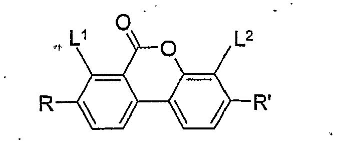

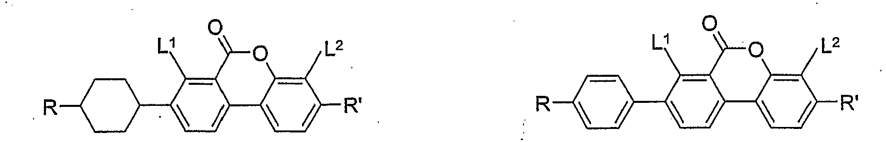

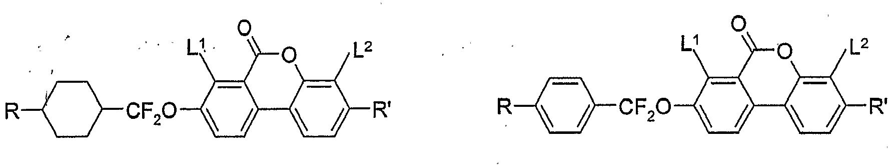

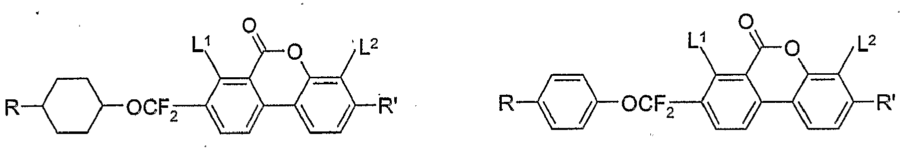

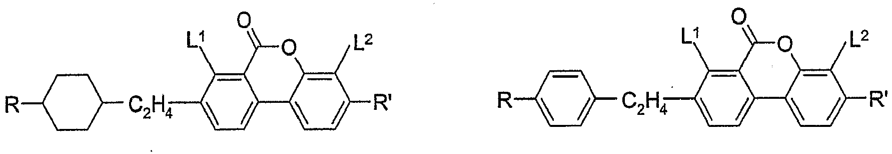

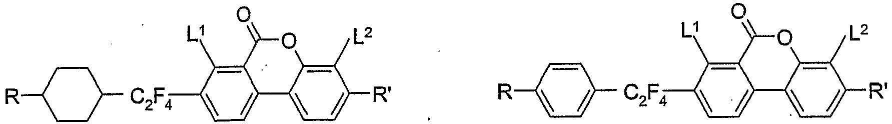

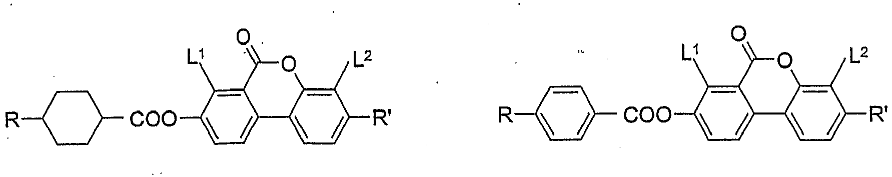

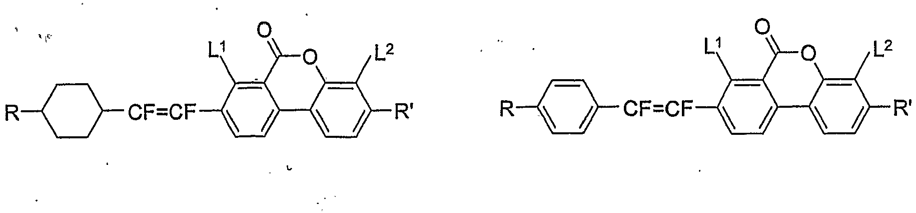

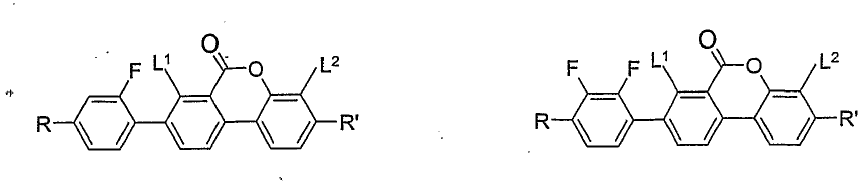

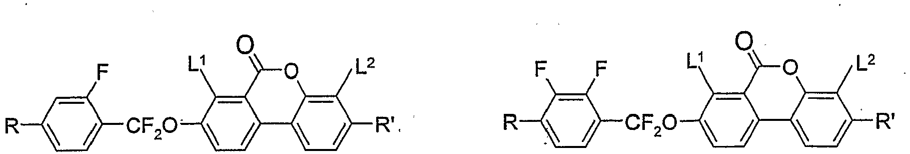

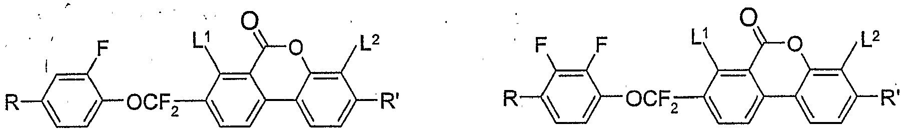

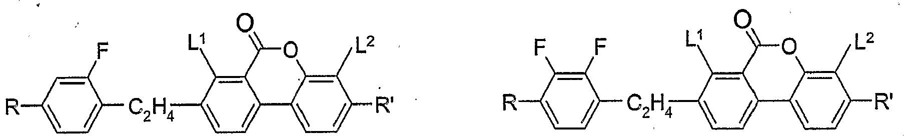

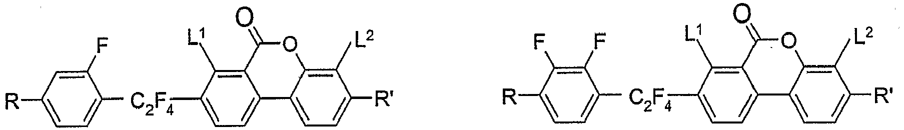

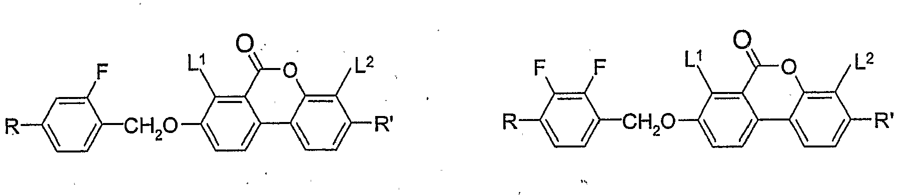

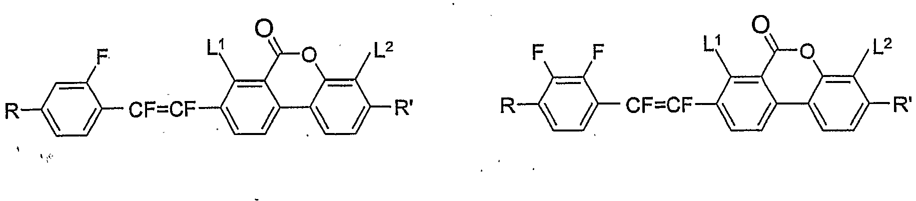

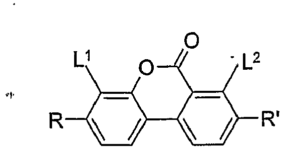

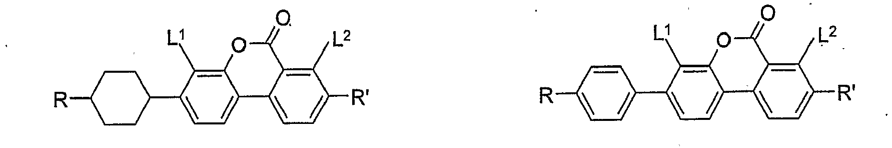

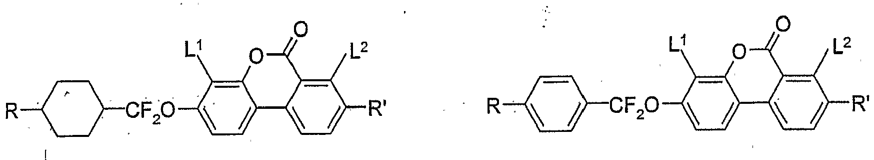

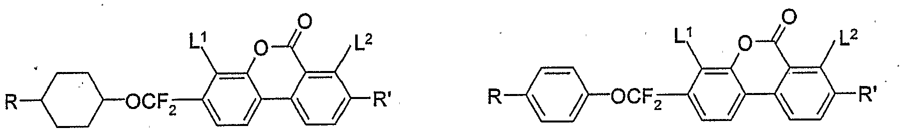

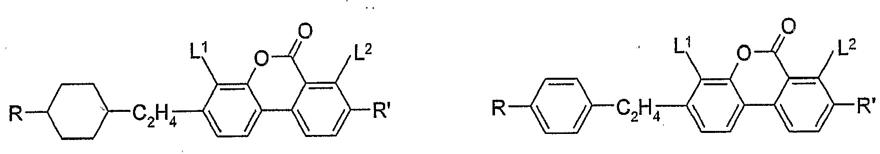

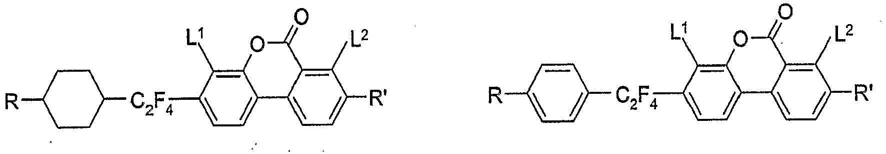

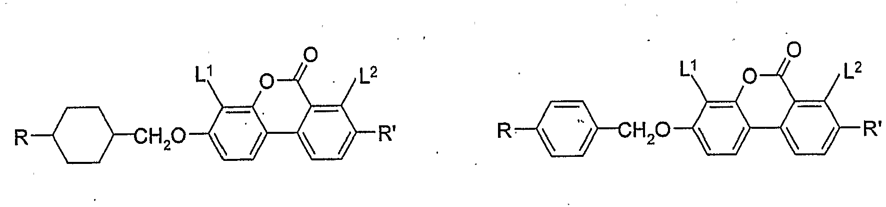

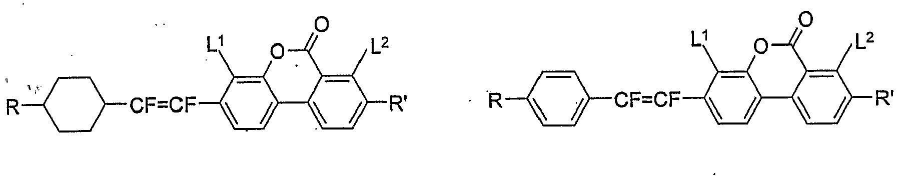

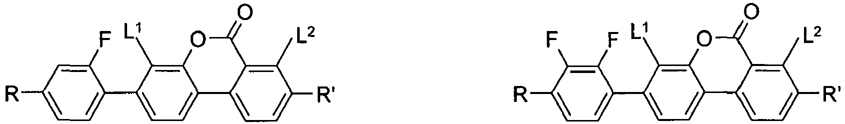

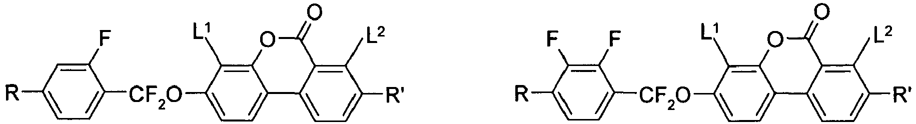

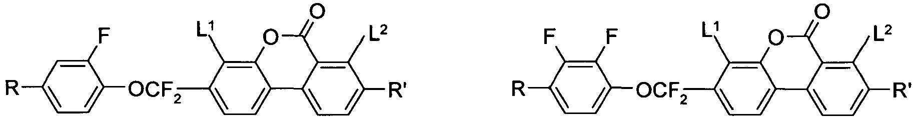

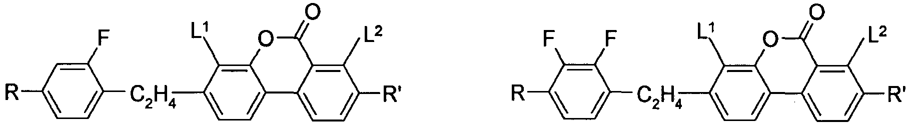

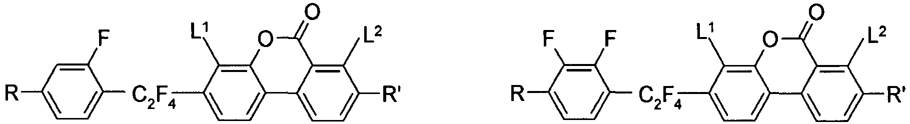

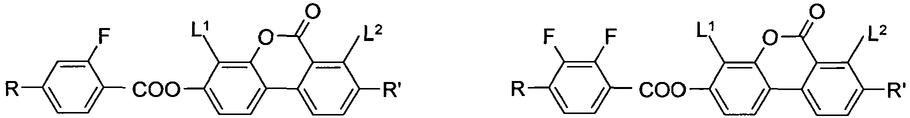

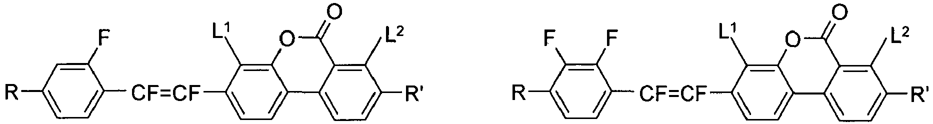

Die nachfolgend aufgeführten Verbindungen sind bevorzugte Verbindungen der Formel I-2:

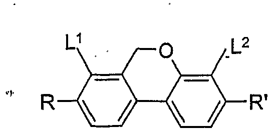

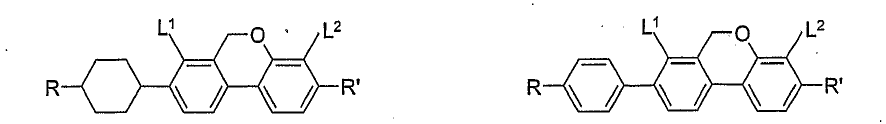

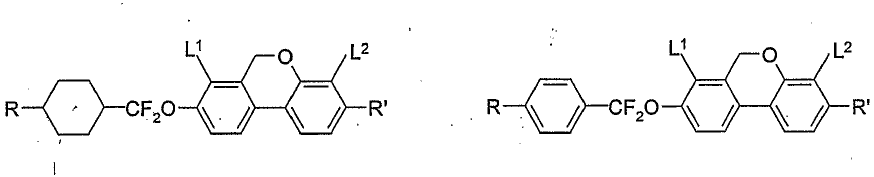

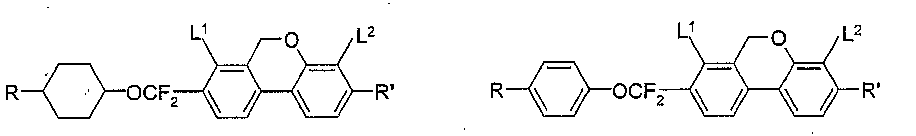

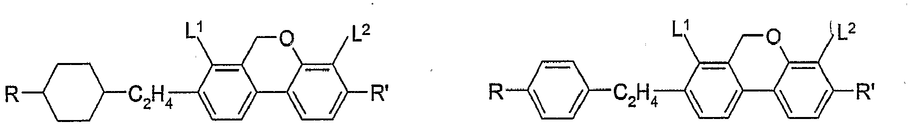

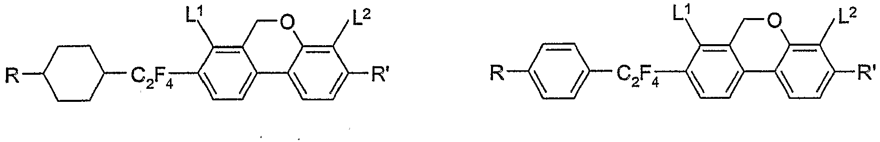

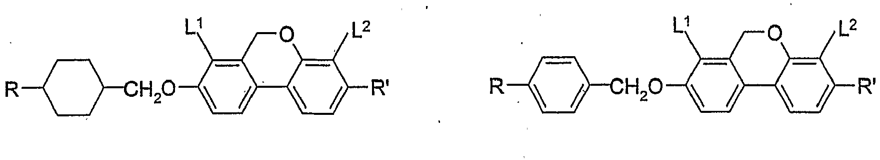

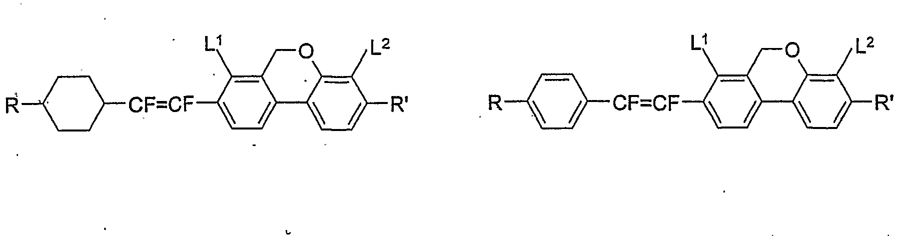

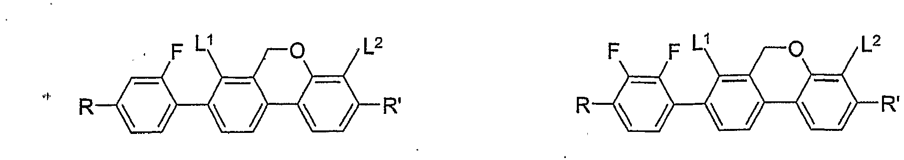

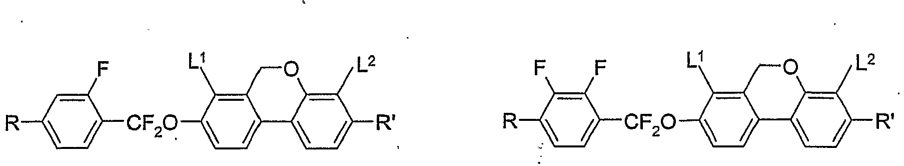

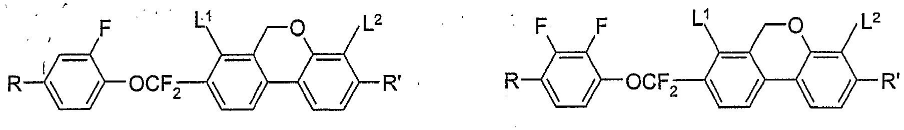

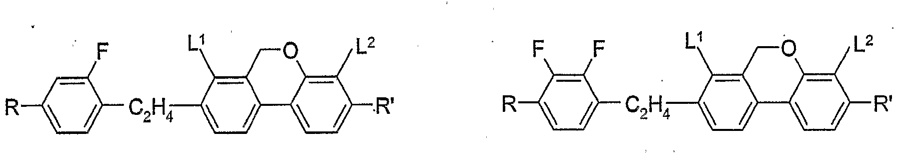

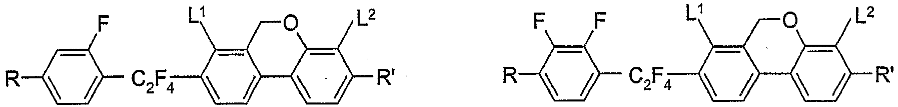

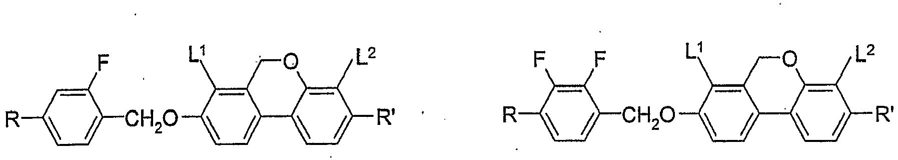

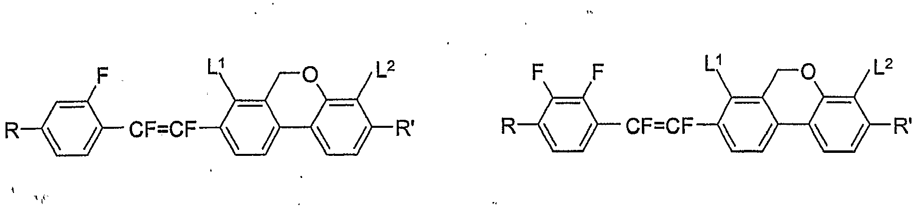

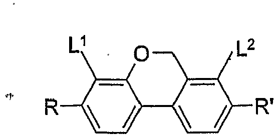

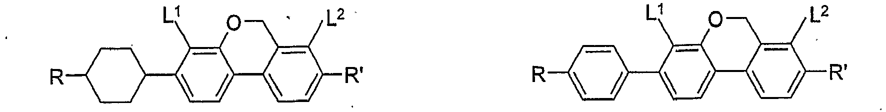

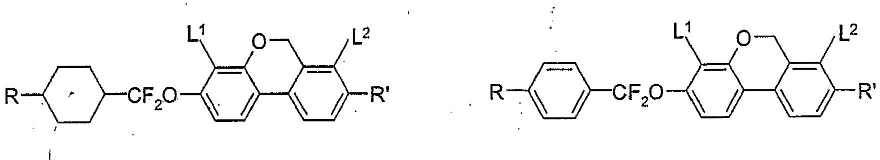

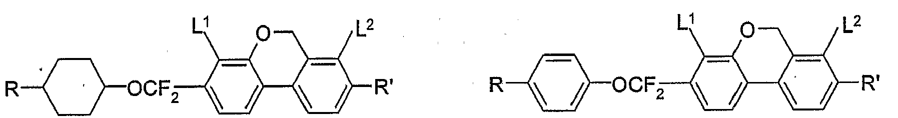

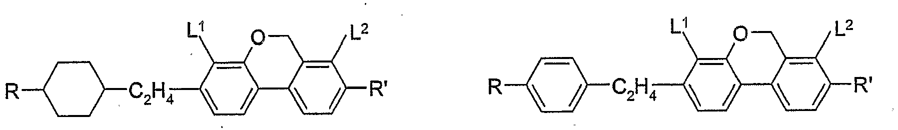

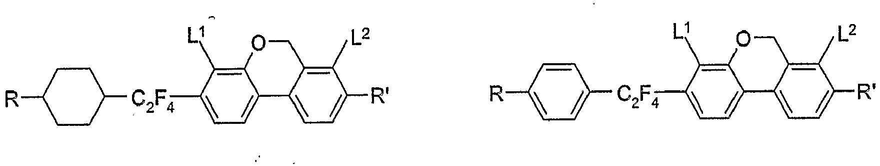

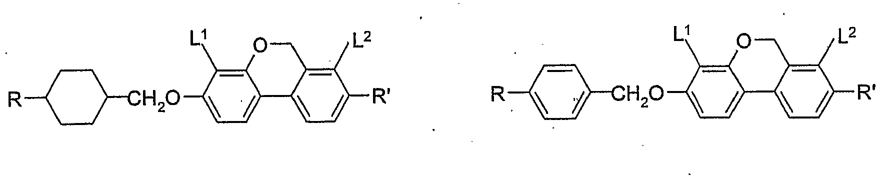

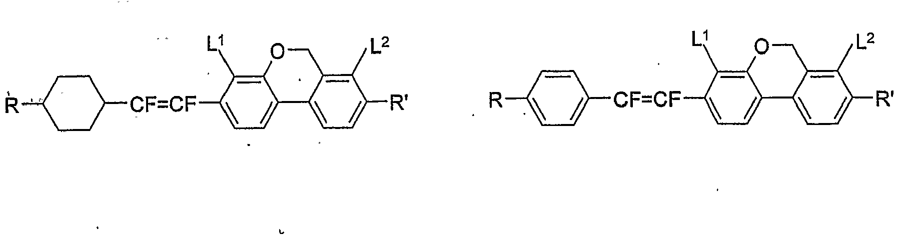

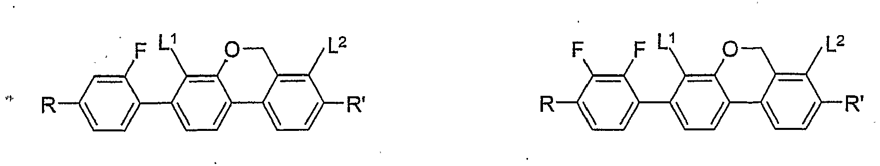

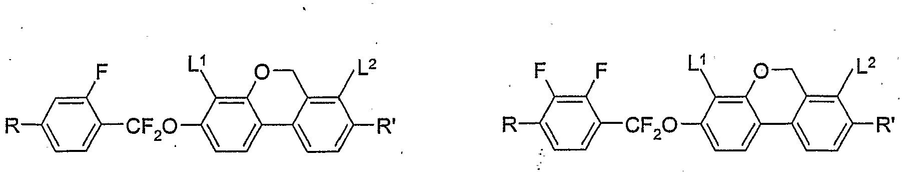

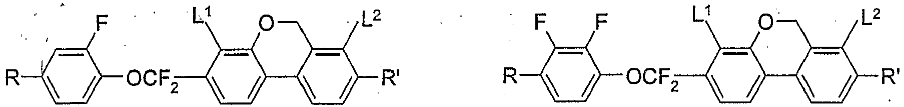

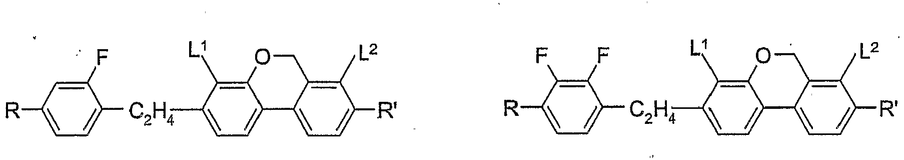

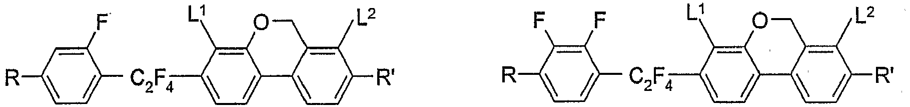

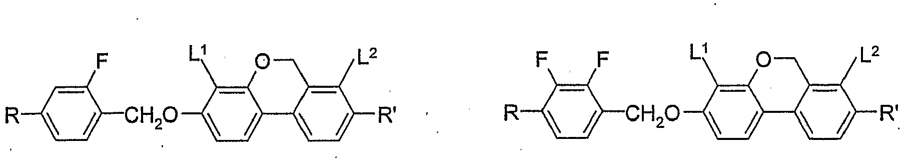

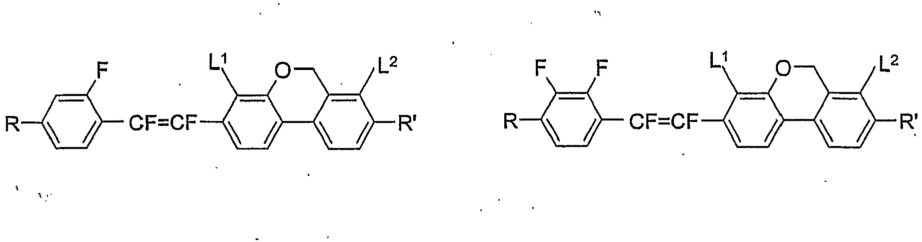

Die erfindungsgemäßen Flüssigkristallmedien enthalten eine oder mehrere Verbindungen der Formel I-2.

In einer bevorzugten Ausführungsform enthalten die Flüssigkristallmedien gemäß der vorliegenden Erfindung
a) eine oder mehrere dielektrisch negative Verbindung(en) der Formel I-2 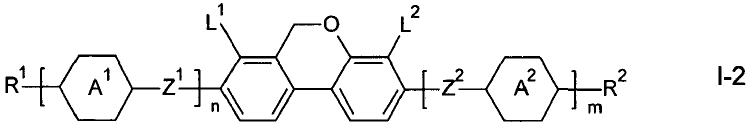 worin
   - L¹ und L²: beide F, 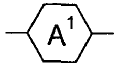
   und 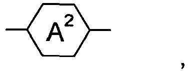 jeweils unabhängig voneinander, und wenn mehrfach vorhanden auch diese unabhängig voneinander,
   (a) einen trans-1,4-Cyclohexylenrest, worin auch eine oder zwei nicht benachbarte CH₂-Gruppen durch-O- und/oder -S- ersetzt sein können,
   (b) einen 1,4-Cyclohexenylenrest,
   (c) einen 1,4-Phenylenrest, worin auch eine oder zwei nicht benachbarte CH-Gruppen durch N ersetzt sein können oder 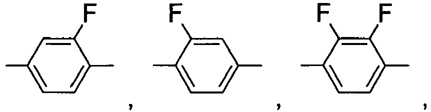 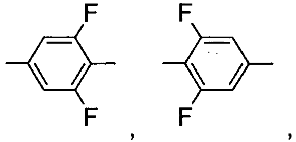 oder
   (d) einen Rest ausgewählt aus der Gruppe 1,4-Bicyclo-[2,2,2]-octylen, 1,3-Bicyclo-[1,1,1]-pentylen, Spiro-[3,3]-heptan-2,4-diyl, Piperidin-1,4-diyl, Naphtalin-2,6-diyl, Decahydronaphthalin-2,6-diyl und 1,2,3,4-Tetrahydronaphthalin-2,6-diyl, bevorzugt 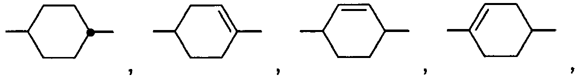 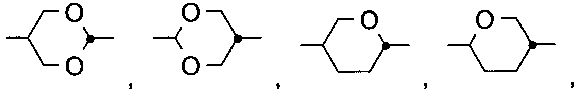 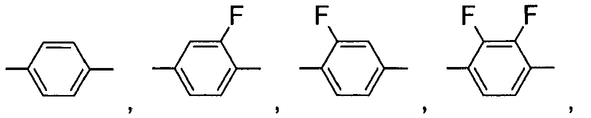 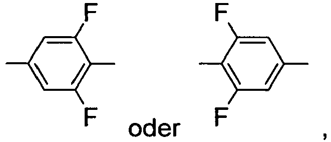
      - R¹ und R²: , jeweils unabhängig voneinander, H, Halogen, -CN, -SCN, -SF₅, -CF₃, -CHF₂, -CH₂F, -OCF₃, -OCHF₂, eine einfach durch CN oder CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-,-CF=CH-, -CH=CF-, 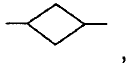 -CO-, -CO-O-, -O-CO- oder -*O*-CO-O- so ersetzt sein können, dass weder O- noch S-Atome direkt miteinander verknüpft sind,
      oder einer von
      - R¹ und R²: Alkyl und Alkoxy mit 1 bis 12 C-Atomen, Alkoxyalkyl, Alkenyl oder Alkenyloxy mit 2 bis 12 C-Atomen und der andere, unabhängig vom ersten, ebenfalls Alkyl und Alkoxy mit 1 bis 12 C-Atomen, Alkoxyalkyl, Alkenyl oder Alkenyloxy mit 2 bis 12 C-Atomen oder auch F, Cl, Br, -CN, -SCN, -SF₅, -CF₃, -CHF₂, -CH₂F, -OCF₃ oder -OCHF₂

      - Z¹ und Z²: , jeweils unabhängig voneinander, -CH₂-CH₂-, -CF₂-CF₂-, -CF₂-CH₂-,-CH₂-CF₂-, -CH=CH-, -CF=CF-, -CF=CH-, -CH=CF-, -C≡C-, -COO-, -OCO-, -CH₂O-, -CH₂O-, -CF₂O-, -OCF₂-, oder eine Kombination von zweien dieser Gruppen, wobei keine zwei O-Atome miteinander verbunden sind, oder eine Einfachbindung,
      - bevorzugt: -(CH₂)₄-, -CH₂-CH₂-, -CF₂-CF₂-, -CH=CH-, -CF=CF-, -C≡C-, -CH₂O-, -CF₂O- oder eine Einfachbindung,
      besonders bevorzugt -CH₂O-, -CH₂-CH₂-, -CF₂-CF₂-, -CF=CF-,
      - CF₂O- oder eine Einfachbindung und
         - n und m: jeweils 0, 1 oder 2, wobei
         - n + m: 0, 1, 2 oder 3, bevorzugt 0, 1 oder 2, besonders bevorzugt 0 oder 1
         bedeuten,
b) eine oder mehrere dielektrisch negative Verbindung(en) der Formel II 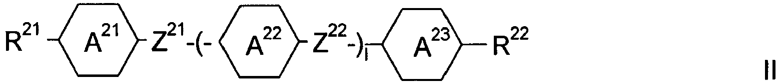 worin
   - R²¹ und R²²: jeweils unabhängig voneinander die oben bei Formel I für R¹ gegebene Bedeutung haben,
   - Z²¹ und Z²²: jeweils unabhängig voneinander die oben bei Formel I für Z¹ gegebene Bedeutung haben,
   mindestens einer der vorhandenen Ringe 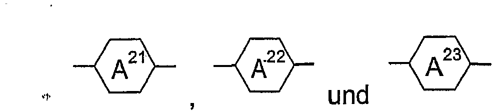 bevorzugt 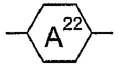 und 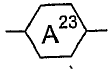 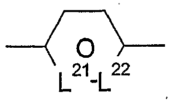 und die anderen, jeweils unabhängig voneinander, 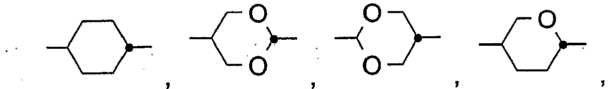 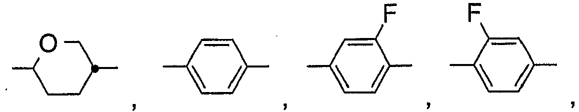 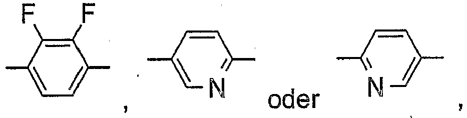 bevorzugt 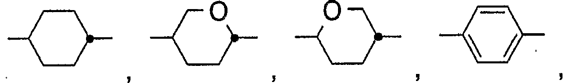 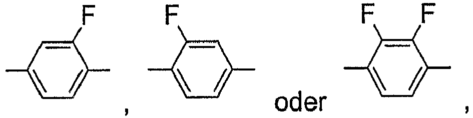 besonders bevorzugt 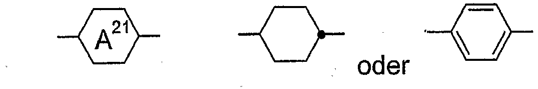 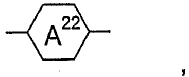 wenn vorhanden, 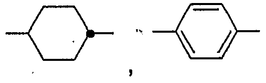 oder 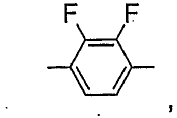 ganz besonders bevorzugt einer von 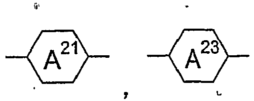 und, wenn vorhanden 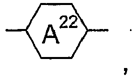 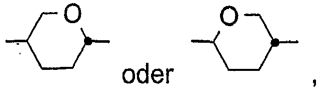 bevorzugt 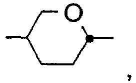
   - L²¹ und L²¹: beide C-F oder einer von beiden N und der andere C-F, bevorzugt beide C-F und
   - I: 0, 1 oder 2, bevorzugt 0 oder 1
   bedeuten; und optional
c) eine oder mehrere dielektrisch neutrale Verbindung der Formel III 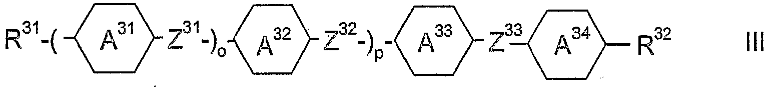 worin
   - R³¹ und R³²: jeweils unabhängig voneinander die oben bei Formel I für R¹ gegebene Bedeutung besitzen und
   - Z³¹, Z³² und Z³³: jeweils unabhängig voneinander -CH₂CH₂-, -CH=CH-, -COO- oder eine Einfachbindung 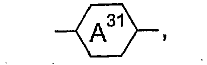 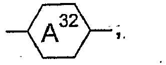 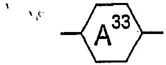
   und 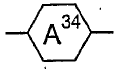 jeweils unabhängig voneinander 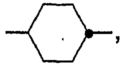 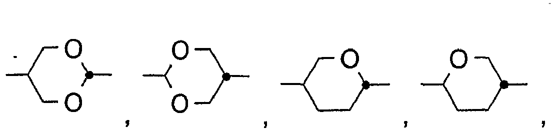 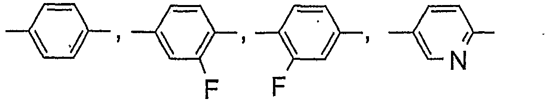 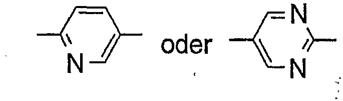 und
   - o und p: unabhängig voneinander 0 oder 1
   bevorzugt jedoch
   - R³¹ und R³²: jeweils unabhängig voneinander Alkyl oder Alkoxy mit 1-5 C-Atomen oder Alkenyl mit 2-5 C-Atomen, 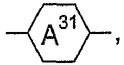 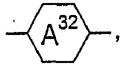 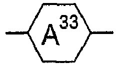
   und 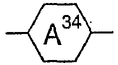 jeweils unabhängig voneinander 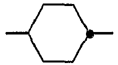 oder 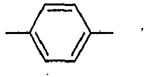 und ganz besonders bevorzugt mindestens zwei dieser Ringe 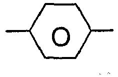 und/oder 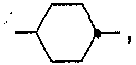 Ganz besonders bevorzugt wobei ganz besonders bevorzugt zwei benachbarte Ringe direkt verknüpft sind und zwar bevorzugt 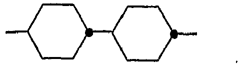 oder 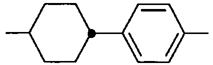 bedeuten.

Bevorzugt enthalten die Flüssigkristallmedien eine oder mehrere Verbindungen der Formel I-2 , die keine Biphenyleinheit enthalten.

Besonders bevorzugt enthalten die Flüssigkristallmedien eine oder mehrere Verbindungen der Formel I-2
worin zwei benachbarte Ringe direkt
Verknüpft sind und zwar bevorzugt 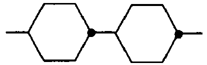 oder 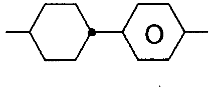 bedeuten.

Bevorzugt enthält das Flüssigkristallmedium eine oder mehrere Verbindungen ausgewählt aus der Gruppe der Verbindungen der Formeln II-1 bis II-3 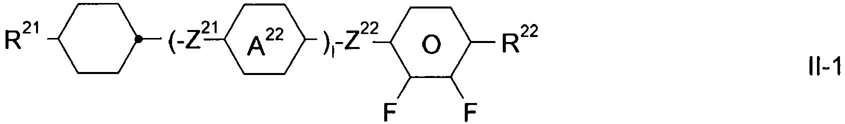 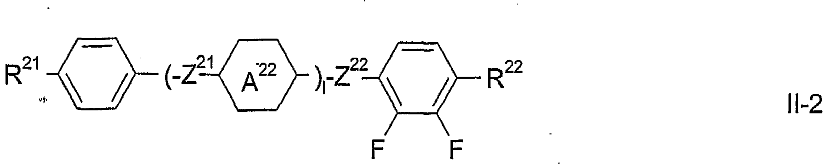 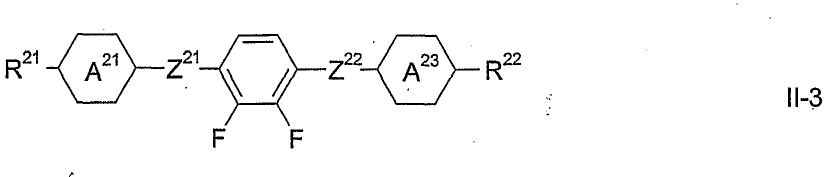 worin
R²¹, R²², Z¹², Z²², 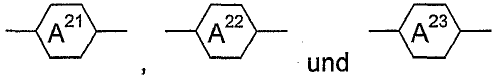 und I jeweils die oben bei Formel II gegebene Bedeutung besitzen. Bevorzugt ist R²¹ Alkyl, bevorzugt mit 1-5 C-Atomen, R²¹ Akyl oder Alkoxy, bevorzugt jeweils mit 1 bis 5 C-Atomen, und Z²² sowie Z²¹, wenn vorhanden, eine Einfachbindung.

Besonders bevorzugt enthält das Flüssigkristallmedium eine oder mehrere Verbindungen ausgewählt aus der Gruppe der Verbindungen der Formeln III-1 bis III-3: 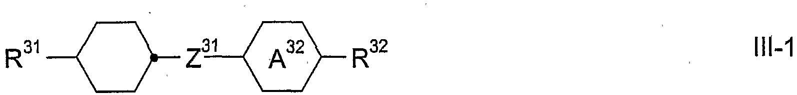 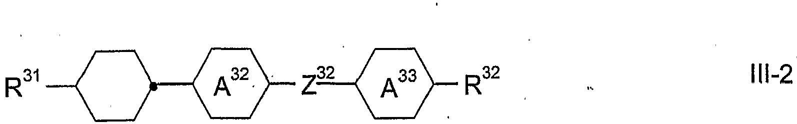 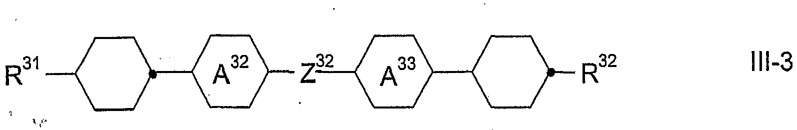 worin R³¹, R³¹, Z³¹, Z³², 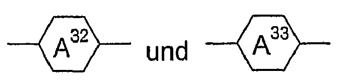 jeweils die oben bei Formel III angegebene Bedeutung haben.

Insbesondere bevorzugt enthält das Flüssigkristallmedium eine oder mehrere Verbindungen ausgewählt aus der Gruppe der Verbindungen der Formeln III-1a bis III-1d, III-1e, III-2a bis III-2g, III-3a bis III-3d und III-4a: 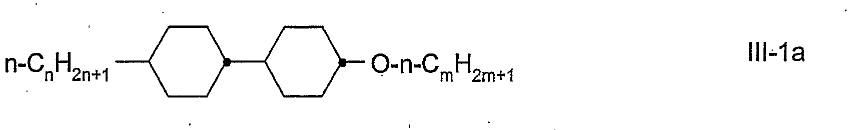 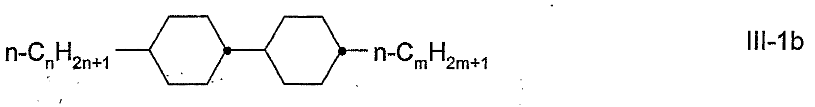 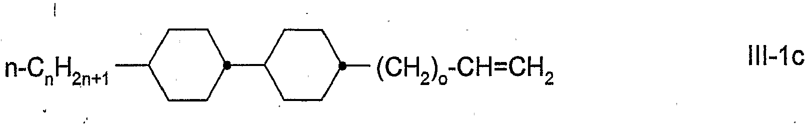 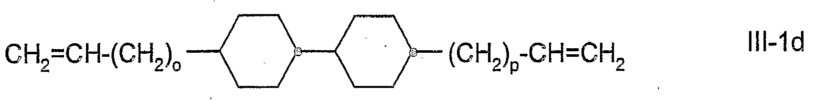 worin n und m jeweils unabhängig voneinander 1 bis 5 und o und p jeweils sowohl davon als auch voneinander unabhängig 0 bis 3 bedeuten, 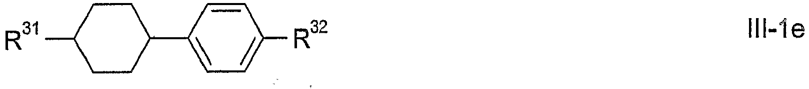 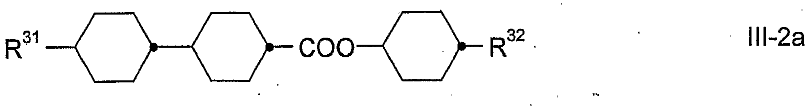 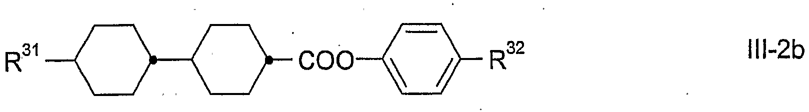 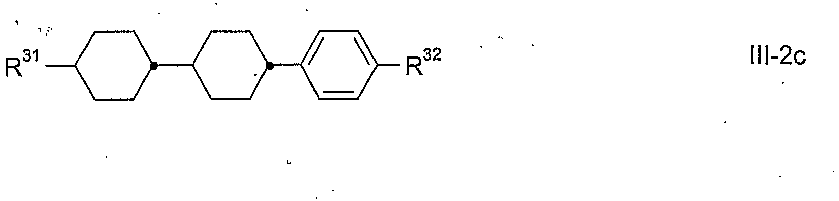 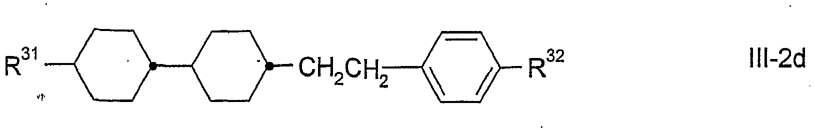 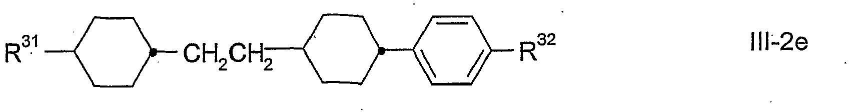 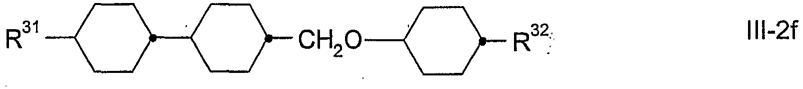 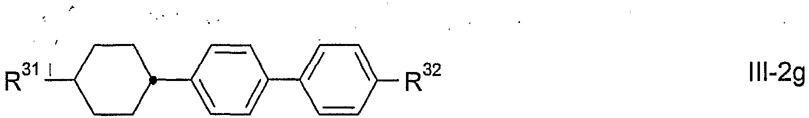 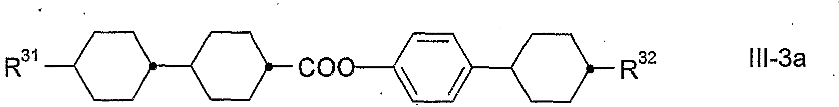 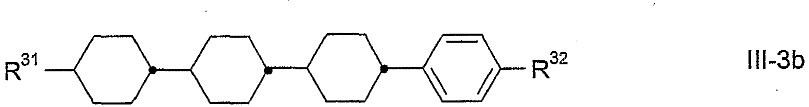 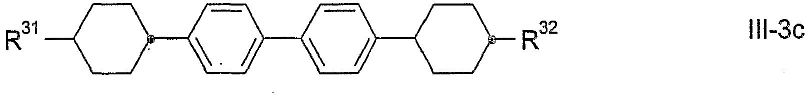 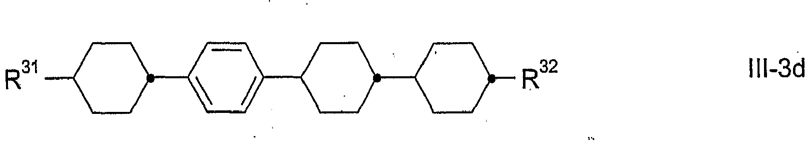 worin R³¹ und R³³ jeweils die oben unter Formel III, bevorzugt die unter Formel III-1, angegebene Bedeutung besitzen und die Phenylringe, insbesondere bei den Verbindungen III-2g und III-3c optional fluoriert sein können, jedoch nicht so, dass die Verbindungen mit denen der Formel II und ihren Unterformeln identisch sind. Bevorzugt ist R³¹ n-Alkyl mit 1 bis 5 C-Atomen, insbesondere bevorzugt mit 1 bis 3 C-Atomen und R³² n-Alkyl oder n-Alkoxy mit 1 bis 5 C-Atomen oder Alkenyl mit 2 bis 5 C-Atomen.

Hiervon sind insbesondere Verbindungen der Formeln III-1a bis III-1d bevorzugt.

Bevorzugte fluorierte Verbindungen der Formeln III-2g und III-3c sind die Verbindungen der Formeln III-2g' und III-3c' 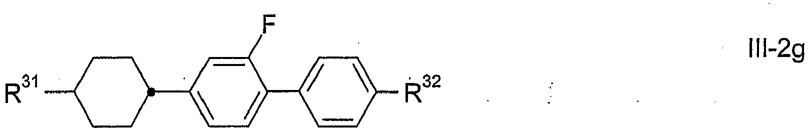 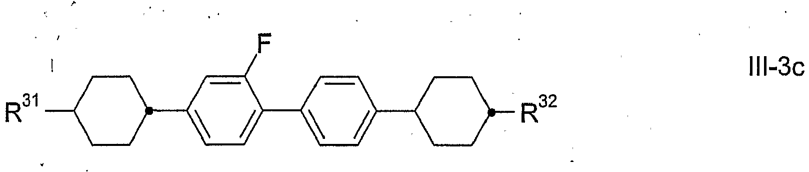 worin R³¹ und R³³ jeweils die oben unter Formel III, bevorzugt die unter Formel III-2g, bzw. III-3c angegebene Bedeutung haben.

In der vorliegenden Anmeldung, bedeutet, wenn nicht ausdrücklich anders angegeben, der Begriff Verbindungen sowohl eine Verbindung, als auch mehrere Verbindungen.

Die erfindungsgemäßen Flüssigkristallmedien weisen bevorzugt nematische Phasen von jeweils mindestens von -20°C bis 80°C, bevorzugt von-30°C bis 85°C und ganz besonders bevorzugt von -40°C bis 100°C auf. Hierbei bedeutet der Begriff eine nematische Phase aufweisen einerseits, daß bei tiefen Temperaturen bei der entsprechenden Temperatur keine smektische Phase und keine Kristallisation beobachtet wird und andererseits, daß beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, einer der elektrooptischen Anwendung entsprechenden Schichtdicke, für mindestens 100 Stunden überprüft. Als Lagerstabilität (tₛₜₒᵣ.(T)) bei der entsprechenden Temperatur (T) wird die Zeit angegenen, bis zu der alle der jeweils drei eingelagerten Testzellen keine Veränderung aufwiesen. Bei hohen Temperaturen wird der Klärpunkt nach üblichen Methoden in Kapillaren gemessen.

Ferner sind die erfindungsgemäßen Flüssigkristallmedien durch niedrige optische Anisotropien gekennzeichnet.

Der Ausdruck "Alkyl" umfaßt vorzugsweise geradkettige und verzweigte Alkylgruppen mit 1 bis 7 Kohlenstoffatomen, insbesondere die geradkettigen Gruppen Methyl, Ethyl, Propyl, Butyl, Pentyl, Hexyl und Heptyl. Gruppen mit 2 bis 5 Kohlenstoffatomen sind im allgemeinen bevorzugt.

Der Ausdruck "Alkenyl" umfaßt vorzugsweise geradkettige und verzweigte Alkenylgruppen mit 2 bis 7 Kohlenstoffatomen, insbesondere die geradkettigen Gruppen. Besonders bevorzugte Alkenylgruppen sind C₂ bis C₇-1E-Alkenyl, C₄ bis C₇-3E-Alkenyl, C₅ bis C₇-4-Alkenyl, C₆ bis C₇-5-Alkenyl und C₇ bis 6-Alkenyl, insbesondere C₂ bis C₇-1E-Alkenyl, C₄ bis C₇-3E-Alkenyl und C₅ bis C₇-4-Alkenyl. Beispiele weiterer bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl", bzw. Alkoxyalkyl umfaßt vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n 1 und m 1 bis 6.

Verbindungen mir einer Vinyl-Endgruppe und Verbindungen mit einer Methyl-Endgruppe haben eine geringe Rotationsviskosität.

In der vorliegenden Anmeldung bedeuten die Begriffe dielektrisch positive Verbindungen solche Verbindungen mit einem Δε > 1,5, dielektrisch neutrale Verbindungen solche mit -1,5 ≤ Δε ≤ 1,5 und dielektrisch negative Verbindungen solche mit Δε < -1,5. Hierbei wird die dielektrische Anisotropie der Verbindungen bestimmt indem 10 % der Verbindungen in einem flüssigkristallinen Host gelöst werden und von dieser Mischung die Kapazität in mindestens jeweils einer Testzelle mit ca. 20 µm Schichtdicke mit homeotroper und mit homogener Oberflächenorientierung bei 1 kHz bestimmt wird. Die Meßspannung beträgt typischerweise 0,5 V bis 1,0 V, jedoch stets weniger als die kapazitive Schwelle der jeweiligen Flüssigkristellmischung.

Als Wirtsmischung für die Bestimmung der anwendungsrelevanten physikalischen Parameter wird ZLI-4792, von von Merck KGaA, Deutschland, verwendet. Als Ausnahme wird bei der Bestimmung der dielektrischen Anisotropie von dielektrisch negativen Verbindungen ZLI-2857, ebenfalls von Merck KGaA, Deutschland, verwendet. Aus der Änderung der Eigenschaften, z.B. der Dielektrizitätskonstanten, der Wirtsmischung nach Zugabe der zu untersuchenden Verbindung und Extrapolation auf 100 % der eingesetzten Verbindung werden die Werte für die jeweilige zu untersuchende Verbindung erhalten.

Die eingesetzte Konzentration der zu untersuchenden Verbindung beträgt 10 %. Ist die Löslichkeit der zu untersuchenden Verbindung hierzu nicht ausreichend wird ausnahmsweise die eingesetzte Konzentration solange halbiert, also auf 5 %, 2,5% usw. verringert, bis die Löslichkeitsgrenze unterschritten ist.

Der Begriff Schwellenspannung bezieht sich üblicherweise auf die optische Schwelle für 10 % relativen Kontrast (V₁₀). In Bezug auf die Flüssigkristallmischungen mit negativer dielektrischer Anisotropie, wird der Begriff Schwellenspannung in der vorliegenden Anmeldung jedoch für die kapazitive Schwellenspannung (V₀), auch Freedericksz-Schwelle genannt, verwendet, sofern nicht explizit anders angegeben.

Alle Konzentrationen in dieser Anmeldung sind, soweit nicht explizit anders vermerkt, in Massenprozent angegeben und beziehen sich auf die entsprechende Gesamtmischung. Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20 °C, sofern nicht explizit anders angegeben. Δn wird bei 589 nm und Δε bei 1 kHz bestimmt.

Bei den Flüssigkristallmedien mit negativer dielektrischer Anisotropie wurde die Schwellenspannung als kapazitive Schwellung V₀ in Zellen mit durch Lecithin homeotrop orientierter Flüssigkristallschicht bestimmt.

Die erfindungsgemäßen Flüssigkristallmedien können bei Bedarf auch weitere Zusatzstoffe und gegebenenfalls auch chirale Dotierstoffe in den üblichen Mengen enthalten. Die eingesetzte Menge dieser Zusatzstoffe beträgt insgesamt 0 % bis 10 % bezogen auf die Menge der gesamten Mischung bevorzugt 0,1 % bis 6 %. Die Konzentrationen der einzelnen eingesetzten Verbindungen betragen jeweils bevorzugt 0,1 bis 3 %. Die Konzentration dieser und ähnlicher Zusatzstoffe wird bei der Angabe der Konzentrationen sowie der Konzentrationsbereiche der Flüssigkristallverbindungen in den Flüssigkristallmedien nicht berücksichtigt.

Die Zusammensetzungen bestehen aus mehreren Verbindungen, bevorzugt aus 3 bis 30, besonders bevorzugt aus 6 bis 20 und ganz besonders bevorzugt aus 10 bis 16 Verbindungen, die auf herkömmliche Weise gemischt werden. In der Regel wird die gewünschte Menge der in geringerer Menge verwendeten Komponenten in den Komponenten gelöst, die den Hauptbestandteil ausmachen, zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z.B. unter Verwendung von Vormischungen oder aus einem sogenannten "multi bottle" Systemen herzustellen.

Mittels geeigneter Zusatzstoffe können die erfindungsgemäßen Flüssigkristallphasen derart modifiziert werden, dass sie in jeder bisher bekannt gewordenen Art von Anzeige und insbesondere von ECB-Anzeigen, sowie IPS-Anzeigen einsetzbar sind.

Die nachstehenden Beispiele dienen zur Veranschaulichung der Erfindung, ohne sie zu beschränken. In den Beispielen sind der Schmelzpunkt T(C,N), der Übergang von der smektischen (S) zur nematischen (N) Phase T(S,N) und Klärpunkt T(N,I) einer Flüssigkristallsubstanz in Grad Celsius angegeben. Die verschiedenen smektischen Phasen werden durch entsprechende Suffixe gekennzeichnet.

Die Prozentangaben sind, soweit nicht explizit anders gekennzeichnet, vor- und nachstehend Massenprozente und die physikalischen Eigenschaften sind die Werte bei 20 °C, sofern nicht explizit anders angegeben.

Alle angegebenen Werte für Temperaturen in dieser Anmeldung sind °C und alle Temperaturdifferenzen entsprechend Differenzgräd, sofern nicht explizit anders angegeben.

Bei den Synthesebeispielen und -schemata bedeuten:
- BuLi: n-Butyllithium,
- DAST: Diethylaminoschwefeltrifluorid,
- DCC: Dicyclohexylcarbodiimid,
- DMAP: Dimethylaminopyridin,
- DMF: N,N-Dimethylformamid,
- dppf: 1,1'-Bis-(diphenylphosphino)-ferrocen,
- dppp: 1,3-Bis-(diphenylphosphino)-propan,
- LDA: Lithiumdiisopropylamid,
- MBT: MBT-Ether, Methyl-tert-butylether und
- THF: Tetrahydrofuran.

In der vorliegenden Anmeldung und in den folgenden Beispielen sind die Strukturen der Flüssigkristallverbindungen durch Abkürzungen, auch Acronyme genannt, angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹, L² und L³:

| Code für R¹, R2, L¹, L², L³ | R¹ | R² | L¹ | L² | L³ |
|---|---|---|---|---|---|
| nm | CₙH₂ₙ₊₁ | CₘH₂ₘ₊₁ | H | H | H |
| nOm | CₙH₂ₙ₊₁ | OCₘH₂ₘ₊₁ | H | H | H |
| nO.m | OCₙH₂ₙ₊₁ | CₘH₂ₘ₊₁ | H | H | H |
| nmFF | CₙH₂ₙ₊₁ | CₘH₂ₘ₊₁ | F | H | F |
| nOmFF | CₙH₂ₙ₊₁ | OCₘH₂ₘ₊₁ | F | H | F |
| nO.mFF | OCₙH₂ₙ₊₁ | CₘH₂ₘ₊₁ | F | H | F |
| nO.OmFF | OCₙH₂ₙ₊₁ | OCₘH₂ₘ₊₁ | F | H | F |
| n | CₙH₂ₙ₊₁ | CN | H | H | H |
| nN.F | CₙH₂ₙ₊₁ | CN | F | H | H |
| nN.F.F | CₙH₂ₙ₊₁ | CN | F | F | H |
| nF | CₙH₂ₙ₊₁ | F | H | H | H |
| nF.F | CₙH₂ₙ₊₁ | F | F | H | H |
| nF.F.F | CₙH₂ₙ₊₁ | F | F | F | H |
| nCl | CₙH₂ₙ₊₁ | Cl | H | H | H |
| nCl.F | CₙH₂ₙ₊₁ | Cl | F | H | H |
| nCl.F.F | CₙH₂ₙ₊₁ | Cl | F | F | H |
| nmF | CₙH₂ₙ₊₁ | CₘH₂ₘ₊₁ | F | H | H |
| nCF₃ | CₙH₂ₙ₊₁ | CF₃ | H | H | H |
| nOCF₃ | CₙH₂ₙ₊₁ | OCF₃ | H | H | H |
| nOCF₃.F | CₙH₂ₙ₊₁ | OCF₃ | F | H | H |
| nOCF₃.F.F | CₙH₂ₙ₊₁ | OCF₃ | F | F | H |
| nOCF₂ | CₙH₂ₙ₊₁ | OCHF₂ | H | H | H |
| nOCF₂.F.F | CₙH₂ₙ₊₁ | OCHF₂ | F | F | H |
| nS | CₙH₂ₙ₊₁ | NCS | H | H | H |
| rVsN | CᵣH₂ᵣ₊₁-CH=CH-CₛH₂ₛ- | CN | H | H | H |
| nEsN | CᵣH₂ᵣ₊₁-O-CₛH₂ₛ- | CN | H | H | H |
| nAm | CₙH₂ₙ₊₁ | COOCₘH₂₊₁ | H | H | H |
| nF.Cl | CₙH₂ₙ₊₁ | F | Cl | H | H |

**Tabelle A:**

| | |
|---|---|
| 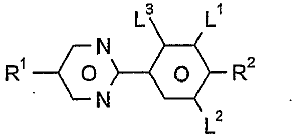 | 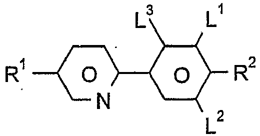 |
| **PYP** | **PYRP** |
| 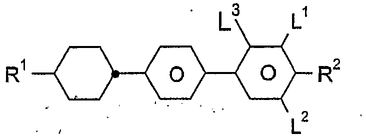 | 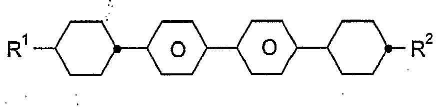 |
| **BCH** | **CBC** |
| 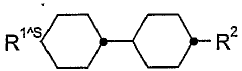 | 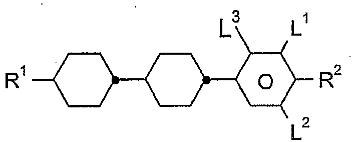 |
| **CCH** | **CCP** |
| 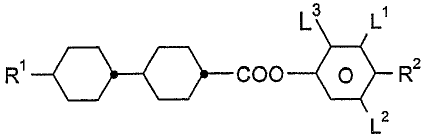 | 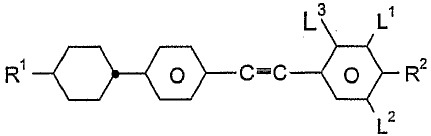 |
| **CP** | **CPTP** |
| 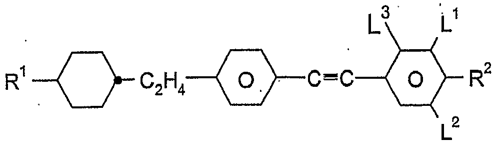 | |
| **CEPTP** | |
| 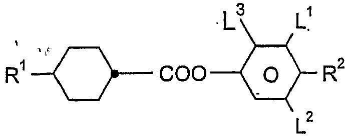 | 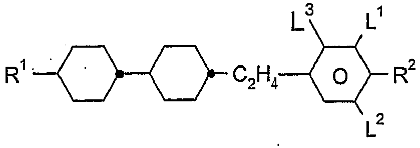 |
| **D** | **ECCP** |
| 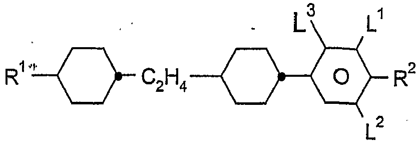 | 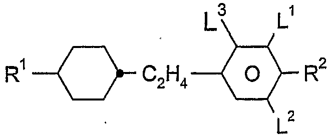 |
| **CECP** | **EPCH** |
| 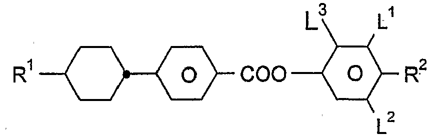 | 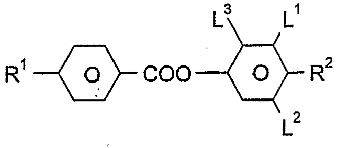 |
| **HP** | **ME** |
| 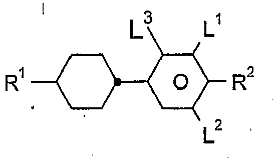 | 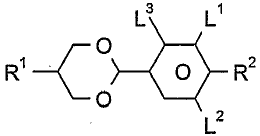 |
| **PCH** | **PDX** |
| 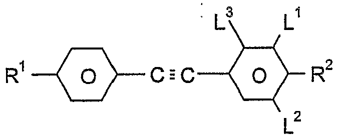 | 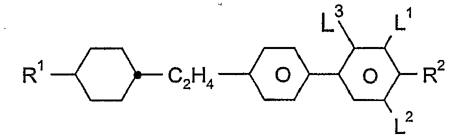 |
| **PTP** | **BECH** |
| 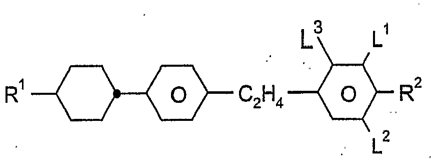 | 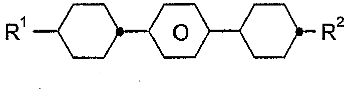 |
| **EBCH** | **CPC** |
| 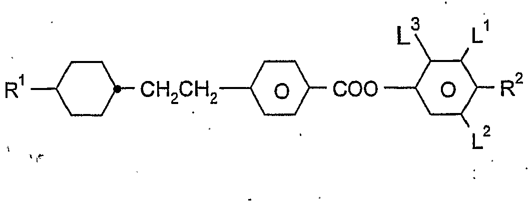 | |
| **EHP** | |
| 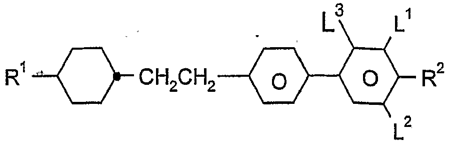 | |
| **BEP** | |
| 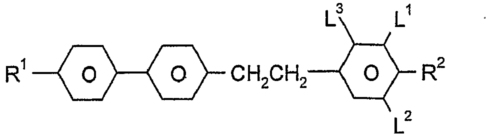 | |
| **ET** | |

**Tabelle B:**

| | |
|---|---|
| 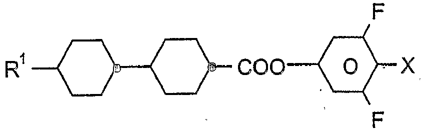 | 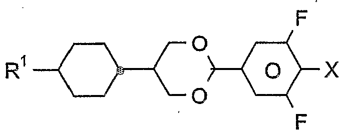 |
| **CCZU-n-X** (X = F, Cl, -OCF3 = "OT") | **CDU-n-X** (X = F, Cl, -OCF3 = "OT") |
| 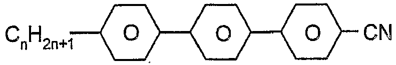 | 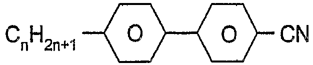 |
| **T3n** | **K3n** |
| 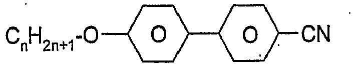 | 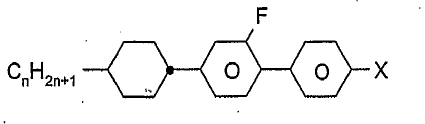 |
| **M3n** | **CGP-n-X** (X = F, Cl, -OCF3 = "OT") |
| 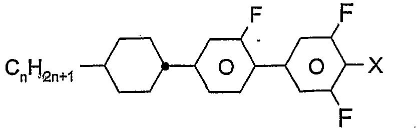 | 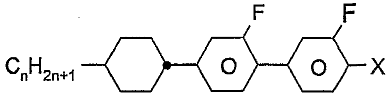 |
| **CGU-n-X** (X = F, Cl, -OCF3 = "OT") | **CGG-n-X** (X = F, Cl, -OCF3 = "OT") |
| 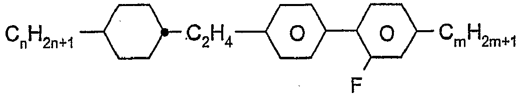 | |
| **Inm** | |
| 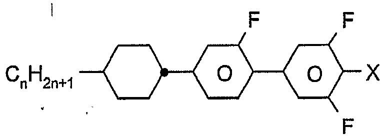 | |
| **CGU-n-X** (X = F, Cl, -OCF3 = "OT") | |
| 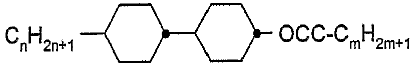 | 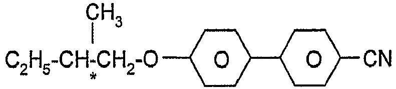 |
| **C-nm** | **C15** |
| 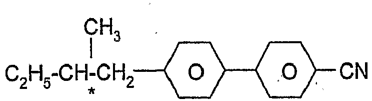 | |
| **CB15** | |
| 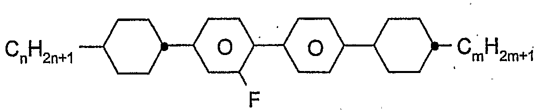 | |
| **CBC-nmF** | |
| 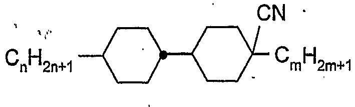 | 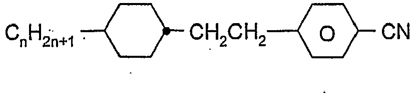 |
| **CCN-nm** | **G3n** |
| 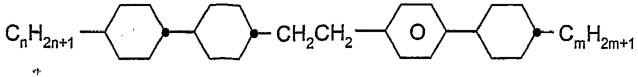 | |
| **CCEPC-nm** | |
| 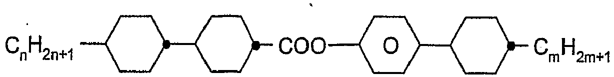 | |
| **CCPC-nm** | |
| 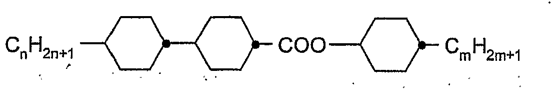 | |
| **CH-nm** | |
| 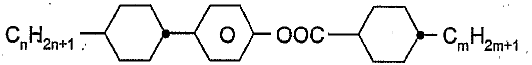 | |
| **HD-nm** | |
| 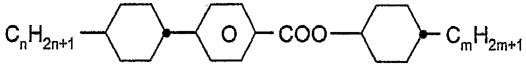 | |
| **HH-nm** | |
| 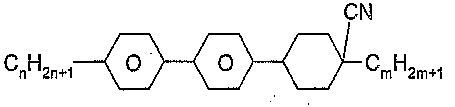 | |
| **NCB-nm** | |
| 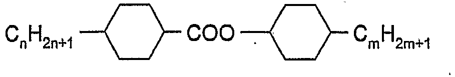 | |
| **OS-nm** | |
| 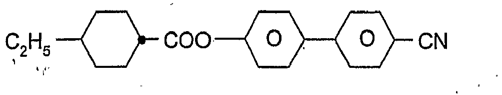 | |
| **CHE** | |
| 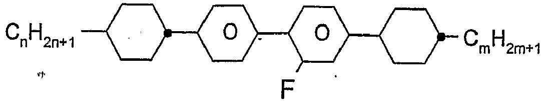 | |
| **CBC-nmF** | |
| 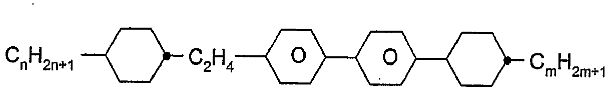 | |
| **ECBC-nm** | |
| 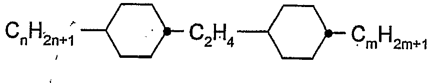 | 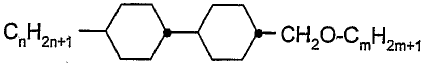 |
| **ECCH-nm** | **CCH-n1EM** |
| 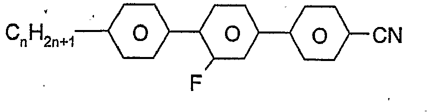 | 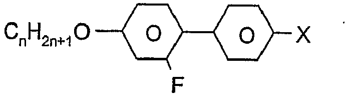 |
| **T-nFN** | **GP-nO-X** (X = F, Cl, -OCF3 = "OT") |
| 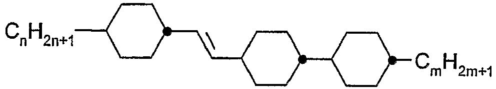 | |
| **CVCC-n-m** | |
| 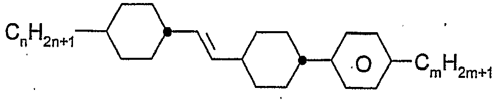 | |
| **CVCP-n-m** | |
| 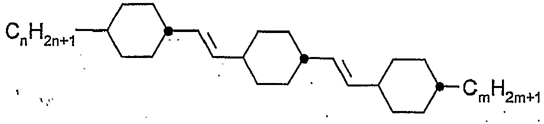 | |
| **CVCVC-n-m** | |
| 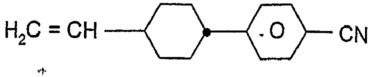 | |
| **CP-V-N** | |
| 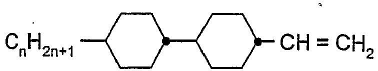 | |
| **CC-n-V** | |
| 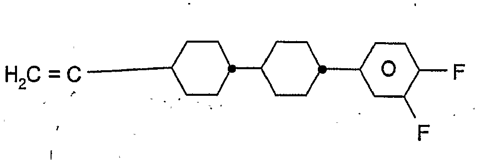 | |
| **CCG-V-F** | |
| 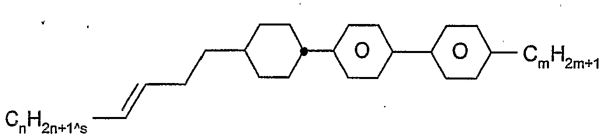 | |
| **CPP-nV2-m** | |
| 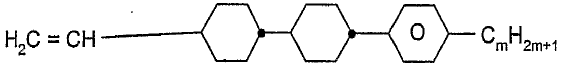 | |
| **CCP-V-m** | |
| 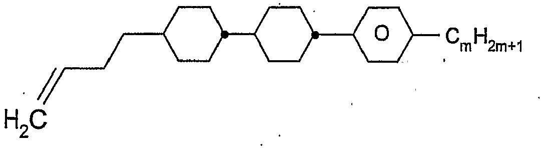 | |
| **CCP-V2-m** | |
| 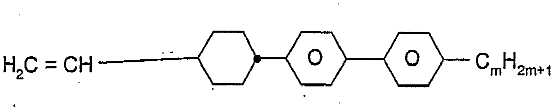 | |
| **CPP-V-m** | |
| 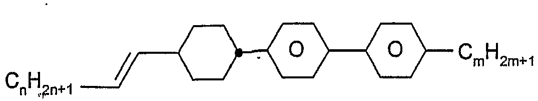 | |
| **CPP-nV-m** | |
| 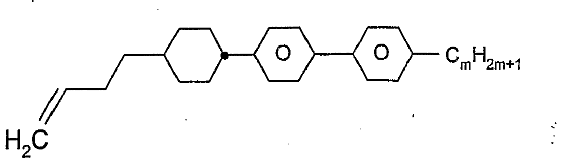 | |
| **CPP-V2-m** | |
| 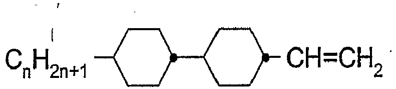 | |
| **CC-n-V** | |
| 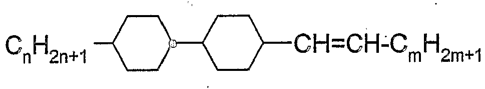 | |
| **CC-n-Vm** | |
| 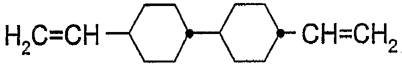 | |
| **CC-V-V** | |
| 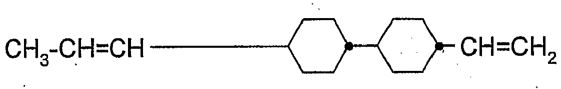 | |
| **CC-1V-V** | |
| 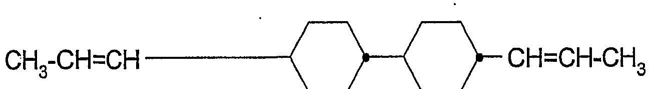 | |
| **CC-1V-V1** | |
| 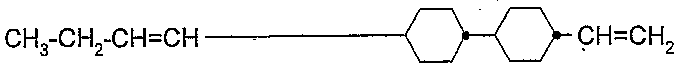 | |
| **CC-2V-V** | |
| 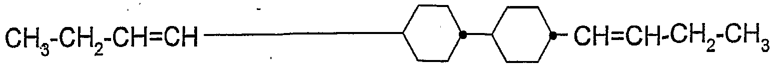 | |
| **CC-2V-V2** | |
| 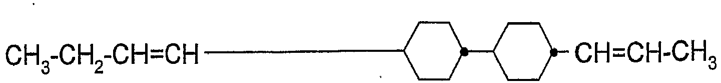 | |
| **CC-2V-V1** | |
| 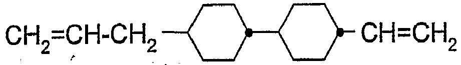 | |
| **CC-V1-V** | |
| 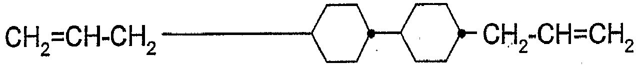 | |
| **CC-V1-1V** | |
| 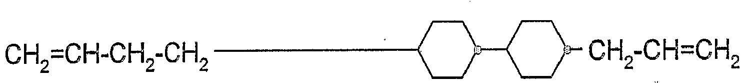 | |
| **CC-V2-1V** | |
| 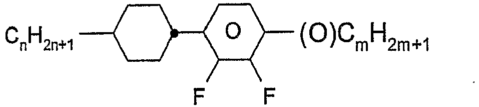 | |
| **PCH-n(O)mFF** | |
| 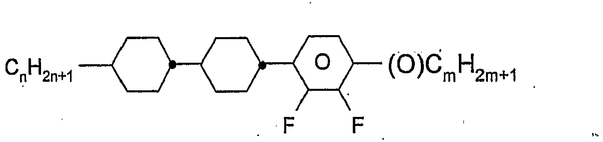 | |
| **CCP-n(O)mFF** | |
| 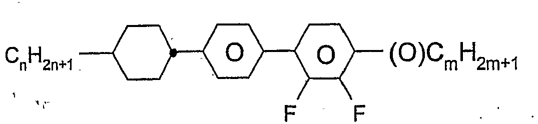 | |
| **BCH-n(O)mFF** | |
| 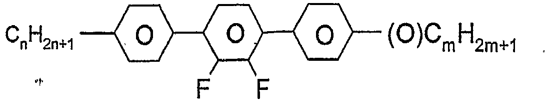 | |
| **PYP-n-(O)m** | |
| 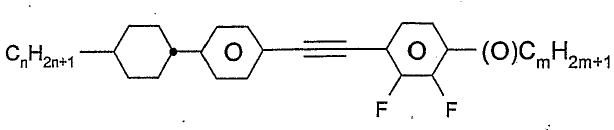 | |
| **CPTP-n(O)mFF** | |
| 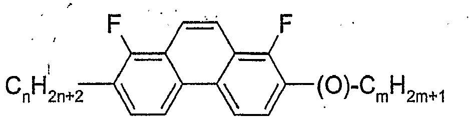 | |
| **Ph-n-(0)mFF** | |
| 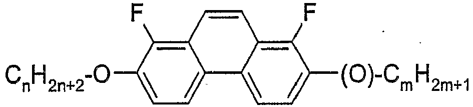 | |
| **Ph-n0-(0)mFF** | |
| 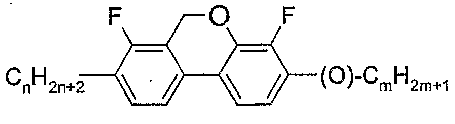 | |
| **BHHO-n-(0)mFF** | |
| 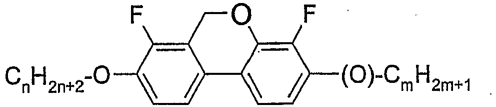 | |
| **BHHO-n0-(0)mFF** | |
| 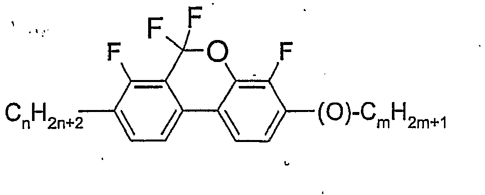 | |
| **BFFO-n-(0)mFF** | |
| 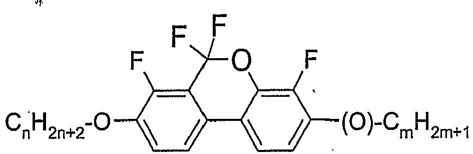 | |
| **BFFO-n0-(0)mFF** | |
| 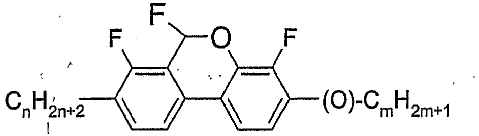 | |
| **BFO-n-(0)mFF** | |
| 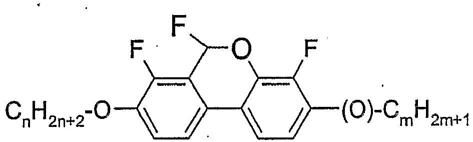 | |
| **BFO-n0-(0)mFF** | |
| 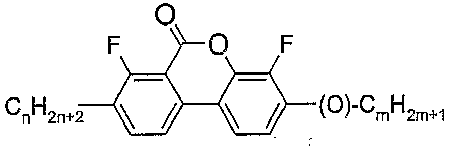 | |
| **BCOO-n-(0)mFF** | |
| 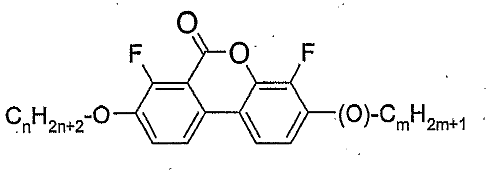 | |
| **BCOO-n0-(0)mFF.** | |
| 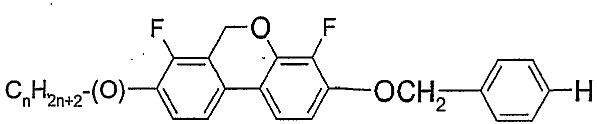 | |
| **BHHO-O1P-n(O)-HFF** | |
| 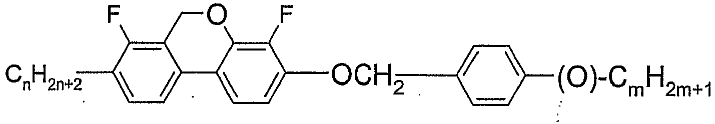 | |
| **BHHO-O1P-n-(O)mFF** | |
| 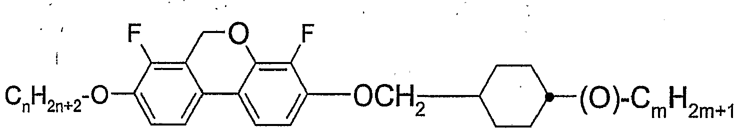 | |
| **BHHO-O1 C-nO-(O)mFF** | |
| 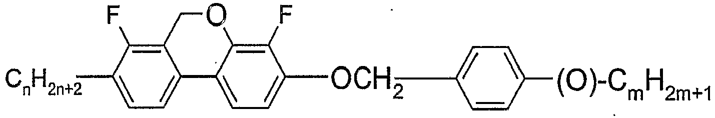 | |
| **BHHO-O1P-n-(O)mFF** | |
| 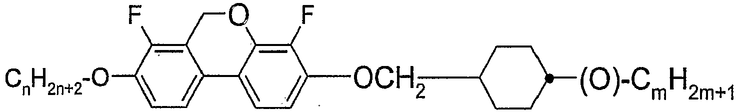 | |
| **BHHO-O1C-nO-(O)mFF** | |
| 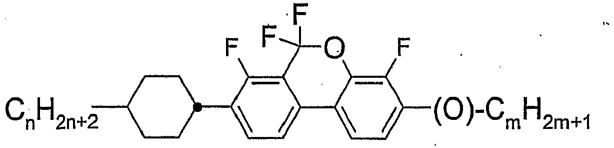 | |
| **C-BFFO-n-(O)mFF** | |
| 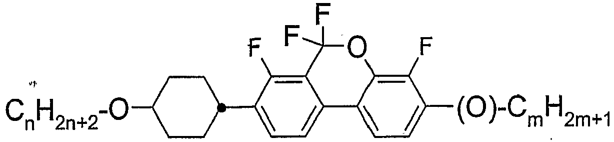 | |
| **C-BFFO-nO-(O)mFF** | |

### Beispiele

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Δn bedeutet optische Anisotropie (589 nm, 20 °C), Δε die dielektrische Anisotropie (1 kHz, 20 °C), H.R. die "voltage holding ratio" (bei 100 °C, nach 5 Minuten im Ofen, 1 V), V₁₀, V₅₀ und V₉₀ (die Schwellenspannung, Mittgrauspannung bzw. Sättigungsspannung), sowie V₀ (die kapazitive Schwellenspannung) wurden jeweils bei 20 °C bestimmt.

### Substanzbeispiele

Die Verbindungen 1 bis 120 sind als Ausgangsverbindungen für die Herstellung von Verbindungen der Formel I-2 geeignet.

### Ausgangsverbindung 1: (4,7-Difluor-8-methyl-3-pentyl-6H-benzo[c]chromen-6-on)

### 1.1 Herstellung von 4-Brom-2-fluor-1-pentylbenzol

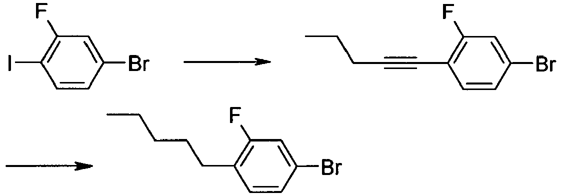

190 g (0,600 mol) 4-Brom-2-fluor-1-iodbenzol und 65,3 ml 1-Pentin wurden in einer Mischung aus 900 ml THF und 1.2 l Triethylamin gelöst, auf 10°C abgekühlt und mit 1,14 g (6 mmol) Kupfer(I)iodid sowie 8,42 g (12 mmol) Bis(triphenylphosphin)palladium(II)chlorid versetzt. Der Ansatz wurde über Nacht bei Raumtemperatur gerührt, anschließend wurde Wasser und MTB zugegeben und 5 min. nachgerührt. Das Reaktionsgemisch wurde über Celite® abgesaugt und die Phasen getrennt. Die wäßrige Phase wurde zweimal mit MTB-Ether extrahiert, die vereinigten organischen Phasen dreimal mit Wasser gewaschen, über Natriumsulfat getrocknet und im Vakuum eingeengt. Das Rohprodukt wurde mit n-Heptan über Kieselgel filtriert. Man erhielt 129 g 4-Brom-2-fluor-1-pent-1-inylbenzol als gelbe

### 1.2 Herstellung von 6-Brom-2-fluor-3-pentylphenol

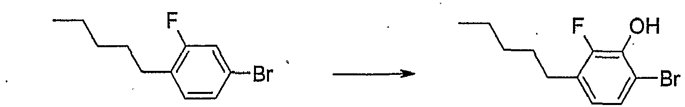

132 g (0,583 mol) 4-Brom-2-fluor-1-pentylbenzol wurden in 900 ml THF gelöst und bei -70 °C 295 ml einer 2 molaren Lösung von LDA in THF zutropfen gelassen. Nach 1 h wurden 65,9 ml (0,590 mol) Trimethylborat zugegeben, 1h nachgerührt und dann bei -15°C mit 150 ml 50-prozentiger Essigsäure angesäuert. Anschließend wurde der Ansatz auf 30°C erwärmt und 139 ml (1,61 mol) 35-prozentige Wasserstoffperoxidlösung zutropfen gelassen. Nach 1h wurde mit Wasser verdünnt und die organische Phase abgetrennt. Die vereinigten organischen Phasen wurden zweimal mit Ammoniumeisen(II)sulfatlösung und einmal mit Wasser gewaschen, über Natriumsulfat getrocknet und im Vakuum eingeengt.

Nach Filtration des Rohproduktes mit *n*-Heptan/1-Chlorbutan (3:1) über Kieselgel erhielt man 86,0 g (61 % d. Th.) 6- Brom-2-fluor-3-pentylphenol als farblose Kristalle.

### 1.3 Herstellung von 6-Brom-2-fluor-3-methylbenzoesäure

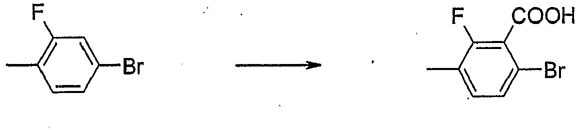

50 ml (0,385 mol) 4-Brom-2-fluortoluol wurden in 750 ml THF gelöst und bei -70 °C 231 ml (0,462 mol) einer 2 M Lösung von LDA in THF zutropfen gelassen. Nach 70 min wurden 37,2 g (0,846 mol) Kohlendioxid eingeleitet und der Ansatz auftauen gelassen. Nach dem Ansäuern mit konz. Salzsäure wurde die Lösung mit MTB-Ether extrahiert, die vereinigten organischen Phasen mit Wasser gewaschen, über Natriumsulfat getrocknet und im Vakuum eingeengt. Nach Kristallisation des Rohproduktes aus 1-Chlor-butan erhielt man 44.1 g (49 %) 6-Brom-2-fluor-3-methylbenzoesäure als weiße Kristalle.

### 1.4 Herstellung von 6-Brom-2-fluor-3-methylbenzoesäure-(6-brom-2-fluor-3-pentylphenyl)ester

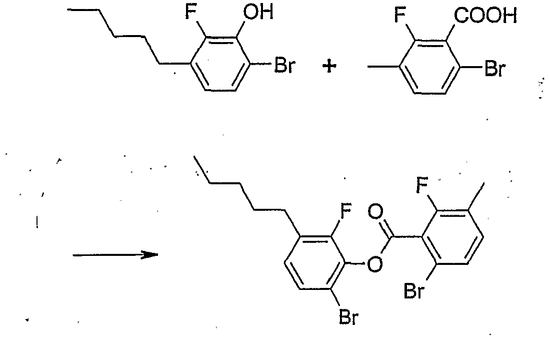

43,2 g (0,165 mol) 6-Brom-2-fluor-3-pentylphenol, 40,5 g (0,173 mol) 6-Brom-2-fluor-3-methylbenzoesäure und 3,52 g (29 mmol) 4-(Dimethylamino)pyridin wurden in 300 ml Dichlormethan vorgelegt und eine Lösung von 35,7 g (0,172 mol) N,N-Dicyclohexylcarbodiimid in 80 ml Dichlormethan hinzugefügt. Der Ansatz wurde über Nacht bei Raumtemperatur gerührt und anschließend mit 4,16 g (33 mmol) Oxalsäure versetzt. Nach 1 h wurde der ausgefallene Feststoff abfiltriert und das Filtrat im Vakuum eingeengt. Das Rohprodukt wurde mit *n-*Heptan/1-Chlorbutan (1:1) über Kieselgel filtriert. Man erhielt 72,7 g (91 % d. Th.) 6-Brom-2-fluor-3-methylbenzoesäure-(6-brom-2-fluor-3-pentylphenyl)ester als farbloses Öl.

### 1.5 Herstellung von 4,7-Difluor-8-methyl-3-pentyl-6H-benzo[c]chromen-6-on

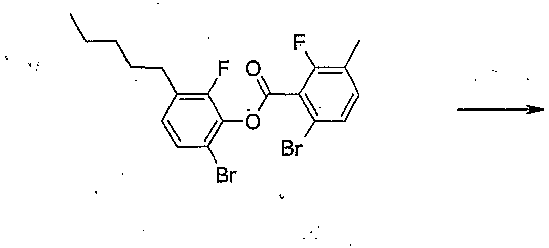

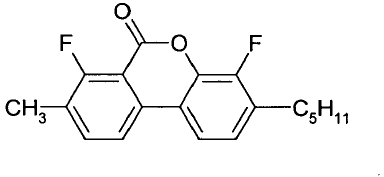

56,2 g (118 mmol) 6-Brom-2-fluor-3-methylbenzoesäure-(6-brom-2-fluor-3-pentyl)phenylester wurde in 650 ml DMF gelöst und in Gegenwart von 75,1 g (1,18 mmol) Kupferpulver 48 h unter Rückfluß erhitzt. Anschließend wurde mit Wasser verdünnt und mit Essigester extrahiert. Die Extrakte wurden vereinigt, über Na₂SO₄ getrocknet und eingeengt. Das Rohprodukt wurde aus 1-Chlorbutan umkristallisiert. Es wurden 9,60 g des Lactons als farbloser Feststoff erhalten. Dies entspricht einer Ausbeute von 26 %.

Die physikalischen Eigenschaften der Verbindung sind in der folgenden Tabelle angegeben.

### Ausgangsverbindungen 2 bis 120

Analog zu Ausgangsverbindung 1 werden hergestellt:

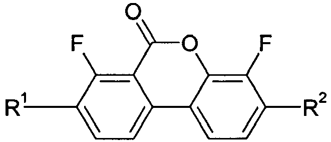

Bemerkung: * aus 10%-iger Lösung in ZLI-4792, bzw. ZLI-2857 (Δε) extrapolierte Werte.

| Nr. | R¹ | R² | Phasensequenz T/°C | Δε* | T*(N,I) /°C |
|---|---|---|---|---|---|
| 2 | CH₃ | CH₃ | C 237 I | | |
| 3 | CH₃ | C₂H₅ | | | |
| 4 | CH₃ | *n*-C₃H₇ | | | |
| 5 | CH₃ | *n*-C₄H₉ | | | |
| 1 | CH₃ | *n*-C₅H₁₁ | C 104 I | -17,8 | 22 |
| 6 | CH₃ | *n*-C₅H₁₃ | | | |
| 7 | CH₃ | *n*-C₇H₁₅ | | | |
| 8 | CH₃ | CH₃O | | | |
| 9 | CH₃ | C₂H₅O | | | |
| 10 | CH₃ | *n*-C₃H₇O | | | |
| 11 | CH₃ | *n*-C₄H₉O | | | |
| 12 | CH₃ | CH₂=CH | | | |
| 13 | CH₃ | *E*-CH₃-CH=CH | | | |
| 14 | CH₃ | CH₂=CH-O | | | |
| 15 | CH₃ | CH₂=CH-CH₂O | | | |
| 16 | C₂H₅ | CH₃ | | | |
| 17 | C₂H₅ | C₂H₅ | | | |
| 18 | C₂H₅ | *n*-C₃H₇ | | | |
| 19 | C₂H₅ | *n*-C₄H₉ | | | |
| 20 | C₂H₅ | *n*-C₅H₁₁ | | | |
| 21 | C₂H₅ | *n*-C₆H₁₃ | | | |
| 22 | C₂H₅ | *n*-C₇H₁₅ | | | |
| 23 | C₂H₅ | CH₃O | | | |
| 24 | C₂H₅ | C₂H₅O | | | |
| 25 | C₂H₅ | *n*-C₃H₇O | | | |
| 26 | C₂H₅ | *n*-C₄H₉O | | | |
| 27 | C₂H₅ | CH₂=CH | | | |
| 28 | C₂H₅ | *E*-CH₃-CH=CH | | | |
| 29 | C₂H₅ | CH₂=CH-O | | | |
| 30 | C₂H₅ | CH₂=CH-CH₂O | | | |
| 31 | *n*-C₃H₇ | CH₃ | | | |
| 32 | *n*-C₃H₇ | C₂H₅ | | | |
| 33 | *n*-C₃H₇ | *n*-C₃H₇ | | | |
| 34 | *n*-C₃H₇ | *n*-C₄H₉ | | | |
| 35 | *n*-C₃H₇ | *n*-C₅H₁₁ | C 103 I | -19,0 | 7 |
| 36 | *n*-C₃H₇ | *n*-C₅H₁₃ | | | |
| 37 | *n*-C₃H₇ | *n*-C₇H₁₅ | | | |
| 38 | *n*-C₃H₇ | CH₃O | | | |
| 39 | *n*-C₃H₇ | C₂H₅O | | | |
| 40 | *n*-C₃H₇ | *n*-C₃H₇O | | | |
| 41 | *n*-C₃H₇ | *n*-C₄H₉O | | | |
| 42 | *n*-C₃H₇ | CH₂=CH | | | |
| 43 | *n*-C₃H₇ | *E*-CH₃-CH=CH | | | |
| 44 | *n*-C₃H₇ | CH₂=CH-O | | | |
| 45 | *n*-C₃H₇ | CH₂=CH-CH₂O | | | |
| 46 | *n*-C₄H₉ | CH₃ | | | |
| 47 | *n*-C₄H₉ | C₂H₅ | | | |
| 48 | *n*-C₄H₉ | *n*-C₃H₇ | | | |
| 49 | *n*-C₄H₉ | *n*-C₄H₉ | | | |
| 50 | *n*-C₄H₉ | *n*-C₅H₁₁ | | | |
| 51 | *n*-C₄H₉ | *n*-C₆H₁₃ | | | |
| 52 | *n*-C₄H₉ | *n*-C₇H₁₅ | | | |
| 53 | *n*-C₄H₉ | CH₃O | | | |
| 54 | *n*-C₄H₉ | C₂H₅O | | | |
| 55 | *n*-C₄H₉ | *n*-C₃H₇O | | | |
| 56 | *n*-C₄H₉ | *n*-C₄H₉O | | | |
| 57 | *n*-C₄H₉ | CH₂=CH | | | |
| 58 | *n*-C₄H₉ | *E*-CH₃-CH=CH | | | |
| 59 | *n*-C₄H₉ | CH₂=CH-O | | | |
| 60a | *n*-C₄H₉ | CH₂=CH-CH₂O | | | |
| 60b | *n*-C₅H₁₁ | *n*-C₄H₉O | C 130 I | -23,5 | 57 |
| 61 | CH₃O | CH₃ | | | |
| 62 | CH₃O | C₂H₅ | | | |
| 63 | CH₃O | *n*-C₃H₇ | | | |
| 64 | CH₃O | *n*-C₄H₉ | | | |
| 65 | CH₃O | *n*-C₅H₁₁ | | | |
| 66 | CH₃O | *n*-C₆H₁₃ | | | |
| 67 | CH₃O | *n*-C₇H₁₅ | | | |
| 68 | CH₃O | CH₃O | | | |
| 69 | CH₃O | C₂H₅O | | | |
| 70 | CH₃O | *n*-C₃H₇O | | | |
| 71 | CH₃O | *n*-C₄H₉O | | | |
| 72 | CH₃O | CH₂=CH | | | |
| 73 | CH₃O | *E*-CH₃-CH=CH | | | |
| 74 | CH₃O | CH₂=CH-O | | | |
| 75 | CH₃O | CH₂=CH-CH₂O | | | |
| 76 | C₂H₅O | CH₃ | | | |
| 77 | C₂H₅O | C₂H₅ | | | |
| 78 | C₂H₅O | *n*-C₃H₇ | | | |
| 79 | C₂H₅O | *n*-C₄H₉ | | | |
| 80 | C₂H₅O | *n*-C₅H₁₁ | C 137 I | | |
| 81 | C₂H₅O | *n*-C₆H₁₃ | | | |
| 82 | C₂H₅O | *n*-C₇H₁₅ | | | |
| 83 | C₂H₅O | CH₃O | | | |
| 84 | C₂H₅O | C₂H₅O | | | |
| 85 | C₂H₅O | *n*-C₃H₇O | | | |
| 86 | C₂H₅O | *n*-C₄H₉O | | | |
| 87 | C₂H₅O | CH₂=CH | | | |
| 88 | C₂H₅O | *E*-CH₃-CH=CH | | | |
| 89 | C₂H₅O | CH₂=CH-O | | | |
| 90 | C₂H₅O | CH₂=CH-CH₂O | | | |

| Nr., | R¹ | R² | Phasensequenz T/°C | Δε* | |
|---|---|---|---|---|---|
| 91 | CH₂=CH | CH₃ | | | |
| 92 | CH₂=CH | C₂H₅ | | | |
| 93 | CH₂=CH | *n*-C₃H₇ | | | |
| 94 | CH₂=CH | *n*-C₄H₉ | | | |
| 95 | CH₂=CH | *n*-C₅H₁₁ | | | |
| 96 | CH₂=CH | *n*-C₆H₁₃ | | | |
| 97 | CH₂=CH | *n*-C₇H₁₅ | | | |
| 98 | CH₂=CH | CH₃O | | | |
| 99 | CH₂=CH | C₂H₅O | | | |
| 100 | CH₂=CH | *n*-C₃H₇O | | | |
| 101 | CH₂=CH | *n*-C₄H₉O | | | |
| 102 | CH₂=CH | CH₂=CH | | | |
| 103 | CH₂=CH | *E*-CH₃-CH=CH | | | |
| 104 | CH₂=CH | CH₂=CH-O | | | |
| 105 | CH₂=CH | CH₂=CH-CH₂O | | | |
| 106 | CH₂=CH-O | CH₃ | | | |
| 107 | CH₂=CH-O | C₂H₅ | | | |
| 108 | CH₂=CH-O | *n*-C₃H₇ | | | |
| 109 | CH₂=CH-O | *n*-C₄H₉ | | | |
| 110 | CH₂=CH-O | *n*-C₅H₁₁ | | | |
| 111 | CH₂=CH-O | *n*-C₆H₁₃ | | | |
| 112 | CH₂=CH-O | *n*-C₇H₁₅ | | | |
| 113 | CH₂=CH-O | CH₃O | | | |
| 114 | CH₂=CH-O | C₂H₅O | | | |
| 115 | CH₂=CH-O | *n*-C₃H₇O | | | |
| 116 | CH₂=CH-O | *n*-C₄H₉O | | | |
| 117 | CH₂=CH-O | CH₂=CH | | | |
| 118 | CH₂=CH-O | *E*-CH₃-CH=CH | | | |
| 119 | CH₂=CH-O | CH₂=CH-O | | | |
| 120 | CH₂=CH-O | CH₂=CH-CH₂O | | | |

### Beispiel 121: (4,7-Difluor-8-methyl-3-pentyl-6H-benzo[c]-chromen)

### Herstellung von 4,7-Difluor-8-methyl-3-pentyl-6H-benzo[c]-chromen

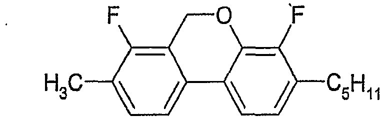

2,00 g (6,33 mmol) der Verbindung des Beispiels 1 wurden in 12 ml THF gelöst. Zu dieser Lösung wurden unter Kühlung mit Eis 2,56 ml (23 mmol) Bortrifluorid-THF-Komplex zugegben. Dann wurden nacheinender 12 ml Diethylenglykoldimethylether und portionsweise 0,58 g (15 mmol) Natriumborhydrid zugegeben und bei Raumtemperatur (ca. 20°C) 16 h lang gerührt. Die Reaktionslösung wurde mit Eiswasser hydrolysiert mit MBT-Ether extrahiert, die Extrakte vereinigt und über Na₂SO₄ getrocknet und eingeengt. Das Rohprodukt wurde aus n-Heptan umkristallisiert. Es wurden 1,60 g farblose Kristalle des Benzochromens erhalten. Dies entspricht einer Ausbeute von 83 %.

### Beispiele 122 bis 240

Analog zu Beispiel 121 werden hergestellt:

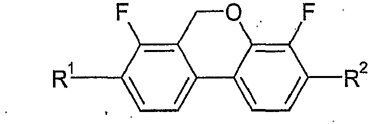

Bemerkung: * 10%-iger Lösung in ZLI-4.792, bzw. ZLI-2857 (Δε) extrapolierte Werte.

| Nr. | R¹ | R² | Phasensequenz T/°C | Δε* | T*(N,I) /°C |
|---|---|---|---|---|---|
| 122 | CH₃ | CH₃ | C 110 I | -3,2 | 27 |
| 123 | CH₃ | C₂H₅ | | | |
| 124 | CH₃ | *n*-C₃H₇ | | | |
| 125 | CH₃ | *n*-C₄H₉ | | | |
| 121 | CH₃ | *n*-C₅H₁₁ | T_{g} -60 C 42 I | -3,1 | 6 |
| 126 | CH₃ | *n*-C₆H₁₃ | | | |
| 127 | CH₃ | *n*-C₇H₁₅ | | | |
| 128 | CH₃ | CH₃O | | | |
| 129 | CH₃ | C₂H₅O | | | |
| 130 | CH₃ | *n*-C₃H₇O | | | |
| 131 | CH₃ | *n*-C₄H₉O | | | |
| 132 | CH₃ | CH₂=CH | | | |
| 133 | CH₃ | *E*-CH₃-CH=CH | | | |
| 134 | CH₃ | CH₂=CH-O | | | |
| 135 | CH₃ | CH₂=CH-CH₂O | | | |
| 136 | C₂H₅ | CH₃ | | | |
| 137 | C₂H₅ | C₂H₅ | | | |
| 138 | C₂H₅ | *n*-C₃H₇ | | | |
| 139 | C₂H₅ | *n*-C₄H₉ | | | |
| 140 | C₂H₅ | *n*-C₅H₁₁ | | | |
| 141 | C₂H₅ | *n*-C₆H₁₃ | | | |
| 142 | C₂H₅ | *n*-C₇H₁₅ | | | |
| 143 | C₂H₅ | CH₃O | | | |
| 144 | C₂H₅ | C₂H₅O | | | |
| 145 | C₂H₅ | *n*-C₃H₇O | | | |
| 146 | C₂H₅ | *n*-C₄H₉O | | | |
| 147 | C₂H₅ | CH₂=CH | | | |
| 148 | C₂H₅ | *E*-CH₃-CH=CH | | | |
| 149 | C₂H₅ | CH₂=CH-O | | | |
| 150 | C₂H₅ | CH₂=CH-CH₂O | | | |
| 151 | *n*-C₃H₇ | CH₃ | | | |
| 152 | *n*-C₃H₇ | C₂H₅ | | | |
| 153 | *n*-C₃H₇ | *n*-C₃H₇ | | | |
| 154 | *n*-C₃H₇ | *n*-C₄H₉ | | | |
| 155 | *n*-C₃H₇ | *n*-C₅H₁₁ | C 34 l | -2,7 | |
| 156 | *n*-C₃H₇ | *n*-C₆H₁₃ | | | |
| 157 | *n*-C₃H₇ | *n*-C₇H₁₅ | | | |
| 158 | *n*-C₃H₇ | CH₃O | | | |
| 159 | *n*-C₃H₇ | C₂H₅O | | | |
| 160 | *n*-C₃H₇ | *n*-C₃H₇O | | | |
| 161 | *n*-C₃H₇ | *n*-C₄H₉O | | | |
| 162 | *n*-C₃H₇ | CH₂=CH | | | |
| 163 | *n*-C₃H₇ | *E*-CH₃-CH=CH | | | |
| 164 | *n*-C₃H₇ | CH₂=CH-O | | | |
| 165 | *n*-C₃H₇ | CH₂=CH-CH₂O | | | |
| 166 | *n*-C₄H₉ | CH₃ | | | |
| 167 | *n*-C₄H₉ | C₂H₅ | | | |
| 168 | *n*-C₄H₉ | *n*-C₃H₇ | | | |
| 169 | *n*-C₄H₉ | *n*-C₄H₉ | | | |
| 170 | *n*-C₄H₉ | *n*-C₅H₁₁ | | | |
| 171 | *n*-C₄H₉ | *n*-C₆H₁₃ | | | |
| 172 | *n*-C₄H₉ | *n*-C₇H₁₅ | | | |
| 173 | *n*-C₄H₉ | CH₃O | | | |
| 174 | *n*-C₄H₉ | C₂H₅O | | | |
| 175 | *n*-C₄H₉ | *n*-C₃H₇O | | | |
| 176 | *n*-C₄H₉ | *n*-C₄H₉O | | | |
| 177 | *n*-C₄H₉ | CH₂=CH | | | |
| 178 | *n*-C₄H₉ | *E*-CH₃-CH=CH | | | |
| 179 | *n*-C₄H₉ | CH₂=CH-O | | | |
| 180a | *n*-C₄H₉ | CH₂=CH-CH₂O | | | |
| 180b | *n*-C₅H₁₁ | *n*-C₄H₉O | | | |
| 181 | CH₃O | CH₃ | | | |
| 182 | CH₃O | C₂H₅ | | | |
| 183 | CH₃O | *n*-C₃H₇ | | | |
| 184 | CH₃O | *n*-C₄H₉ | | | |
| 185 | CH₃O | *n*-C₅H₁₁ | | | |
| 186 | CH₃O | *n*-C₆H₁₃ | | | |
| 187 | CH₃O | *n*-C₇H₁₅ | | | |
| 188 | CH₃O | CH₃O | | | |
| 189 | CH₃O | C₂H₅O | | | |
| 190 | CH₃O | *n*-C₃H₇O | | | |
| 191 | CH₃O | *n*-C₄H₉O | | | |
| 192 | CH₃O | CH₂=CH | | | |
| 193 | CH₃O | *E*-CH₃-CH=CH | | | |
| 194 | CH₃O | CH₂=CH-O | | | |
| 195 | CH₃O | CH₂=CH-CH₂O | | | |
| 196 | C₂H₅O | CH₃ | | | |
| 197 | C₂H₅O | C₂H₅ | | | |
| 198 | C₂H₅O | *n*-C₃H₇ | | | |
| 199 | C₂H₅O | *n*-C₄H₉ | | | |
| 200 | C₂H₅O | *n*-C₅H₁₁ | Tg -39 C 55 N (17,4) I | -6,5 | 39 |
| 201 | C₂H₅O | *n*-C₆H₁₃ | | | |
| 202 | C₂H₅O | *n*-C₇H₁₅ | | | |
| 203 | C₂H₅O | CH₃O | | | |
| 204 | C₂H₅O | C₂H₅O | | | |
| 205 | C₂H₅O | *n*-C₃H₇O | | | |
| 206 | C₂H₅O | *n*-C₄H₉O | | | |
| 207 | C₂H₅O | CH₂=CH | | | |
| 208 | C₂H₅O | *E*-CH₃-CH=CH | | | |
| 209 | C₂H₅O | CH₂=CH-O | | | |
| 210a | C₂H₅O | CH₂=CH-CH₂O | | | |
| 210b | *n*-C₄H₉O | *n*-C₅H₁₁ | | | |
| 211 | CH₂=CH | CH₃ | | | |
| 212 | CH₂=CH | C₂H₅ | | | |
| 213 | CH₂=CH | *n*-C₃H₇ | | | |
| 214 | CH₂=CH | *n*-C₄H₉ | | | |
| 215 | CH₂=CH | *n*-C₅H₁₁ | | | |
| 216 | CH₂=CH | *n*-C₆H₁₃ | | | |
| 217 | CH₂=CH | *n*-C₇H₁₅ | | | |
| 218 | CH₂=CH | CH₃O | | | |
| 219 | CH₂=CH | C₂H₅O | | | |
| 220 | CH₂=CH | *n*-C₃H₇O | | | |
| 221 | CH₂=CH | *n*-C₄H₉O | | | |
| 222 | CH₂=CH | CH₂=CH | | | |
| 223 | CH₂=CH | *E*-CH₃-CH=CH | | | |
| 224 | CH₂=CH | CH₂=CH-O | | | |
| 225 | CH₂=CH | CH₂=CH-CH₂O | | | |
| 226 | CH₂=CH-O | CH₃ | | | |
| 227 | CH₂=CH-O | C₂H₅ | | | |
| 228 | CH₂=CH-O | *n*-C₃H₇ | | | |
| 229 | CH₂=CH-O | *n*-C₄H₉ | | | |
| 230 | CH₂=CH-O | *n*-C₅H₁₁ | | | |
| 231 | CH₂=CH-O | *n*-C₆H₁₃ | | | |
| 232 | CH₂=CH-O | *n*-C₇H₁₅ | | | |
| 233 | CH₂=CH-O | CH₃O | | | |
| 234 | CH₂=CH-O | C₂H₅O | | | |
| 235 | CH₂=CH-O | *n*-C₃H₇O | | | |
| 236 | CH₂=CH-O | *n*-C₄H₉O | | | |
| 237 | CH₂=CH-O | CH₂=CH | | | |
| 238 | CH₂=CH-O | *E*-CH₃-CH=CH | | | |
| 239 | CH₂=CH-O | CH₂=CH-O | | | |
| 240 | CH₂=CH-O | CH₂=CH-CH₂O | | | |

### Referenzbeispiel 241: (8-Ethoxy-4,6,6,7-tetrafluor-3-pentyl-6H-benzo[c]chromen)

### Herstellung von 8-Ethoxy-4,6,6,7-tetrafluor-3-pentyl-6H-benzo[c]chromen

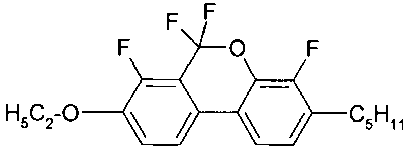

4,00 g (11,5 mmol) der Verbindung des Beispiels 80 und 5,14 g (12,7 mmol) Lawessons Reagenz wurden in 60 ml Chlorbenzol gelöst und 48 h unter Rückfluß erhitzt. Anschließend wurde der Ansatz eingeengt und wie üblich aufgereinigt. Es wurden 2,90 g (8,00 mmol) des entsprechenden Thionolactons, als orangefarbener Feststoff erhalten. Dies entspricht einer Ausbeute von 69 %.

Das Thionolacton wurde in 40 ml Dichlormethan gelöst. Dann wurden 2,1 ml (16,1 mmol) DAST zugegeben und 16 h lang bei ca. 20°C gerührt. Es wurde wie üblich aufgereinigt. Das Rohprodukt wurde mit einer Mischung aus n-Heptan/Essigester (9:1) über Kieselgel aufgereinigt und aus Ethanol umkristallisiert. Es wurden 0,46 g des Difluorbenzochromens erhalten. Dies entspricht einer Ausbeute von 15 %.

### Referenzbeispiele 242 bis 360

Analog zu Beispiel 241 werden hergestellt:

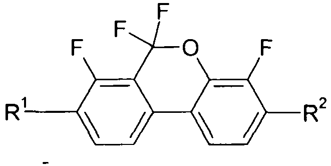

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 242 | CH₃ | CH₃ | | |
| 243 | CH₃ | C₂H₅ | | |
| 244 | CH₃ | *n*-C₃H₇ | | |
| 245 | CH₃ | *n*-C₄H₉ | | |
| 246 | CH₃ | *n*-C₅H₁₁ | | |
| 247 | CH₃ | *n*-C₆H₁₃ | | |
| 248 | CH₃ | *n*-C₇H₁₅ | | |
| 249 | CH₃ | CH₃O | | |
| 250 | CH₃ | C₂H₅O | | |
| 251 | CH₃ | *n*-C₃H₇O | | |
| 252 | CH₃ | *n*-C₄H₉O | | |
| 253 | CH₃ | CH₂=CH | | |
| 254 | CH₃ | *E*-CH₃-CH=CH | | |
| 255 | CH₃ | CH₂=CH-O | | |
| 256 | CH₃ | CH₂=CH-CH₂O | | |
| 257 | C₂H₅ | CH₃ | | |
| 258 | C₂H₅ | C₂H₅ | | |
| 259 | C₂H₅ | *n*-C₃H₇ | | |
| 260 | C₂H₅ | *n*-C₄H₉ | | |
| 261 | C₂H₅ | *n*-C₅H₁₁ | | |
| 262 | C₂H₅ | *n*-C₆H₁₃ | | |
| 263 | C₂H₅ | *n*-C₇H₁₅ | | |
| 264 | C₂H₅ | CH₃O | | |
| 265 | C₂H₅ | C₂H₅O | | |
| 266 | C₂H₅ | *n*-C₃H₇O | | |
| 267 | C₂H₅ | *n*-C₄H₉O | | |
| 268 | C₂H₅ | CH₂=CH | | |
| 269 | C₂H₅ | *E*-CH₃-CH=CH | | |
| 270 | C₂H₅ | CH₂=CH-O | | |
| 271 | C₂H₅ | CH₂=CH-CH₂O | | |
| 272 | *n*-C₃H₇ | CH₃ | | |
| 273 | *n*-C₃H₇ | C₂H₅ | | |
| 274 | *n*-C₃H₇ | *n*-C₃H₇ | | |
| 275 | *n*-C₃H₇ | *n*-C₄H₉ | | |
| 276 | *n*-C₃H₇ | *n*-C₅H₁₁ | C 53 l | -10,4 |
| 277 | *n*-C₃H₇ | *n*-C₆H₁₃ | | |
| 278 | *n*-C₃H₇ | *n*-C₇H₁₅ | | |
| 279 | *n*-C₃H₇ | CH₃O | | |
| 280 | *n*-C₃H₇ | C₂H₅O | | |
| 281 | *n*-C₃H₇ | *n*-C₃H₇O | | |
| 282 | *n*-C₃H₇ | *n*-C₄H₉O | | |
| 283 | *n*-C₃H₇ | CH₂=CH | | |
| 284 | *n*-C₃H₇ | *E*-CH₃-CH=CH | | |
| 285 | *n*-C₃H₇ | CH₂=CH-O | | |
| 286 | *n*-C₃H₇ | CH₂=CH-CH₂O | | |
| 287 | *n*-C₄H₉ | CH₃ | | |
| 288 | *n*-C₄H₉ | C₂H₅ | | |
| 289 | *n*-C₄H₉ | *n*-C₃H₇ | | |
| 290 | *n*-C₄H₉ | *n*-C₄H₉ | | |
| 291 | *n*-C₄H₉ | *n*-C₅H₁₁ | | |
| 292 | *n*-C₄H₉ | *n*-C₆H₁₃ | | |
| 293 | *n*-C₄H₉ | *n*-C₇H₁₅ | | |
| 294 | *n*-C₄H₉ | CH₃O | | |
| 295 | *n*-C₄H₉ | C₂H₅O | | |
| 296 | *n*-C₄H₉ | *n*-C₃H₇O | | |
| 297 | *n*-C₄H₉ | *n*-C₄H₉O | | |
| 298 | *n*-C₄H₉ | CH₂=CH | | |
| 299 | *n*-C₄H₉ | *E*-CH₃-CH=CH | | |
| 300 | *n*-C₄H₉ | CH₂=CH-O | | |
| 301a | *n*-C₄H₉ | CH₂=CH-CH₂O | | |
| 301b | *n*-C₅H₁₁ | *n*-C₄H₉O | | |
| 302 | CH₃O | CH₃ | | |
| 303 | CH₃O | C₂H₅ | | |
| 304 | CH₃O | *n*-C₃H₇ | | |
| 305 | CH₃O | *n*-C₄H₉ | | |
| 306 | CH₃O | *n*-C₅H₁₁ | | |
| 307 | CH₃O | *n*-C₆H₁₃ | | |
| 308 | CH₃O | *n*-C₇H₁₅ | | |
| 309 | CH₃O | CH₃O | | |
| 310 | CH₃O | C₂H₅O | | |
| 311 | CH₃O | *n*-C₃H₇O | | |
| 312 | CH₃O | *n*-C₄H₉O | | |
| 313 | CH₃O | CH₂=CH | | |
| 314 | CH₃O | *E*-CH₃-CH=CH | | |
| 315 | CH₃O | CH₂=CH-O | | |
| 316 | CH₃O | CH₂=CH-CH₂O | | |
| 317 | C₂H₅O | CH₃ | | |
| 318 | C₂H₅O | C₂H₅ | | |
| 319 | C₂H₅O | *n-*C₃H₇ | | |
| 320 | C₂H₅O | *n*-C₄H₉ | | |
| 241 | C₂H₅O | *n*-C₅H₁₁ | C 85 l | -15,4 |
| 321 | C₂H₅O | *n*-C₆H₁₃ | | |
| 322 | C₂H₅O | *n*-C₇H₁₅ | | |
| 323 | C₂H₅O | CH₃O | | |
| 324 | C₂H₅O | C₂H₅O | | |
| 325 | C₂H₅O | *n*-C₃H₇O | | |
| 326 | C₂H₅O | *n*-C₄H₉O | | |
| 327 | C₂H₅O | CH₂=CH | | |
| 328 | C₂H₅O | *E*-CH₃-CH=CH | | |
| 329 | C₂H₅O | CH₂=CH-O | | |
| 330a | C₂H₅O | CH₂=CH-CH₂O | | |
| 330b | *n*-C₄H₉O | *n*-C₅H₁₁ | | |
| 331 | CH₂=CH | CH₃ | | |
| 332 | CH₂=CH | C₂H₅ | | |
| 333 | CH₂=CH | *n*-C₃H₇ | | |
| 334 | CH₂=CH | *n*-C₄H₉ | | |
| 335 | CH₂=CH | *n*-C₅H₁₁ | | |
| 336 | CH₂=CH | *n*-C₆H₁₃ | | |
| 337 | CH₂=CH | *n*-C₇H₁₅ | | |
| 338 | CH₂=CH | CH₃O | | |
| 339 | CH₂=CH | C₂H₅O | | |
| 340 | CH₂=CH | *n*-C₃H₇O | | |
| 341 | CH₂=CH | *n*-C₄H₉O | | |
| 342 | CH₂=CH | CH₂=CH | | |
| 343 | CH₂=CH | *E*-CH₃-CH=CH | | |
| 344 | CH₂=CH | CH₂=CH-O | | |
| 345 | CH₂=CH | CH₂=CH-CH₂O | | |
| 346 | CH₂=CH-O | CH₃ | | |
| 347 | CH₂=CH-O | C₂H₅ | | |
| 348 | CH₂=CH-O | *n*-C₃H₇ | | |
| 349 | CH₂=CH-O | *n*-C₄H₉ | | |
| 350 | CH₂=CH-O | *n*-C₅H₁₁ | | |
| 351 | CH₂=CH-O | *n*-C₆H₁₃ | | |
| 352 | CH₂=CH-O | *n*-C₇H₁₅ | | |
| 353 | CH₂=CH-O | CH₃O | | |
| 354 | CH₂=CH-O | C₂H₅O | | |
| 355 | CH₂=CH-O | *n*-C₃H₇O | | |
| 356 | CH₂=CH-O | *n*-C₄H₉O | | |
| 357 | CH₂=CH-O | CH₂=CH | | |
| 358 | CH₂=CH-O | *E*-CH₃-CH=CH | | |
| 359 | CH₂=CH-O | CH₂=CH-O | | |
| 360 | CH₂=CH-O | CH₂=CH-CH₂O | | |

### Referenzbeispiel 361: (3-Butoxy-4,6,6,7-tetrafluor-8-(4-pentyl-cyclohexyl)-6H-benzo[c]chromen)

### 361.1 Herstellung von 4-Brom-2-fluor-1-(4-pentylcyclohex-1-enyl)benzol

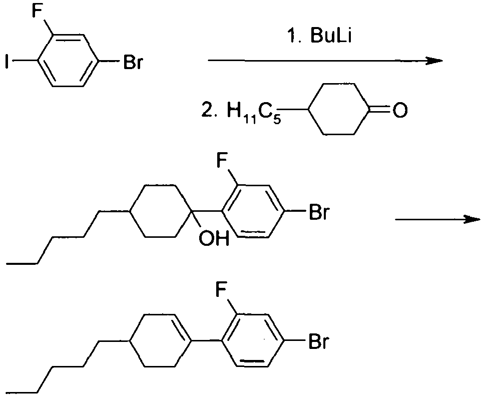

200 g (0,665 mol) 1-Brom-3-fluor-4-iodbenzol wurde in 800 ml Tetrahydrofuran gelöst und bei -70°C wurden 440 ml (0,698 mmol) einer 15-proz. Lösung von n-Butyllithium in Hexan zutropfen gelassen. Nach 30 min gab man eine Lösung von 117 g (0,698 mol) 4-Pentylcyclohexanon in 200 ml Tetrahydrofuran hinzu, ließ 60 min rühren, hydrolysierte mit Wasser und säuerte den Ansatz mit konz. Salzsäure an. Die org. Phase wurde abgetrennt, mit Wasser gewaschen, über Natriumsulfat getrocknet und das Lösungsmittel wurde i. Vak. entfernt. Das Rohprodukt wurde anschließend in 1,4 I Toluol gelöst und nach Zugabe von 6 g Toluolsulfonsäure am Wasserabscheider erhitzt, bis kein Reaktionswasser mehr abgeschieden wurde. Die Lösung wurde mit Wasser gewaschen, eingeengt und der Rückstand wurde mit n-Heptan über Kieselgel filtriert. Man erhielt 124 g (58 %) 4-Brom-2-fluor-1-(4-pentylcyclohex-1-enyl)benzol als gelbes Öl.

### 361.2 Herstellung von.4-Brom-2-fluor-1-(4-pentylcyclohexyl)benzol

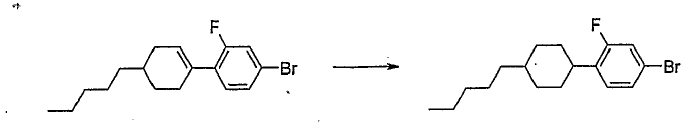

124 g (0,382 mol) 4-Brom-2-fluor-1-(4-pentylcyclohex-1-enyl)benzol wurde in Tetrahydrofuran an Platin-Aktivkohlekatalysator bei 5 bar und 50 °C bis zum Stillstand hydriert. Nach Filtration und Entfernung des Lösungsmittels i. Vak. erhielt man 114 g (80 %) eines Gemisches aus *cis-* und *trans-*4-Brom-2-fluor-1-(4-pentylcyclohexyl)benzol als gelbes Öl. Zur Isomerisierung wurde dieses in 200 ml Dichlormethan gelöst und tropfenweise zu einer Suspension von 12,5 g (95,7 mmol) Aluminiumchlorid in 220 ml Dichlormethan gegeben. Nach 30 min wurden 300 ml Wasser hinzugegeben, die org. Phase abgetrennt, mit Wasser gewaschen und über Natriumsulfat getrocknet. Das Lösungsmittel wurde i. Vak. entfernt und der Rückstand mit n-Pentan über Kieselgel filtriert. Man erhielt 85,2 g Rohprodukt mit einem Gehalt an *trans*-4-Brom-2-fluor-1-(4-pentylcyclohexyl)benzol von 52,8 % und einem Gehalt an *cis*-4-Brom-2-fluor-1-(4-pentylcyclohexyl)benzol von 9,7 % als gelbe Flüssigkeit, die ohne weitere Aufreinigung in die nächste Stufe eingesetzt wurde.

### 361.3 Herstellung von trans- 6-Brom-2-fluor-3-(4-pentylcyclohexyl)-benzoesäure

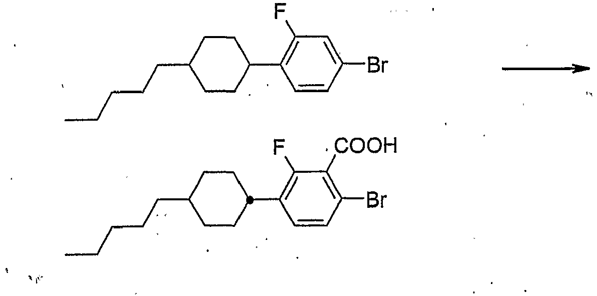

85,2 g (0,163 mol) des Rohproduktes der vorhergehenden Stufe wurden in 400 ml Tetrahydrofuran bei -70°C vorgelegt und eine Lösung von Lithiumdiisopropylamid, hergestellt aus 213 ml 15-proz. *n*-Butyllithium in Hexan und 46 ml (0,.326 mmol) Diisopropylamin in 100 ml Tetrahydrofuran, wurde tropfenweise zugegeben. Nach 1 h wurden 22,9 g (0,521 mol) Kohlendioxid eingeleitet. Der Ansatz wurde auftauen gelassen, mit konz. Salzsäure angesäuert und zweimal mit MTB-Ether extrahiert. Die vereinigten org. Phasen wurden mit Wasser gewaschen, über Natriumsulfat getrocknet und das Lösungsmittel wurde i. Vak. entfernt. Durch Kristallisation aus n-Heptan erhielt man 17 g (28 %) *trans-*6-Brom-2-fluor-3-(4-pentylcyclohexyl)benzoesäure als farblose Kristalle.

### 361.4 Herstellung von trans-6-Brom-2-fluor-3-(4-pentylcyclohexyl)benzoesäuremethylester

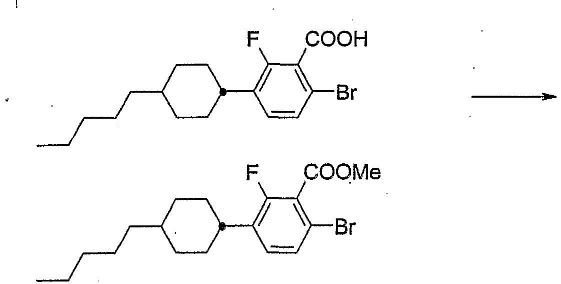

17,1 g (46,0 mmol) *trans*-6-Brom-2-fluor-3-(4-pentylcyclohexyl)benzoesäure wurden in 70 ml Aceton gelöst, 7,66 g (55,4 mmol) Kaliumcarbonat und 3,14 ml (50,6 mmol) Methyliodid hinzugefügt und über Nacht unter Rückfluß erhitzt. Der Ansatz wurde filtriert und das Lösungsmittel abdestilliert. Man erhielt 18 g (100 %) 6-Brom-2-fluor-3-(4-pentylcycloheyxl)benzoesäuremethylester als farbloses Öl, das ohne weitere Aufreinigung weiter umgesetzt wurde.

### 361.5 Herstellung von 1-Brom-4-butoxy-3-fluor-2-(2-methoxy-ethoxymethoxy)-benzol

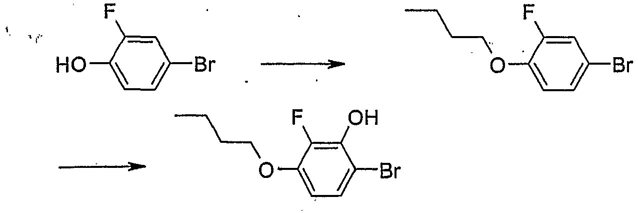

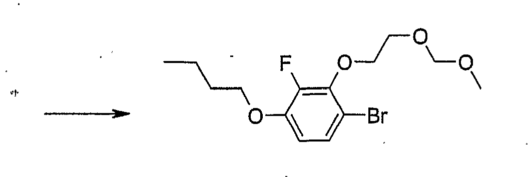

In Analogie zu den Beispielen 841.1 und 841.4 erhielt man aus 4-Brom-2-fluorphenol das 6-Brom-3-butoxy-2-fluorphenol als braunes Öl (80%, 2 Stufen).

77,4 g (0,294 mol) 6-Brom-3-butoxy-2-fluorphenol wurden in 500 ml Dichlormethan gelöst, unter Eiskühlung mit 60,4 ml (0,355 mol) Ethyldiisopropylamin versetzt und nach tropfenweiser Zugabe von 40,3 ml (0,355 mol) 2-Methxoxyethoxymethylchlorid über Nacht bei Raumtemp. rühren gelassen. Der Ansatz wurde mit Wasser hydrolysiert, mit Dichlormethan extrahiert, eingeengt und das Rohprodukt wurde mit *n-*Heptan/MTB-Ether (3:1) über Kieselgel filtriert. Man erhielt 103 g (99 %) 1-Brom-4-butoxy-3-fluor-2-(2-methoxy-ethoxymethoxy)-benzol als gelbes Öl.

### 361.6 Herstellung von 3-Butoxy-4,7-difluor-8-(4-pentyl-cyclohexyl)-benzo[c]chromen-6-on

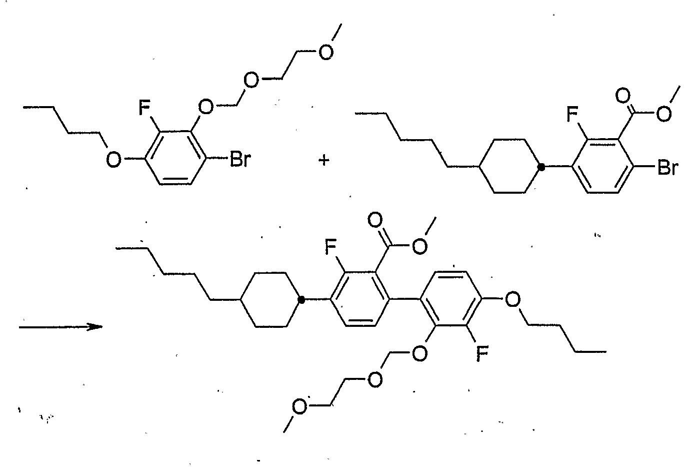

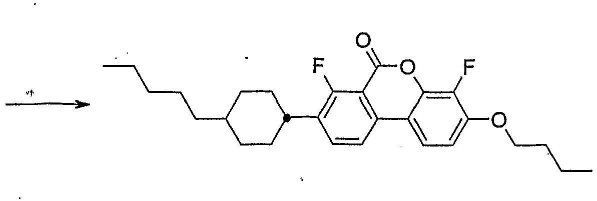

16,4 g (0,046 mol) 1-Brom-4-butoxy-3-fluor-2-(2-methoxy-ethoxymethoxy)-benzol wurden in 20 ml Tetrahydrofuran gelöst und bei -70°C mit 31,6 ml (0,052 mol) einer 15-proz. Lösung von *n*-Butyllithium in Hexan versetzt. Nach Zugabe einer Lösung von 5,94 g (0,027 mol) Zinkbromid in 30 ml Tetrahydrofuran ließ man den Ansatz auftauen und gab die erhaltene Lösung bei Siedetemperatur zu einer Mischung aus 18 g (0,046 mol) 6-Brom-2-fluor-3-(4-pentyl-cyclohexyl)-benzoesäuremethylester und 730 mg (1,00 mmol) Pd(dppf)₂Cl₂ in 60 ml Tetrahydrofuran. Der Ansatz wurde 4 h unter Rückfluß erhitzt, über Nacht bei Raumtemp. rühren gelassen, mit verd. Salzsäure angesäuert und mit MTB-Ether extrahiert. Die vereinigten org. Phasen wurden mit Wasser gewaschen, über Natriumsulfat getrocknet und eingeengt. Das Rohprodukt wurde mit *n*-Pentan/MTB-Ether (3:1) an Kieselgel chromatographiert. Man erhielt 14,6 g (55 %) 4'-Butoxy-3,3'-difluor-2'-(2-methoxy-ethoxymethoxy)-4-(4-pentyl-cyclohexyl)-biphenyl-2-carbonsäuremethylester als gelbes Öl. Dieses wurde in 56 ml Tetrahydrofuran gelöst, mit 11 ml konz. Salzsäure versetzt und über Nacht bei Raumtemp. rühren gelassen. Das ausgefallene Produkt wurde abgesaugt, mit Essigester gewaschen und getrocknet. Man erhielt 7,6 g (68 %) 3-Butoxy-4,7-difluor-8-(4-pentyl-cyclohexyl)-benzo[c]chromen-6-on als farblose Kristalle.

### 361.7 Herstellung von 3-Butoxy-4,7-difluor-8-(4-pentyl-cyclohexyl)-benzo[c]chromen-6-thion

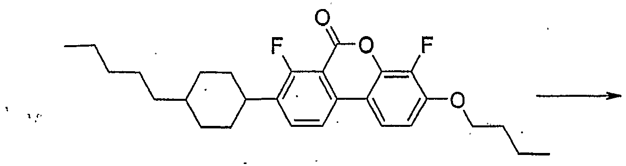

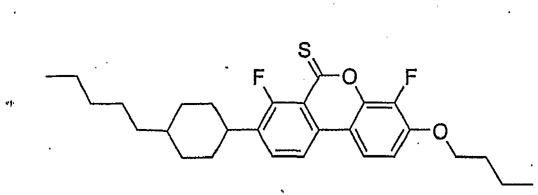

11,7 g (25,6 mmol) 3-Butoxy-4,7-difluor-8-(4-pentyl-cyclohexyl)-benzo[c]chromen-6-on und 11,4 g (28,2 mmol) Lawessons Reagenz wurden 16 h in 130 ml Chlorbenzol unter Rückfluß erhitzt. Die Lösung wurde anschließend über Kieselgel filtriert, eingeengt und das Rohprodukt durch Kristallisation aus MTB-Ether gereinigt. Man erhielt 7,9 g (65 %) 3-Butoxy-4,7-difluor-8-(4-pentyl-cyclohexyl)-benzo[c]chromen-6-thion als gelbe Kristalle.

### 361.8 Herstellung von 3-Butoxy-4,6,6,7-tetrafluor-8-(4-pentyl-cyclohexyl)-6H-benzo[c]chromen

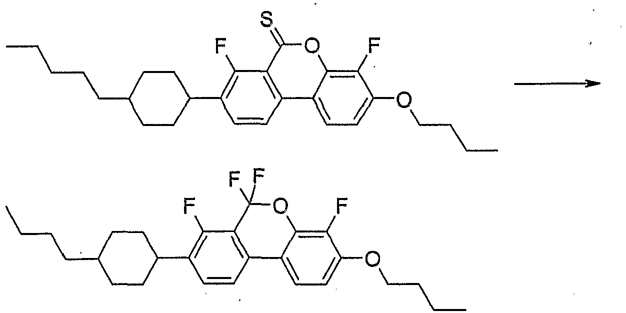

2,00 g (4,18 mmol) 3-Butoxy-4,7-difluor-8-(4-pentyl-cyclohexyl)-benzo[c]chromen-6-thion wurden in 20 ml Dichlormethan gelöst, auf -70°C gekühlt und nach Zugabe von 0,55 ml (20 mmol) einer 65-proz. Lösung von Fluorwasserstoff in Pyridin portionsweise mit einer Suspension von 2,56 g (8,36 mmol) 1,3-Dibrom-5,5-dimethylhydantoin in 12 ml Dichlormethan versetzt. Nach 2 h wurde der Ansatz auftauen gelassen, mit ges. Natriumhydrogencarbonatlsg. neutralisiert und mit Dichlormethan extrahiert. Die org. Phasen wurden mit Wasser gewaschen, über Natriumsulfat getrocknet, eingeengt und das Rohprodukt wurde mit Heptan/Toluol (1:2) an Kieselgel chromatographiert. Kristallisation aus *n-*Heptan lieferte 1,0 g (52 %) 3-Butoxy-4,6,6,7-tetrafluor-8-(4-pentyl-cyclohexyl)-6H-benzo[c]chromen als farblose Kristalle vom Schmp. 99°C.

Die Verbindung zeigte das folgende Phasenverhalten: K 99°C N 130,5°C I und hat einen extrapolieten Klärpunkt von 148°C, sowie bei 20°C eine extrapolierte Doppelbrechung von 0,153 und eine extrapolierte dielektrische Anisotropie von -16,7.

### Referenzbeispiele 361b und 362 bis 390

Analog zu Beispiel 241 und Beispiel 361a werden hergestellt: 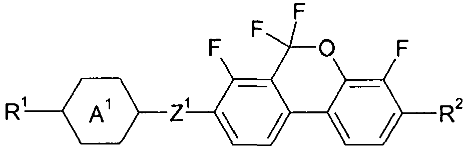 worin 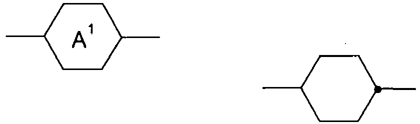 und
- Z¹: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasen sequenz T/°C | Δε* | T*(N,I) /°C |
|---|---|---|---|---|---|
| 361b | CH₃ | CH₃ | | | |
| 362 | CH₃ | C₂H₅ | | | |
| 363 | CH₃ | *n*-C₃H₇ | | | |
| 364 | C₂H₅ | CH₃ | | | |
| 365 | C₂H₅ | C₂H₅ | | | |
| 366 | C₂H₅ | *n*-C₃H₇ | | | |
| 367 | *n*-C₃H₇ | CH₃ | | | |
| 368 | *n*-C₃H₇ | C₂H₅ | | | |
| 369 | *n*-C₃H₇ | *n*-C₃H₇ | | | |
| 370 | *n*-C₃H₇ | *n*-C₅H₁₁ | | | |
| 371 | *n*-C₅H₁₁ | *n*-C₃H₇ | | | |
| 372 | *n*-C₅H₁₁ | *n*-C₅H₁₁ | | | |
| 373 | CH₂=CH | CH₃ | | | |
| 374 | CH₂=CH | C₂H₅ | | | |
| 375 | CH₂=CH | *n*-C₃H₇ | | | |
| 376 | CH₂=CH | CH₂=CH | | | |
| 377 | CH₃ | CH₂=CH | | | |
| 378 | C₂H₅ | CH₂=CH | | | |
| 379 | *n*-C₃H₇ | CH₂=CH | | | |
| 380 | *E*-CH₃-CH=CH | CH₂=CH | | | |
| 381 | *E*-CH₃-CH=CH | *E*-CH₃-CH=CH | | | |
| 382 | CH₃ | CH₃O | | | |
| 383 | CH₃ | C₂H₅O | | | |
| 384 | CH₃ | *n*-C₃H₇O | | | |
| 385 | *n*-C₃H₇ | CH₃O | | | |
| 386 | *n*-C₃H₇ | C₂H₅O | | | |
| 387 | *n*-C₃H₇ | *n*-C₃H₇O | | | |
| 361a | *n*-C₅H₁₁ | *n*-C₄H₉O | C 99 N 130,5 I | -16,7 | 148 |
| 388a | *n*-C₄H₉O | *n*-C₅H₁₁ | | | |
| 388b | CH₃O | CH₃O | | | |
| 389 | C₂H₅O | C₂H₅O | | | |
| 390 | *n*-C₃H₇O | *n*-C₃H₇O | | | |

### Referenzbeispiele 391 bis 420

Analog zu Beispiel 241 und Beispiel 361a werden hergestellt: 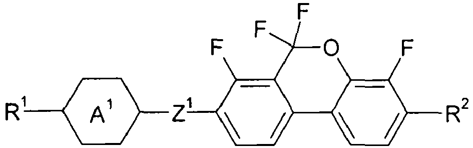 worin 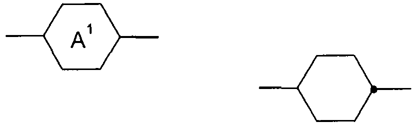 und
- Z¹: -CF₂-O-
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 391 | CH₃ | CH₃ | | |
| 392 | CH₃ | C₂H₅ | | |
| 393 | CH₃ | *n*-C₃H₇ | | |
| 394 | C₂H₅ | CH₃ | | |
| 395 | C₂H₅ | C₂H₅ | | |
| 396 | C₂H₅ | *n*-C₃H₇ | | |
| 397 | *n*-C₃H₇ | CH₃ | | |
| 398 | *n*-C₃H₇ | C₂H₅ | | |
| 399 | *n*-C₃H₇ | *n*-C₃H₇ | | |
| 400 | *n*-C₃H₇ | *n*-C₅H₁₁ | | |
| 401 | *n*-C₅H₁₁ | *n*-C₃H₇ | | |
| 402 | *n*-C₅H₁₁ | *n*-C₅H₁₁ | | |
| 403 | CH₂=CH | CH₃ | | |
| 404 | CH₂=CH | C₂H₅ | | |
| 405 | CH₂=CH | *n*-C₃H₇ | | |
| 406 | CH₂=CH | CH₂=CH | | |
| 407 | CH₃ | CH₂=CH | | |
| 408 | C₂H₅ | CH₂=CH | | |
| 409 | *n*-C₃H₇ | CH₂=CH | | |
| 410 | *E*-CH₃-CH=CH | CH₂=CH | | |
| 411 | *E*-CH₃-CH=CH | *E*-CH₃-CH=CH | | |
| 412 | CH₃ | CH₃O | | |
| 413 | CH₃ | C₂H₅O | | |
| 414 | CH₃ | *n*-C₃H₇O | | |
| 415 | *n*-C₃H₇ | CH₃O | | |
| 416 | *n*-C₃H₇ | C₂H₅O | | |
| 417a | *n*-C₃H₇ | *n*-C₃H₇O | | |
| 417b | *n*-C₅H₁₁ | *n*-C₄H₉O | | |
| 417c | *n*-C₄H₉O | *n*-C₅H₁₁ | | |
| 418 | CH₃O | CH₃O | | |
| 419 | C₂H₅O | C₂H₅O | | |
| 420 | *n*-C₃H₇O | *n*-C₃H₇O | | |

### Referenzbeispiele 421 bis 450

Analog zu Beispiel 241 und Beispiel 361a werden hergestellt: 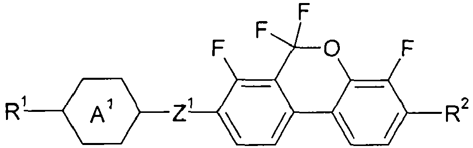 worin 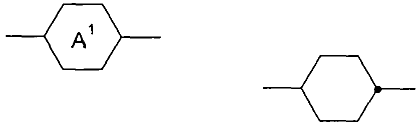 und
- Z¹: -CH₂-O-
bedeutet.

Bemerkung: * aus 10 %-iger Lösung in ZLI-4792 extrapolierte Werte.

| Nr. | R¹ | R² | Phasensequenz Δε* T/°C |
|---|---|---|---|
| 421 | CH₃ | CH₃ | |
| 422 | CH₃ | C₂H₅ | |
| 423 | CH₃ | *n*-C₃H₇ | |
| 424 | C₂H₅ | CH₃ | |
| 425 | C₂H₅ | C₂H₅ | |
| 426 | C₂H₅ | *n*-C₃H₇ | |
| 427 | *n*-C₃H₇ | CH₃ | |
| 428 | *n*-C₃H₇ | C₂H₅ | |
| 429 | *n*-C₃H₇ | *n*-C₃H₇ | |
| 430 | *n*-C₃H₇ | *n*-C₅H₁₁ | |
| 431 | *n*-C₅H₁₁ | *n*-C₃H₇ | |
| 432 | *n*-C₅H₁₁ | *n*-C₅H₁₁ | |
| 453 | CH₂=CH | CH₃ | |
| 434 | CH₂=CH | C₂H₅ | |
| 435 | CH₂=CH | *n*-C₃H₇ | |
| 436 | CH₂=CH | CH₂=CH | |
| 437 | CH₃ | CH₂=CH | |
| 438 | C₂H₅ | CH₂=CH | |
| 439 | *n*-C₃H₇ | CH₂=CH | |
| 440 | *E*-CH₃-CH=CH | CH₂=CH | |
| 441 | *E*-CH₃-CH=CH | *E*-CH₃-CH=CH | |
| 442 | CH₃ | CH₃O | |
| 443 | CH₃ | C₂H₅O | |
| 444 | CH₃ | *n*-C₃H₇O | |
| 445 | *n*-C₃H₇ | CH₃O | |
| 446 | *n*-C₃H₇ | C₂H₅O | |
| 447a | *n*-C₃H₇ | *n*-C₃H₇O | |
| 447b | *n*-C₅H₁₁ | *n*-C₄H₉O | |
| 447c | *n*-C₄H₉O | *n*-C₅H₁₁ | |
| 448 | CH₃O | CH₃O | |
| 449 | C₂H₅O | C₂H₅O | |
| 450 | *n*-C₃H₇O | *n*-C₃H₇O | |

### Referenzbeispiele 451 bis 480

Analog zu Beispiel 241 und Beispiel 361a werden hergestellt: 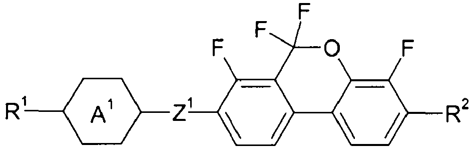 worin 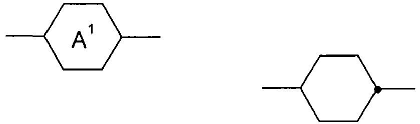 und
- Z¹: -CF=CF-
bedeutet.

Bemerkung: * 10%-iger Lösung in ZLI-4792, bzw. ZLI-2857 (Δε) extrapolierte Werte.

| Nr. | R¹ | R² | Phasensequenz Δε* T/°C |
|---|---|---|---|
| 451 | CH₃ | CH₃ | |
| 452 | CH₃ | C₂H₅ | |
| 453 | CH₃ | *n*-C₃H₇ | |
| 454 | C₂H₅ | CH₃ | |
| 455 | C₂H₅ | C₂H₅ | |
| 456 | C₂H₅ | *n*-C₃H₇ | |
| 457 | *n*-C₃H₇ | CH₃ | |
| 458 | *n*-C₃H₇ | C₂H₅ | |
| 459 | *n*-C₃H₇ | *n*-C₃H₇ | |
| 460 | *n*-C₃H₇ | *n*-C₅H₁₁ | |
| 461 | *n*-C₅H₁₁ | *n*-C₃H₇ | |
| 462 | *n*-C₅H₁₁ | *n*-C₅H₁₁ | |
| 463 | CH₂=CH | CH₃ | |
| 464 | CH₂=CH | C₂H₅ | |
| 465 | CH₂=CH | *n*-C₃H₇ | |
| 466 | CH₂=CH | CH₂=CH | |
| 467 | CH₃ | CH₂=CH | |
| 468 | C₂H₅ | CH₂=CH | |
| 469 | *n*-C₃H₇ | CH₂=CH | |
| 470 | *E*-CH₃-CH=CH | CH₂=CH | |
| 471 | *E*-CH₃-CH=CH | *E*-CH₃-CH=CH | |
| 472 | CH₃ | CH₃O | |
| 473 | CH₃ | C₂H₅O | |
| 474 | CH₃ | *n*-C₃H₇O | |
| 475 | *n*-C₃H₇ | CH₃O | |
| 476 | *n*-C₃H₇ | C₂H₅O | |
| 477a | *n*-C₃H₇ | *n*-C₃H₇O | |
| 477b | *n*-C₅H₁₁ | *n*-C₄H₉O | |
| 477c | *n*-C₄H₉O | *n*-C₅H₁₁ | |
| 478 | CH₃O | CH₃O | |
| 479 | C₂H₅O | C₂H₅O | |
| 480 | *n*-C₃H₇O | *n*-C₃H₇O | |

### Referenzbeispiele 481 bis 510

Analog zu Beispiel 241 und Beispiel 361a werden hergestellt: 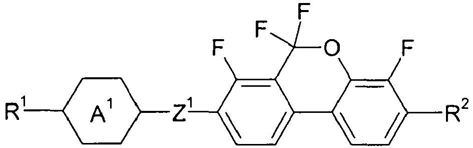 worin 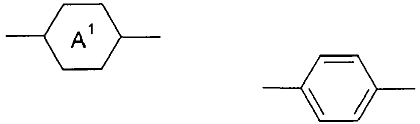 und
- Z¹: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz Δε* T/°C |
|---|---|---|---|
| 481 | CH₃ | CH₃ | |
| 482 | CH₃ | C₂H₅ | |
| 483 | CH₃ | *n*-C₃H₇ | |
| 484 | C₂H₅ | CH₃ | |
| 485 | C₂H₅ | C₂H₅ | |
| 486 | C₂H₅ | *n*-C₃H₇ | |
| 487 | *n*-C₃H₇ | CH₃ | |
| 488 | *n*-C₃H₇ | C₂H₅ | |
| 489 | *n*-C₃H₇ | *n*-C₃H₇ | |
| 490 | *n*-C₃H₇ | *n*-C₅H₁₁ | |
| 491 | *n*-C₅H₁₁ | *n*-C₃H₇ | |
| 492 | *n*-C₅H₁₁ | *n*-C₅H₁₁ | |
| 493 | CH₂=CH | CH₃ | |
| 494 | CH₂=CH | C₂H₅ | |
| 495 | CH₂=CH | *n*-C₃H₇ | |
| 496 | CH₂=CH | CH₂=CH | |
| 497 | CH₃ | CH₂=CH | |
| 498 | C₂H₅ | CH₂=CH | |
| 499 | *n*-C₃H₇ | CH₂=CH | |
| 500 | *E*-CH₃-CH=CH | CH₂=CH | |
| 501 | *E*-CH₃-CH=CH | *E*-CH₃-CH=CH | |
| 502 | CH₃ | CH₃O | |
| 503 | CH₃ | C₂H₅O | |
| 504 | CH₃ | *n*-C₃H₇O | |
| 505 | *n*-C₃H₇ | CH₃O | |
| 506 | *n*-C₃H₇ | C₂H₅O | |
| 507a | *n*-C₃H₇ | *n*-C₃H₇O | |
| 507b | *n*-C₅H₁₁ | *n*-C₄H₉O | |
| 507c | *n*-C₄H₉O | *n*-C₅H₁₁ | |
| 508 | CH₃O | CH₃O | |
| 509 | C₂H₅O | C₂H₅O | |
| 510 | *n*-C₃H₇O | *n*-C₃H₇O | |

### Referenzbeispiele 511 bis 540

Analog zu Beispiel 241 und Beispiel 361a werden hergestellt: 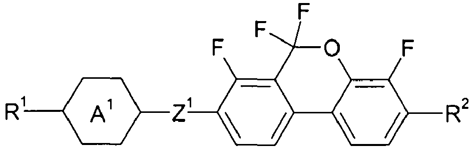 worin 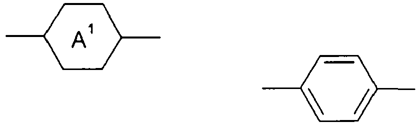 und
- Z¹: -CF₂-O-
bedeutet.

Bemerkung: * 10%-iger Lösung in ZLI-4792, bzw. ZLI-2857 (Δε) extrapolierte Werte.

| Nr. | R¹ | R² | Phasensequenz Δε* T/°C |
|---|---|---|---|
| 511 | CH₃ | CH₃ | |
| 512 | CH₃ | C₂H₅ | |
| 513 | CH₃ | *n*-C₃H₇ | |
| 514 | C₂H₅ | CH₃ | |
| 515 | C₂H₅ | C₂H₅ | |
| 516 | C₂H₅ | *n*-C₃H₇ | |
| 517 | *n*-C₃H₇ | CH₃ | |
| 518 | *n*-C₃H₇ | C₂H₅ | |
| 519 | *n*-C₃H₇ | *n*-C₃H₇ | |
| 520 | *n*-C₃H₇ | *n*-C₅H₁₁ | |
| 521 . | *n*-C₅H₁₁ | *n*-C₃H₇ | |
| 522 | *n*-C₅H₁₁ | *n*-C₅H₁₁ | |
| 523 | CH₂=CH | CH₃ | |
| 524 | CH₂=CH | C₂H₅ | |
| 525 | CH₂=CH | *n*-C₃H₇ | |
| 526 | CH₂=CH | CH₂=CH | |
| 527 | CH₃ | CH₂=CH | |
| 528 | C₂H₅ | CH₂=CH | |
| 529 | *n*-C₃H₇ | CH₂=CH | |
| 530 | *E*-CH₃-CH=CH | CH₂=CH | |
| 531 | *E*-CH₃-CH=CH | *E*-CH₃-CH=CH | |
| 532 | CH₃ | CH₃O | |
| 533 | CH₃ | C₂H₅O | |
| 534 | CH₃ | *n*-C₃H₇O | |
| 535 | *n*-C₃H₇ | CH₃O | |
| 536 | *n*-C₃H₇ | C₂H₅O | |
| 537a | *n*-C₃H₇ | *n*-C₃H₇O | |
| 537b | *n*-C₅H₁₁ | *n*-C₄H₉O | |
| 537c | *n*-C₄H₉O | *n*-C₅H₁₁ | |
| 538 | CH₃O | CH₃O | |
| 539 | C₂H₅O | C₂H₅O | |
| 540 | *n*-C₃H₇O | *n*-C₃H₇O | |

### Referenzbeispiele 541 bis 570

Analog zu Beispiel 241 und Beispiel 361a werden hergestellt: 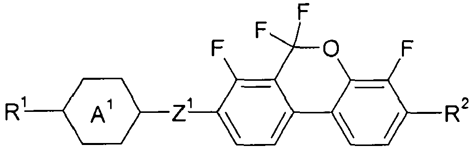 worin 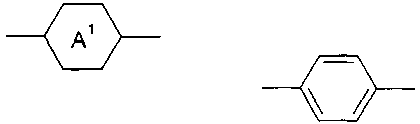 und
- Z¹: -CH₂-O-
bedeutet.

Bemerkung: * 10%-iger Lösung in ZLI-4792, bzw. ZLI-2857 (Δε) extrapolierte Werte.

| Nr. | R¹ | R² | Phasensequenz Δε* T/°C |
|---|---|---|---|
| 541 | CH₃ | CH₃ | |
| 542 | CH₃ | C₂H₅ | |
| 543 | CH₃ | *n*-C₃H₇ | |
| 544 | C₂H₅ | CH₃ | |
| 545 | C₂H₅ | C₂H₅ | |
| 546 | C₂H₅ | *n*-C₃H₇ | |
| 547 | *n*-C₃H₇ | CH₃ | |
| 548 | *n*-C₃H₇ | C₂H₅ | |
| 549 | *n*-C₃H₇ | *n*-C₃H₇ | |
| 550 | *n*-C₃H₇ | *n*-C₅H₁₁ | |
| 551 | *n*-C₅H₁₁ | *n*-C₃H₇ | |
| 552 | *n*-C₅H₁₁ | *n*-C₅H₁₁ | |
| 553 | CH₂=CH | CH₃ | |
| 554 | CH₂=CH | C₂H₅ | |
| 555 | CH₂=CH | *n*-C₃H₇ | |
| 556 | CH₂=CH | CH₂=CH | |
| 557 | CH₃ | CH₂=CH | |
| 558 | C₂H₅ | CH₂=CH | |
| 559 | *n*-C₃H₇ | CH₂=CH | |
| 560 | *E*-CH₃-CH=CH | CH₂=CH | |
| 561 | *E*-CH₃-CH=CH | *E*-CH₃-CH=CH | |
| 562 | CH₃ | CH₃O | |
| 563 | CH₃ | C₂H₅O | |
| 564 | CH₃ | *n*-C₃H₇O | |
| 565 | *n*-C₃H₇ | CH₃O | |
| 566 | *n*-C₃H₇ | C₂H₅O | |
| 567a | *n*-C₃H₇ | *n*-C₃H₇O | |
| 567b | *n*-C₅H₁₁ | *n*-C₄H₉O | |
| 567c | *n*-C₄H₉O | *n*-C₅H₁₁ | |
| 568 | CH₃O | CH₃O | |
| 569 | C₂H₅O | C₂H₅O | |
| 570 | *n*-C₃H₇O | *n*-C₃H₇O | |

### Referenzbeispiele 571 bis 600

Analog zu Beispiel 241 und Beispiel 361a werden hergestellt: 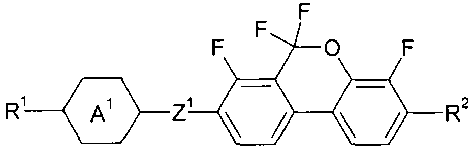 worin 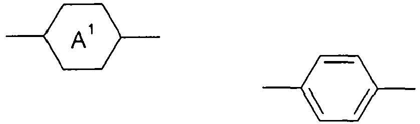 und
- Z¹: -CF=CF-
bedeutet.

Bemerkung: * 10%-iger Lösung in ZLI-4792, bzw. ZLI-2857 (Δε) extrapolierte Werte.

| Nr. | R¹ | R² | Phasensequenz Δε* T/°C |
|---|---|---|---|
| 571 | CH₃ | CH₃ | |
| 572 | CH₃ | C₂H₅ | |
| 573 | CH₃ | *n*-C₃H₇ | |
| 574 | C₂H₅ | CH₃ | |
| 575 | C₂H₅ | C₂H₅ | |
| 576 | C₂H₅ | *n*-C₃H₇ | |
| 577 | *n*-C₃H₇ | CH₃ | |
| 578 | *n*-C₃H₇ | C₂H₅ | |
| 579 | *n*-C₃H₇ | *n*-C₃H₇ | |
| 580 | *n*-C₃H₇ | *n*-C₅H₁₁ | |
| 581 . | *n*-C₅H₁₁ | *n*-C₃H₇ | |
| 582 | *n*-C₅H₁₁ | *n*-C₅H₁₁ | |
| 583 | CH₂=CH | CH₃ | |
| 584 | CH₂=CH | C₂H₅ | |
| 585 | CH₂=CH | *n*-C₃H₇ | |
| 586 | CH₂=CH | CH₂=CH | |
| 587 | CH₃ | CH₂=CH | |
| 588 | C₂H₅ | CH₂=CH | |
| 589 | *n*-C₃H₇ | CH₂=CH | |
| 590 | *E*-CH₃-CH=CH | CH₂=CH | |
| 591 | *E*-CH₃-CH=CH | *E*-CH₃-CH=CH | |
| 592 | CH₃ | CH₃O | |
| 593 | CH₃ | C₂H₅O | |
| 594 | CH₃ | *ₙ*-C₃H₇O | |
| 595 | *n*-C₃H₇ | CH₃O | |
| 596 | *n*-C₃H₇ | C₂H₅O | |
| 597a | *n*-C₃H₇ | *n*-C₃H₇O | |
| 597b | *n*-C₅H₁₁ | *n*-C₄H₉O | |
| 597c | *n*-C₄H₉O | *n*-C₅H₁₁ | |
| 598 | CH₃O | CH₃O | |
| 599 | C₂H₅O | C₂H₅O | |
| 600 | *n*-C₃H₇O | *n*-C₃H₇O | |

### Referenzbeispiele 601 bis 630

Analog zu Beispiel 241 und Beispiel 361a werden hergestellt: 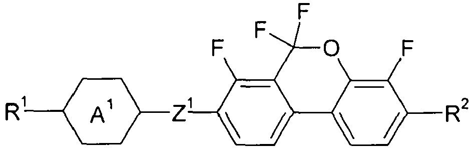 worin 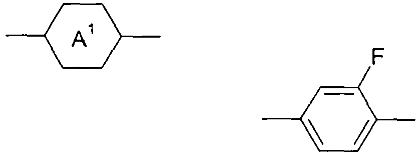 und
- Z¹: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz Δε* T/°C |
|---|---|---|---|
| 601 | CH₃ | CH₃ | |
| 602 | CH₃ | C₂H₅ | |
| 603 | CH₃ | *n*-C₃H₇ | |
| 604 | C₂H₅ | CH₃ | |
| 605 | C₂H₅ | C₂H₅ | |
| 606 | C₂H₅ | *n*-C₃H₇ | |
| 607 | *n*-C₃H₇ | CH₃ | |
| 608 | *n*-C₃H₇ | C₂H₅ | |
| 609 | *n*-C₃H₇ | *n*-C₃H₇ | |
| 610 | *n*-C₃H₇ | *n*-C₅H₁₁ | |
| 611 . | *n*-C₅H₁₁ | *n*-C₃H₇ | |
| 612 | *n*-C₅H₁₁ | *n*-C₅H₁₁ | |
| 613 | CH₂=CH | CH₃ | |
| 614 | CH₂=CH | C₂H₅ | |
| 615 | CH₂=CH | *n*-C₃H₇ | |
| 616 | CH₂=CH | CH₂=CH | |
| 617 | CH₃ | CH₂=CH | |
| 618 | C₂H₅ | CH₂=CH | |
| 619 | *n*-C₃H₇ | CH₂=CH | |
| 620 | *E*-CH₃-CH=CH | CH₂=CH | |
| 621 | *E*-CH₃-CH=CH | *E*-CH₃-CH=CH | |
| 622 | CH₃ | CH₃O | |
| 623 | CH₃ | C₂H₅O | |
| 624 | CH₃ | *n*-C₃H₇O | |
| 625 | *n*-C₃H₇ | CH₃O | |
| 626 | *n*-C₃H₇ | C₂H₅O | |
| 627a | *n*-C₃H₇ | *n*-C₃H₇O | |
| 627b | *n*-C₅H₁₁ | *n*-C₄H₉O | |
| 527c | *n*-C₄H₉O | *n*-C₅H₁₁ | |
| 628 | CH₃O | CH₃O | |
| 629 | C₂H₅O | C₂H₅O | |
| 630 | *n*-C₃H₇O | *n*-C₃H₇O | |

### Referenzbeispiele 631 bis 660

Analog zu Beispiel 241 und Beispiel 361a werden hergestellt: 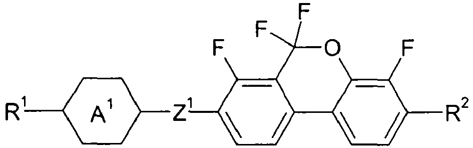 worin 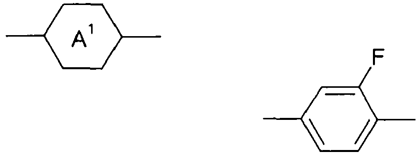 und
- Z¹: -CF₂-O-
bedeutet.

Bemerkung: * 10%-iger Lösung in ZLI-4792, bzw. ZLI-2857 (Δε) extrapolierte Werte.

| Nr. | R¹ | R² | Phasensequenz Δε* T/°C |
|---|---|---|---|
| 631 | CH₃ | CH₃ | |
| 632 | CH₃ | C₂H₅ | |
| 633 | CH₃ | *n*-C₃H₇ | |
| 634 | C₂H₅ | CH₃ | |
| 635 | C₂H₅ | C₂H₅ | |
| 636 | C₂H₅ | *n*-C₃H₇ | |
| 637 | *n*-C₃H₇ | CH₃ | |
| 638 | *n*-C₃H₇ | C₂H₅ | |
| 639 | *n*-C₃H₇ | *n*-C₃H₇ | |
| 640 | *n*-C₃H₇ | *n*-C₅H₁₁ | |
| 641 | *n*-C₅H₁₁ | *n*-C₃H₇ | |
| 642 | *n*-C₅H₁₁ | *n*-C₅H₁₁ | |
| 643 | CH₂=CH | CH₃ | |
| 644 | CH₂=CH | C₂H₅ | |
| 645 | CH₂=CH | *n*-C₃H₇ | |
| 646 | CH₂=CH | CH₂=CH | |
| 677 | CH₃ | CH₂=CH | |
| 648 | C₂H₅ | CH₂=CH | |
| 649 | *n*-C₃H₇ | CH₂=CH | |
| 650 | *E*-CH₃-CH=CH | CH₂=CH | |
| 651 | *E*-CH₃-CH=CH | *E*-CH₃-CH=CH | |
| 652 | CH₃ | CH₃O | |
| 653 | CH₃ | C₂H₅O | |
| 654 | CH₃ | *n*-C₃H₇O | |
| 655 | *n*-C₃H₇ | CH₃O | |
| 656 | *n*-C₃H₇ | C₂H₅O | |
| 657a | *n*-C₃H₇ | *n*-C₃H₇O | |
| 657b | *n*-C₅H₁₁ | *n*-C₄H₉O | |
| 5617c | *n*-C₄H₉O | *n*-C₅H₁₁ | |
| 658 | CH₃O | CH₃O | |
| 659 | C₂H₅O | C₂H₅O | |
| 660 | *n*-C₃H₇O | *ₙ*-C₃H₇O | |

### Referenzbeispiele 661 bis 690

Analog zu Beispiel 241 und Beispiel 361a werden hergestellt: 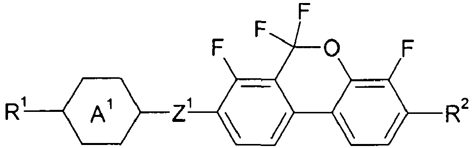 worin 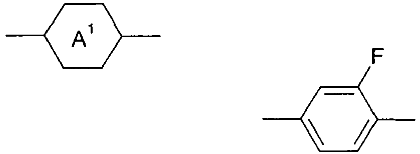 und
- Z¹: -CH₂-O-
bedeutet.

Bemerkung: * 10%-iger Lösung in ZLI-4792, bzw. ZLI-2857 (Δε) extrapolierte Werte.

| Nr. | R¹ | R² | Phasensequenz Δε* T/°C |
|---|---|---|---|
| 661 | CH₃ | CH₃ | |
| 662 | CH₃ | C₂H₅ | |
| 663 | CH₃ | *n*-C₃H₇ | |
| 664 | C₂H₅ | CH₃ | |
| 665 | C₂H₅ | C₂H₅ | |
| 666 | C₂H₅ | *n*-C₃H₇ | |
| 667 | *n*-C₃H₇ | CH₃ | |
| 668 | *n*-C₃H₇ | C₂H₅ | |
| 669 | *n*-C₃H₇ | *n*-C₃H₇ | |
| 670 | *n*-C₃H₇ | *n*-C₅H₁₁ | |
| 671 | *n*-C₅H₁₁ | *n*-C₃H₇ | |
| 672 | *n*-C₅H₁₁ | *n*-C₅H₁₁ | |
| 673 | CH₂=CH | CH₃ | |
| 674 | CH₂=CH | C₂H₅ | |
| 675 | CH₂=CH | *n*-C₃H₇ | |
| 676 | CH₂=CH | CH₂=CH | |
| 677 | CH₃ | CH₂=CH | |
| 678 | C₂H₅ | CH₂=CH | |
| 679 | *n*-C₃H₇ | CH₂=CH | |
| 680 | *E*-CH₃-CH=CH | CH₂=CH | |
| 681 | *E*-CH₃-CH=CH | *E*-CH₃-CH=CH | |
| 682 | CH₃ | CH₃O | |
| 683 | CH₃ | C₂H₅O | |
| 684 | CH₃ | *n*-C₃H₇O | |
| 685 | *n*-C₃H₇ | CH₃O | |
| 686 | *n*-C₃H₇ | C₂H₅O | |
| 687a | *n*-C₃H₇ | *n*-C₃H₇O | |
| 687b | *n*-C₅H₁₁ | *n*-C₄H₉O | |
| 687c | *n*-C₄H₉O | *n*-C₅H₁₁ | |
| 688 | CH₃O | CH₃O | |
| 689 | C₂H₅O | C₂H₅O | |
| 690 | *n*-C₃H₇O | *n*-C₃H₇O | |

### Referenzbeispiele 691 bis 720

Analog zu Beispiel 241und Beispiel 361a werden hergestellt: 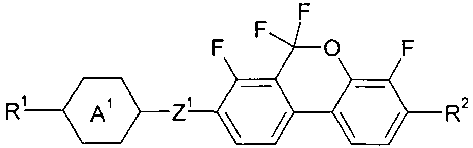 worin 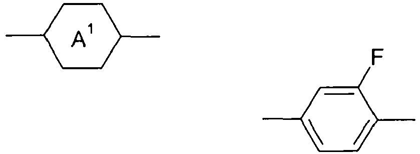 und
- Z¹: -CF=CF-
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 691 | CH₃ | CH₃ | | |
| 692 | CH₃ | C₂H₅ | | |
| 693 | CH₃ | *n*-C₃H₇ | | |
| 694 | C₂H₅ | CH₃ | | |
| 695 | C₂H₅ | C₂H₅ | | |
| 696 | C₂H₅ | *n*-C₃H₇ | | |
| 697 | *n*-C₃H₇ | CH₃ | | |
| 698 | *n*-C₃H₇ | C₂H₅ | | |
| 699 | *n*-C₃H₇ | *n*-C₃H₇ | | |
| 700 | *n*-C₃H₇ | *n*-C₅H₁₁ | | |
| 701 | *n*-C₅H₁₁ | *n*-C₃H₇ | | |
| 702 | *n*-C₅H₁₁ | *n*-C₅H₁₁ | | |
| 703 | CH₂=CH | CH₃ | | |
| 704 | CH₂=CH | C₂H₅ | | |
| 705 | CH₂=CH | *n*-C₃H₇ | | |
| 706 | CH₂=CH | CH₂=CH | | |
| 707 | CH₃ | CH₂=CH | | |
| 708 | C₂H₅ | CH₂=CH | | |
| 709 | *n*-C₃H₇ | CH₂=CH | | |
| 710 | *E*-CH₃-CH=CH | CH₂=CH | | |
| 711 | *E*-CH₃-CH=CH | *E*-CH₃-CH=CH | | |
| 712 | CH₃ | CH₃O | | |
| 713 | CH₃ | C₂H₅O | | |
| 714 | CH₃ | *n*-C₃H₇O | | |
| 715 | *n*-C₃H₇ | CH₃O | | |
| 716 | *n*-C₃H₇ | C₂H₅O | | |
| 717a | *n*-C₃H₇ | *n*-C₃H₇O | | |
| 717b | *n*-C₅H₁₁ | *n*-C₄H₉O | | |
| 717c | *n*-C₄H₉O | *n*-C₅H₁₁ | | |
| 718 | CH₃O | CH₃O | | |
| 719 | C₂H₅O | C₂H₅O | | |
| 720 | *n*-C₃H₇O | *n*-C₃H₇O | | |

### Referenzbeispiele 721 bis 750

Analog zu Beispiel 241und Beispiel 361a werden hergestellt: 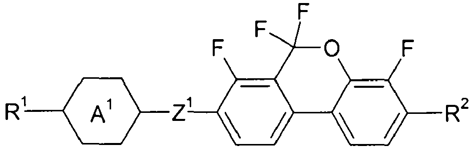 worin 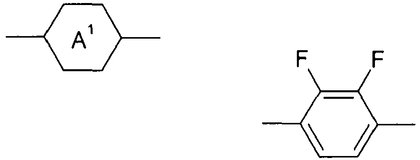 und
- Z¹: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 721 | CH₃ | CH₃ | | |
| 722 | CH₃ | C₂H₅ | | |
| 723 | CH₃ | *n*-C₃H₇ | | |
| 724 | C₂H₅ | CH₃ | | |
| 725 | C₂H₅ | C₂H₅ | | |
| 726 | C₂H₅ | *n*-C₃H₇ | | |
| 727 | *n*-C₃H₇ | CH₃ | | |
| 728 | *n*-C₃H₇ | C₂H₅ | | |
| 729 | *n*-C₃H₇ | *n*-C₃H₇ | | |
| 730 | *n*-C₃H₇ | *n*-C₅H₁₁ | | |
| 731 | *n*-C₅H₁₁ | *n*-C₃H₇ | | |
| 732 | *n*-C₅H₁₁ | *n*-C₅H₁₁ | | |
| 733 | CH₂=CH | CH₃ | | |
| 734 | CH₂=CH | C₂H₅ | | |
| 735 | CH₂=CH | *n*-C₃H₇ | | |
| 736 | CH₂=CH | CH₂=CH | | |
| 737 | CH₃ | CH₂=CH | | |
| 738 | C₂H₅ | CH₂=CH | | |
| 739 | *n*-C₃H₇ | CH₂=CH | | |
| 740 | *E*-CH₃-CH=CH | CH₂=CH | | |
| 741 | *E*-CH₃-CH=CH | *E*-CH₃-CH=CH | | |
| 742 | CH₃ | CH₃O | | |
| 743 | CH₃ | C₂H₅O | | |
| 744 | CH₃ | *n*-C₃H₇O | | |
| 745 | *n*-C₃H₇ | CH₃O | | |
| 746 | *n*-C₃H₇ | C₂H₅O | | |
| 747a | *n*-C₃H₇ | *n*-C₃H₇O | | |
| 747b | *n*-C₅H₁₁ | *n*-C₄H₉O | | |
| 747c | *n*-C₄H₉O | *n*-C₅H₁₁ | | |
| 748 | CH₃O | CH₃O | | |
| 749 | C₂H₅O | C₂H₅O | | |
| 750 | *n*-C₃H₇O | *n*-C₃H₇O | | |

### Referenzbeispiele 751 bis 780

Analog zu Beispiel 241und Beispiel 361a werden hergestellt: 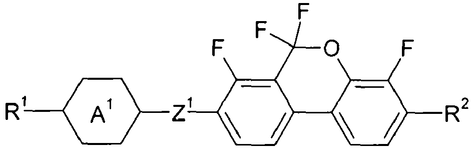 worin 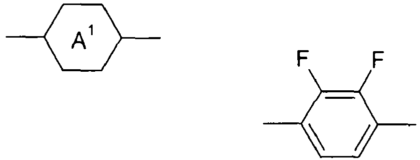 und
- Z¹: -CF₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C. | Δε* |
|---|---|---|---|---|
| 751 | CH₃ | CH₃ | | |
| 752 | CH₃ | C₂H₅ | | |
| 753 | CH₃ | *n*-C₃H₇ | | |
| 754 | C₂H₅ | CH₃ | | |
| 755 | C₂H₅ | C₂H₅ | | |
| 756 | C₂H₅ | *n*-C₃H₇ | | |
| 757 | *n*-C₃H₇ | CH₃ | | |
| 758 | *n*-C₃H₇ | C₂H₅ | | |
| 759 | *n*-C₃H₇ | *n*-C₃H₇ | | |
| 760 | *n*-C₃H₇ | *n*-C₅H₁₁ | | |
| 761 | *n*-C₅H₁₁ | *n*-C₃H₇ | | |
| 762 | *n*-C₅H₁₁ | *n*-C₅H₁₁ | | |
| 763 | CH₂=CH | CH₃ | | |
| 764 | CH₂=CH | C₂H₅ | | |
| 765 | CH₂=CH | *n*-C₃H₇ | | |
| 766 | CH₂=CH | CH₂=CH | | |
| 767 | CH₃ | CH₂=CH | | |
| 768 | C₂H₅ | CH₂=CH | | |
| 769 | *n*-C₃H₇ | CH₂=CH | | |
| 770 | *E*-CH₃-CH=CH | CH₂=CH | | |
| 771 | *E*-CH₃-CH=CH | *E*-CH₃-CH=CH | | |
| 772 | CH₃ | CH₃O | | |
| 773 | CH₃ | C₂H₅O | | |
| 774 | CH₃ | *n*-C₃H₇O | | |
| 775 | *n*-C₃H₇ | CH₃O | | |
| 776 | *n*-C₃H₇ | C₂H₅O | | |
| 777a | *n*-C₃H₇ | *n*-C₃H₇O | | |
| 777b | *n*-C₅H₁₁ | *n*-C₄H₉O | | |
| 777c | *n*-C₄H₉O | *n*-C₅H₁₁ | | |
| 778 | CH₃O | CH₃O | | |
| 779 | C₂H₅O | C₂H₅O | | |
| 780 | *n*-C₃H₇O | *n*-C₃H₇O | | |

### Referenzbeispiele 781 bis 810

Analog zu Beispiel 241 und Beispiel 361a werden hergestellt: 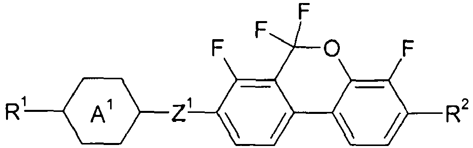 worin 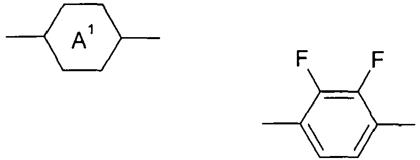 und
- Z¹: -CH₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 781 | CH₃ | CH₃ | | |
| 782 | CH₃ | C₂H₅ | | |
| 783 | CH₃ | *n*-C₃H₇ | | |
| 784 | C₂H₅ | CH3 | | |
| 785 | C₂H₅ | C₂H₅ | | |
| 786 | C₂H₅ | *n*-C₃H₇ | | |
| 787 | *n*-C₃H₇ | CH₃ | | |
| 788 | *n*-C₃H₇ | C₂H₅ | | |
| 789 | *n*-C₃H₇ | *n*-C₃H₇ | | |
| 790 | *n*-C₃H₇ | *n*-C₅H₁₁ | | |
| 791 | *n*-C₅H₁₁ | *n*-C₃H₇ | | |
| 792 | *n*-C₅H₁₁ | *n*-C₅H₁₁ | | |
| 793 | CH₂=CH | CH₃ | | |
| 794 | CH₂=CH | C₂H₅ | | |
| 795 | CH₂=CH | *n*-C₃H₇ | | |
| 796 | CH₂=CH | CH₂=CH | | |
| 797 | CH₃ | CH₂=CH | | |
| 798 | C₂H₅ | CH₂=CH | | |
| 799 | *n*-C₃H₇ | CH₂=CH | | |
| 800 | *E*-CH₃-CH=CH | CH₂=CH | | |
| 801 | *E*-CH₃-CH=CH | *E*-CH₃-CH=CH | | |
| 802 | CH₃ | CH₃O | | |
| 803 | CH₃ | C₂H₅O | | |
| 804 | CH₃ | *n*-C₃H₇O | | |
| 805 | *n*-C₃H₇ | CH₃O | | |
| 806 | *n*-C₃H₇ | C₂H₅O | | |
| 807a | *n*-C₃H₇ | *n*-C₃H₇O | | |
| 807b | *n*-C₆H₁₁ | *n*-C₄H₉O | | |
| 807c | *n*-C₄H₉O | *n*-C₅H₁₁ | | |
| 808 | CH₃O | CH₃O | | |
| 809 | C₂H₅O | C₂H₅O | | |
| 810 | *n*-C₃H₇O | *n*-C₃H₇O | | |

### Referenzbeispiele 811 bis 840

Analog zu Beispiel 241und Beispiel 361a werden hergestellt: 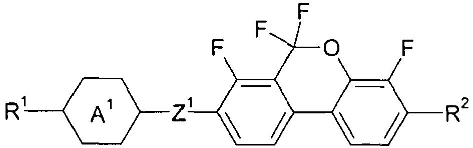 worin 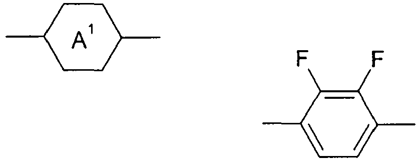 und
- Z¹: -CF=CF-
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 811 | CH₃ | CH₃ | | |
| 812 | CH₃ | C₂H₅ | | |
| 813 | CH₃ | *n*-C₃H₇ | | |
| 814 | C₂H₅ | CH₃ | | |
| 815 | C₂H₅ | C₂H₅ | | |
| 816 | C₂H₅ | *n*-C₃H₇ | | |
| 817 | *n*-C₃H₇ | CH₃ | | |
| 818 | *n*-C₃H₇ | C₂H₅ | | |
| 819 | *n*-C₃H₇ | *n*-C₃H₇ | | |
| 820 | *n*-C₃H₇ | *n*-C₅H₁₁ | | |
| 821 | *n*-C₅H₁₁ | *n*-C₃H₇ | | |
| 822 | *n*-C₅H₁₁ | *n*-C₅H₁₁ | | |
| 823 | CH₂=CH | CH₃ | | |
| 824 | CH₂=CH | C₂H₅ | | |
| 825 | CH₂=CH | *n*-C₃H₇ | | |
| 826 | CH₂=CH | CH₂=CH | | |
| 827 | CH₃ | CH₂=CH | | |
| 828 | C₂H₅ | CH₂=CH | | |
| 829 | *n*-C₃H₇ | CH₂=CH | | |
| 830 | *E*-CH₃-CH=CH | CH₂=CH | | |
| 831 | *E*-CH₃-CH=CH | *E*-CH₃-CH=CH | | |
| 832 | CH₃ | CH₃O | | |
| 833 | CH₃ | C₂H₅O | | |
| 834 | CH₃ | *n*-C₃H₇O | | |
| 835 | *n*-C₃H₇ | CH₃O | | |
| 836 | *n*-C₃H₇ | C₂H₅O | | |
| 837a | *n*-C₃H₇ | *n*-C₃H₇O | | |
| 837b | *n*-C₅H₁₁ | *n*-C₄H₉O | | |
| 837c | *n*-C₄H₉O | *n*-C₅H₁₁ | | |
| 838 | CH₃O | CH₃O | | |
| 839 | C₂H₅O | C₂H₅O | | |
| 840 | *n*-C₃H₇O | *n*-C₃H₇O | | |

### Ausgangsverbindung 841: (8-Ethoxy-4,7-difluor-3-benzyloxybenzo[c]chromen-6-on)

### 841.1 Herstellung von 4-Brom-1-ethoxy-2-fluorbenzol

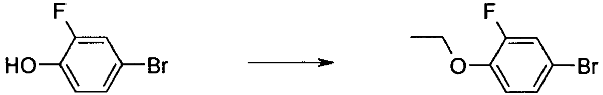

100 g (0,524 mol) 4-Brom-2-fluorphenol und 66,7 g (0,612 mol) Ethylbromid wurden in 2 l Ethylmethylketon gelöst und in Gegenwart von 185 g (1,34 mol) Kaliumcarbonat 24 h unter Rückfluß erhitzt. Anschließend wurde die Lösung filtriert, das Filtrat eingeengt und das Rohprodukt mit *n*-Hexan über Kieselgel filtriert. Man erhielt 114 g (99 % d. Th.) 4-Brom-1-ethoxy-2-fluorbenzol als farblose Flüssigkeit.

### 841.2 Herstellung von 6-Brom-3-ethoxy-2-fluorbenzoesäure

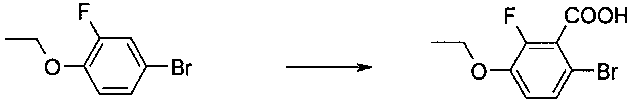

In Analogie zu der oben (bei Beispiel 1.3) beschriebenen Synthese erhielt man aus 236 g 4-Brom-1-ethoxy-2-fluorbenzol 190 g (64 % d. Th.) 6-Brom-3-ethoxy -2-fluorbenzoesäure als farblose Kristalle.

### 841.3 Herstellung von 1-Benzyloxy-4-brom-2-fluorbenzol

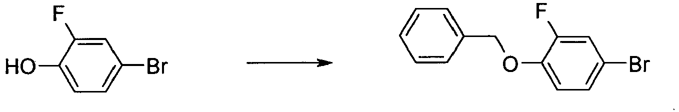

In Analogie zu der oben (unter Beispiel 2.1, bzw. 872.1) beschriebenen Synthese erhielt man aus 250 g (1.31 mol) 4-Brom-2-fluorphenol und 179 ml (1.51 mol) Benzylbromid 366 g (97 %) 1-Benzyloxy-4-brom-2-fluorbenzol als farblose Kristalle.

### 841.4 Herstellung von 3-Benzyloxy-6-brom-2-fluorphenol

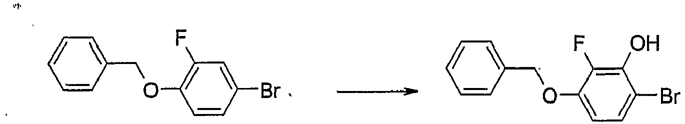

In Analogie zu der oben (bei Beispiel 1.2) beschriebenen Synthese erhielt man aus 366 g (1,27 mol) 1-Benzyloxy-4-brom-2-fluorbenzol 270 g (72 % d. Th.) 3-Benzyloxy-6-brom-2-fluorphenol als farblose Kristalle.

### 841.5 Herstellung von 6-Brom-3-ethoxy-2-fluorbenzoesäure-(3-benzyloxy-6-brom-2-fluorphenyl)ester

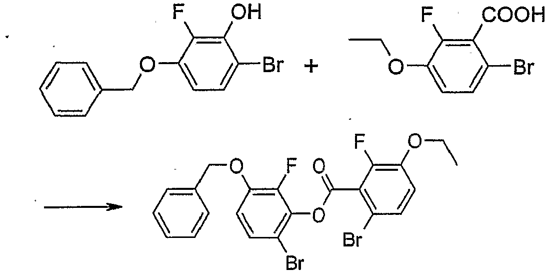

In Analogie zu der oben (bei Beispiel 1.4) beschriebenen Synthese erhielt man aus 253 g (0.851 mol) 3-Benzyloxy-6-brom-2-fluorphenol und 246 g (0.936 mol) 6-Brom-3-ethoxy-2-fluorbenzoesäure 405 g (87 % d. Th.) 6-Brom-3-ethoxy-2-fluorbenzoesäure-(3-benzyloxy-6-brom-2-fluorphenyl)ester als farblose Kristalle.

### 841.6 Herstellung von 8-Ethoxy-4,7-difluor-3-benzyloxybenzo[c]chromen-6-on

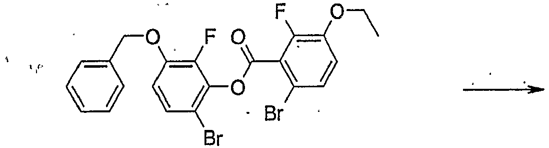

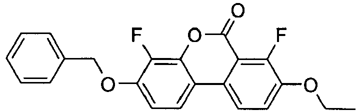

103 g (0,188 mol) 6-Brom-3-ethoxy-2-fluorbenzoesäure-(3-benzyloxy-6-brom-2-fluorphenyl)ester wurden in 1 l DMF gelöst und in Gegenwart von 119 g (1,.88 mol) Kupferpulver 72 h unter Rückfluß erhitzt. Anschließend wurde der Ansatz mit Wasser verdünnt, mit Essigester extrahiert und die vereinigten Extrakte wurden über Natriumsulfat getrocknet und eingeengt. Nach Kristallisation des Rohproduktes aus THF erhielt man 15 g (21 % d. Th.) 8-Ethoxy-4,7-difluor-3-benzyloxybenzo[c]chromen-6-on als schwach gelbe Kristalle.

### Ausgangsverbindungen 842 bis 871

Analog zu Ausgangsverbindung 841 werden hergestellt: 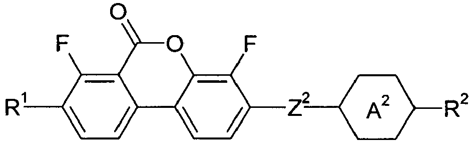 worin 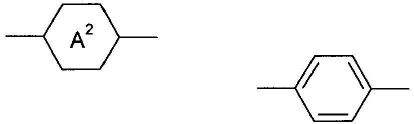 und
- Z²: -O-CH₂-
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε | T*(N,I) /°C |
|---|---|---|---|---|---|
| 842 | CH₃ | CH₃ | | | |
| 843 | CH₃ | C₂H₅ | | | |
| 844 | CH₃ | *n*-C₃H₇ | | | |
| 845 | C₂H₅ | CH₃ | | | |
| 846 | C₂H₅ | C₂H₅ | | | |
| 847 | C₂H₅ | *n*-C₃H₇ | | | |
| 848 | *n*-C₃H₇ | CH₃ | | | |
| 849 | *n*-C₃H₇ | C₂H₅ | | | |
| 850 | *n*-C₃H₇ | *n*-C₃H₇ | | | |
| 851 | *n*-C₃H₇ | *n*-C₅H₁₁ | | | |
| 852 | *n*-C₅H₁₁ | *n*-C₃H₇ | | | |
| 853 | *n*-C₅H₁₁ | *n*-C₅H₁₁ | | | |
| 854 | CH₂=CH | CH₃ | | | |
| 855 | CH₂=CH | C₂H₅ | | | |
| 856 | CH₂=CH | *n*-C₃H₇ | | | |
| 857 | CH₂=CH | CH₂=CH | | | |
| 858 | CH₃ | CH₂=CH | | | |
| 859 | C₂H₅ | CH₂=CH | | | |
| 860 | *n*-C₃H₇ | CH₂=CH | | | |
| 861 | *E*-CH₃-CH=CH | CH₂=CH | | | |
| 862 | *E*-CH₃-CH=CH | *E*-CH₃-CH=CH | | | |
| 863 | CH₃ | CH₃O | | | |
| 864 | CH₃ | C₂H₅O | | | |
| 865 | CH₃ | *n*-C₃H₇O | | | |
| 866 | *n*-C₃H₇ | CH₃O | | | |
| 867 | *n*-C₃H₇ | C₂H₅O | | | |
| 868a | *n*-C₃H₇ | *n*-C₃H₇O | | | |
| 868b | *n*-C₅H₁₁ | *n*-C₄H₉O | | | |
| 868c | *n*-C₄H₉O | *n*-C₅H₁₁ | | | |
| 869 | CH₃O | CH₃O | | | |
| 841 | C₂H₅O | H | | | |
| 870 | C₂H₅O | C₂H₅O | | | |
| 871 | *n*-C₃H₇O | *n*-C₃H₇O | | | |

### Beispiel 872: (8-Ethoxy-4,7-difluor-3-(trans-4-vinylcyclohexylmethoxy)-6H-benzo[c]chromen)

### 872.1 Herstellung von 8-Ethoxy-4,7-difluor-3-hydroxy-6H-benzo[c]chromen

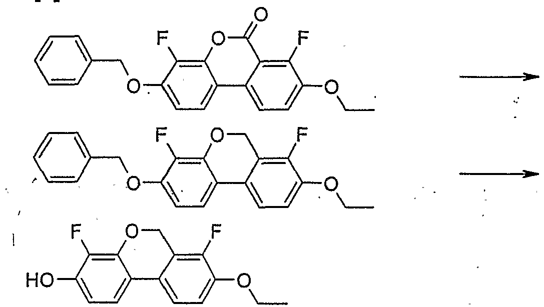

2,00 g (5,24 mmol) 3-Benzyloxy-8ethoxy-4,7-difluor-6H-benzo[c]chromen-6-on, der Verbindung des Beispiels 841 wurden in 12 ml THF gelöst und unter Kühlung mit Eis mit 2,37 ml (23,0 mmol) Bortrifluorid-THF-Komplex versetzt. Anschließend wurden 24 ml Ethylenglykoldimethylether und danach portionsweise 530 mg Natriumborhydrid zugegeben. Anschließend wurde 18 h bei Raumtemperatur gerührt und danach auf Eis gegeben. Es wurde wie üblich aufgereinigt. Es wurden 1,5 g (78% d. Th) 3-Benzyloxy-8-ethoxy-4,7-difluor-6H-benzo[c]chromen als farblose Kristalle erhalten. Diese wurden in THF gelöst, in Gegenwart von 0,6 g Pd/C (5%) bei einem Druck von 1 bar hydrier, filtriert und eingeengt. Man erhielt 1,2 g (99% d.Th.) 8-Ethoxy-4,7-difluor-3-hydroxy-6H-benzo[c]chromen als farblose Kristalle.

### 872.2 Herstellung von 8-Ethoxy-4,7-difluor-3-(trans-4-vinylcyclohexylmethoxy)-6H-benzo[c]chromen

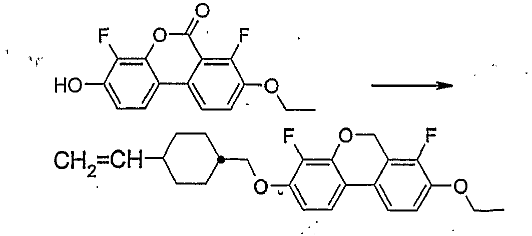

1,2 g (4,32 mmol) 8-Ethoxy-4,7-difluor-3-hydroxy-6H-benzo[c]chromen, 2,16 g (8,64 mmol) (*trans*-4-Vinylcyclohexyl)methyliodid und 650 mg (5 mmol) Kaliumcarbonat wurden in 15 ml Aceton 16 h unter Rückfluß erhitzt. Dann wurde auf MTB-Ether gegeben und wie üblich aufgereinigt. Man erhielt 460 mg (27% d.Th.) 8-Ethoxy-4,7-difluor-3-(trans-4-vinylcyclohexylmethoxy)-6H-benzo[c]chromen als farblose Kristalle.

### Beispiele 873-902

Analog zu Beispiel 872 werden hergestellt: 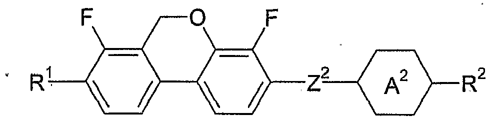 worin 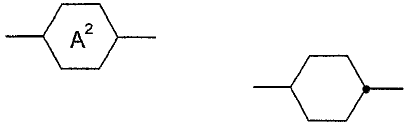 und
- Z²: -O-CH₂-
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* | T*(N,I) /°C |
|---|---|---|---|---|---|
| 873 | CH₃ | CH₃ | | | |
| 874 | CH₃ | C₂H₅ | | | |
| 875 | CH₃ | *n*-C₃H₇ | | | |
| 876 | C₂H₅ | CH₃ | | | |
| 877 | C₂H₅ | C₂H₅ | | | |
| 878 | C₂H₅ | *n*-C₃H₇ | | | |
| 879 | *n*-C₃H₇ | CH₃ | | | |
| 880 | *n*-C₃H₇ | C₂H₅ | | | |
| 881 | *n*-C₃H₇ | *n*-C₃H₇ | | | |
| 882 | *n*-C₃H₇ | *n*-C₅H₁₁ | | | |
| 883 | *n*-C₅H₁₁ | *n*-C₃H₇ | | | |
| 884 | *n*-C₅H₁₁ | *n*-C₅H₁₁ | | | |
| 885 | CH₂=CH | CH₃ | | | |
| 886 | CH₂=CH | C₂H₅ | | | |
| 887 | CH₂=CH | *n*-C₃H₇ | | | |
| 872 | CH₂=CH | C₂H₅O | C 123 N 167 I | -10,4 | 207 |
| 888 | CH₂=CH | CH₂=CH | | | |
| 889 | CH₃ | CH₂=CH | | | |
| 890 | C₂H₅ | CH₂=CH | | | |
| 891 | *n*-C₃H₇ | CH₂=CH | | | |
| 892 | *E*-CH₃-CH=CH | CH₂=CH | | | |
| 893 | *E*-CH₃-CH=CH | *E*-CH₃-CH=CH | | | |
| 894 | CH₃ | CH₃O | | | |
| 895 | CH₃ | C₂H₅O | | | |
| 896 | CH₃ | *n*-C₃H₇O | | | |
| 897 | *n*-C₃H₇ | CH₃O | | | |
| 898 | *n*-C₃H₇ | C₂H₅O | | | |
| 899a | *n*-C₃H₇ | *n*-C₃H₇O | | | |
| 899b | *n*-C₅H₁₁ | *n*-C₄H₉O | C 109 SA 157 N 167,5 I | -11,2 | 199 |
| 899c | *n*-C₄H₉O | *n*-C₅H₁₁ | | | |
| 900 | CH₃O | CH₃O | | | |
| 901 | C₂H₅O | C₂H₅O | | | |
| 902 | *n*-C₃H₇O | *n*-C₃H₇O | | | |

### Ausgangsverbindungen 903 bis 934

Analog zu Ausgangsverbindung 841 werden hergestellt: 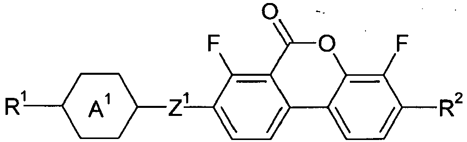 worin 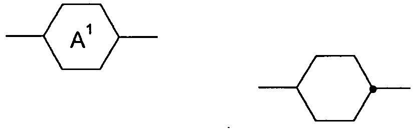 und
- Z¹: eine Einfachbindung
bedeutet.

Bemerkung: *10%-iger Lösung in ZLI-4792, bzw. ZLI-2857 (Δε) extrapolierte Werte.

| Nr. | R¹ | R² | Phasen sequenz T/°C | Δε* |
|---|---|---|---|---|
| 903 | CH₃ | CH₃ | | |
| 904 | CH₃ | C₂H₅ | | |
| 905 | CH₃ | *n*-C₃H₇ | | |
| 906 | C₂H₅ | CH₃ | | |
| 907 | C₂H₅ | C₂H₅ | | |
| 908 | C₂H₅ | *n*-C₃H₇ | | |
| 909 | *n*-C₃H₇ | CH₃ | | |
| 910 | *n*-C₃H₇ | C₂H₅ | | |
| 911 | *n*-C₃H₇ | *n*-C₃H₇ | | |
| 912 | *n*-C₃H₇ | *n*-C₅H₁₁ | | |
| 913 | *n*-C₅H₁₁ | *n*-C₃H₇ | | |
| 914 | *n*-C₅H₁₁ | *n*-C₅H₁₁ | | |
| 915 | CH₂=CH | CH₃ | | |
| 916 | CH₂=CH | C₂H₅ | | |
| 917 | CH₂=CH | *n*-C₃H₇ | | |
| 918 | CH₂=CH | CH₂=CH | | |
| 919 | CH₃ | CH₂=CH | | |
| 920 | C₂H₅ | CH₂=CH | | |
| 921 | *n*-C₃H₇ | CH₂=CH | | |
| 922 | *E*-CH₃-CH=CH | CH₂=CH | | |
| 923 | *E*-CH₃-CH=CH | *E*-CH₃-CH=CH | | |
| 924 | CH₃ | CH₃O | | |
| 925 | CH₃ | C₂H₅O | | |
| 926 | CH₃ | *n*-C₃H₇O | | |
| 927 | *n*-C₃H₇ | CH₃O | | |
| 928 | *n*-C₃H₇ | C₂H₅O | | |
| 929 | *n*-C₃H₇ | *n*-C₃H₇O | | |
| 930 | *n*-C₅H₁₁ | *n*-C₄H₉O | | |
| 931 | *n*-C₄H₉O | *n*-C₅H₁₁ | | |
| 932 | CH₃O | CH₃O | | |
| 933 | C₂H₅O | C₂H₅O | | |
| 934 | *n*-C₃H₇O | *n*-C₃H₇O | | |

### Ausgangsverbindungen 935 bis 966

Analog zu Ausgangsverbindung_841 werden hergestellt: 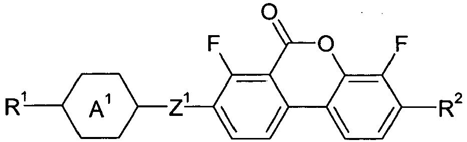 worin 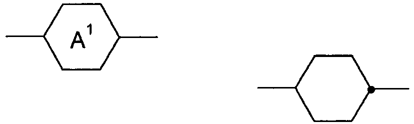 und
- Z¹: -CH₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 935 | CH₃ | CH₃ | | |
| 936 | CH₃ | C₂H₅ | | |
| 937 | CH₃ | *n*-C₃H₇ | | |
| 938 | C₂H₅ | CH₃ | | |
| 939 | C₂H₅ | C₂H₅ | | |
| 940 | C₂H₅ | *n*-C₃H₇ | | |
| 941 | *n*-C₃H₇ | CH₃ | | |
| 942 | *n*-C₃H₇ | C₂H₅ | | |
| 943 | *n*-C₃H₇ | *n*-C₃H₇ | | |
| 944 | *n*-C₃H₇ | *n*-C₅H₁₁ | | |
| 945 | *n*-C₅H₁₁ | *n*-C₃H₇ | | |
| 946 | *n*-C₅H₁₁ | *n*-C₅H₁₁ | | |
| 947 | CH₂=CH | CH₃ | | |
| 948 | CH₂=CH | C₂H₅ | | |
| 949 | CH₂=CH | *n*-C₃H₇ | | |
| 950 | CH₂=CH | CH₂=CH | | |
| 951 | CH₃ | CH₂=CH | | |
| 952 | C₂H₅ | CH₂=CH | | |
| 953 | *n*-C₃H₇ | CH₂=CH | | |
| 954 | *E*-CH₃-CH=CH | CH₂=CH | | |
| 955 | *E*-CH₃-CH=CH | *E*-CH₃-CH=CH | | |
| 956 | CH₃ | CH₃O | | |
| 957 | CH₃ | C₂H₅O | | |
| 958 | CH₃ | *n*-C₃H₇O | | |
| 959 | *n*-C₃H₇ | CH₃O | | |
| 960 | *n*-C₃H₇ | C₂H₅O | | |
| 961 | *n*-C₃H₇ | *n*-C₃H₇O | | |
| 962 | *n*-C₅H₁₁ | *n*-C₄H₉O | | |
| 963 | *n*-C₄H₉O | *n*-C₅H₁₁ | | |
| 964 | CH₃O | CH₃O | | |
| 965 | C₂H₅O | C₂H₅O | | |
| 966 | *n*-C₃H₇O | *n*-C₃H₇O | | |

### Ausgangsverbindungen 967 bis 998

Analog zu Ausgangsverbindung_841 werden hergestellt: 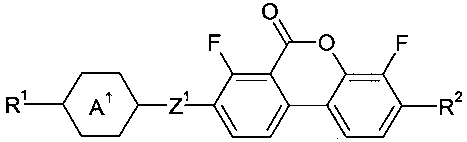 worin 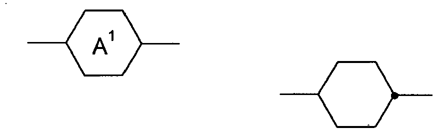 und
- Z¹: -CF₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 967 | CH₃ | CH₃ | | |
| 968 | CH₃ | C₂H₅ | | |
| 969 | CH₃ | *n*-C₃H₇ | | |
| 970 | C₂H₅ | CH₃ | | |
| 971 | C₂H₅ | C₂H₅ | | |
| 972 | C₂H₅ | *n*-C₃H₇ | | |
| 973 | *n*-C₃H₇ | CH₃ | | |
| 974 | *n*-C₃H₇ | C₂H₅ | | |
| 975 | *n*-C₃H₇ | *n*-C₃H₇ | | |
| 976 | *n*-C₃H₇ | *n*-C₅H₁₁ | | |
| 977 | *n*-C₅H₁₁ | *n*-C₃H₇ | | |
| 978 | *n*-C₅H₁₁ | *n*-C₅H₁₁ | | |
| 979 | CH₂=CH | CH₃ | | |
| 980 | CH₂=CH | C₂H₅ | | |
| 981 | CH₂=CH | *n*-C₃H₇ | | |
| 982 | CH₂=CH | CH₂=CH | | |
| 983 | CH₃ | CH₂=CH | | |
| 984 | C₂H₅ | CH₂=CH | | |
| 985 | *n*-C₃H₇ | CH₂=CH | | |
| 986 | *E*-CH₃-CH=CH | CH₂=CH | | |
| 987 | *E*-CH₃-CH=CH | *E*-CH₃-CH=CH | | |
| 988 | CH₃ | CH₃O | | |
| 989 | CH₃ | C₂H₅O | | |
| 990 | CH₃ | *n*-C₃H₇O | | |
| 991 | *n*-C₃H₇ | CH₃O | | |
| 992 | *n*-C₃H₇ | C₂H₅O | | |
| 993 | *n*-C₃H₇ | *n*-C₃H₇O | | |
| 994 | *n*-C₅H₁₁ | *n*-C₄H₉O | | |
| 995 | *n*-C₄H₉O | *n*-C₅H₁₁ | | |
| 996 | CH₃O | CH₃O | | |
| 997 | C₂H₅O | C₂H₅O | | |
| 998 | *n*-C₃H₇O | *n*-C₃H₇O | | |

### Ausgangsverbindungen 999 bis 1030

Analog zu Ausgangsverbindung_841 werden hergestellt: 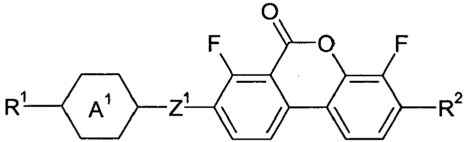 worin 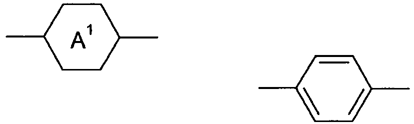 und
- Z¹: eine Einfachbindung
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 999 | CH₃ | CH₃ | | |
| 1000 | CH₃ | C₂H₅ | | |
| 1001 | CH₃ | *n*-C₃H₇ | | |
| 1002 | C₂H₅ | CH₃ | | |
| 1003 | C₂H₅ | C₂H₅ | | |
| 1004 | C₂H₅ | *n*-C₃H₇ | | |
| 1005 | *n*-C₃H₇ | CH₃ | | |
| 1006 | *n*-C₃H₇ | C₂H₅ | | |
| 1007 | *n*-C₃H₇ | *n*-C₃H₇ | | |
| 1008 | *n*-C₃H₇ | *n*-C₅H₁₁ | | |
| 1009 | *n*-C₅H₁₁ | *n*-C₃H₇ | | |
| 1010 | *n*-C₅H₁₁ | *n*-C₅H₁₁ | | |
| 1011 | CH₂=CH | CH₃ | | |
| 1012 | CH₂=CH | C₂H₅ | | |
| 1013 | CH₂=CH | *n*-C₃H₇ | | |
| 1014 | CH₂=CH | CH₂=CH | | |
| 1015 | CH₃ | CH₂=CH | | |
| 1016 | C₂H₅ | CH₂=CH | | |
| 1017 | *n*-C₃H₇ | CH₂=CH | | |
| 1018 | *E*-CH₃-CH=CH | CH₂=CH | | |
| 1019 | *E*-CH₃-CH=CH | *E*-CH₃-CH=CH | | |
| 1020 | CH₃ | CH₃O | | |
| 1021 | CH₃ | C₂H₅O | | |
| 1022 | CH₃ | *n*-C₃H₇O | | |
| 1023 | *n*-C₃H₇ | CH₃O | | |
| 1024 | *n*-C₃H₇ | C₂H₅O | | |
| 1025 | *n*-C₃H₇ | *n*-C₃H₇O | | |
| 1026 | *n*- C₅H₁₁ | *n*-C₄H₉O | C 187 S_{A} 230 N 230,4 l | |
| 1027 | *n*-C₄H₉O | *n*-C₅H₁₁ | | |
| 1028 | CH₃O | CH₃O | | |
| 1029 | C₂H₅O | C₂H₅O | | |
| 1030 | *n*-C₃H₇O | *n*-C₃H₇O | | |

### Ausgangsverbindungen 1031 bis 1062

Analog zu Ausgangsverbindung 841 werden hergestellt: 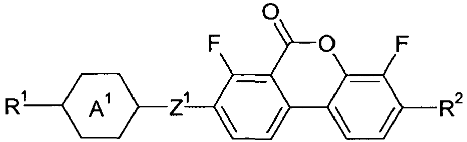 worin 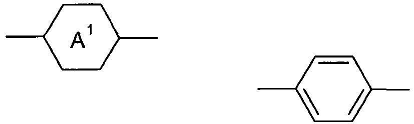 und
- Z¹: -CH₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1031 | CH₃ | CH₃ | | |
| 1032 | CH₃ | C₂H₅ | | |
| 1033 | CH₃ | *n*-C₃H₇ | | |
| 1034 | C₂H₅ | CH₃ | | |
| 1035 | C₂H₅ | C₂H₅ | | |
| 1036 | C₂H₅ | *n*-C₃H₇ | | |
| 1037 | *n*-C₃H₇ | CH₃ | | |
| 1038 | *n*-C₃H₇ | C₂H₅ | | |
| 1039 | *n*-C₃H₇ | *n*-C₃H₇ | | |
| 1040 | *n*-C₃H₇ | *n*-C₅H₁₁ | | |
| 1041 | *n*-C₅H₁₁ | *n*-C₃H₇ | | |
| 1042 | *n*-C₅H₁₁ | *n*-C₅H₁₁ | | |
| 1043 | CH₂=CH | CH₃ | | |
| 1044 | CH₂=CH | C₂H₅ | | |
| 1045 | CH₂=CH | *n*-C₃H₇ | | |
| 1046 | CH₂=CH | CH₂=CH | | |
| 1047 | CH₃ | CH₂=CH | | |
| 1048 | C₂H₅ | CH₂=CH | | |
| 1049 | *n*-C₃H₇ | CH₂=CH | | |
| 1050 | *E*-CH₃-CH=CH | CH₂=CH | | |
| 1051 | *E*-CH₃-CH=CH | *E*-CH₃-CH=CH | | |
| 1052 | CH₃ | CH₃O | | |
| 1053 | CH₃ | C₂H₅O | | |
| 1054 | CH₃ | *n*-C₃H₇O | | |
| 1055 | *n*-C₃H₇ | CH₃O | | |
| 1056 | *n*-C₃H₇ | C₂H₅O | | |
| 1057 | *n*-C₃H₇ | *n*-C₃H₇O | | |
| 1058 | *n*-C₅H₁₁ | *n*-C₅H₁₁O | C 151 S_{B}188 S_{A} 191 I | |
| 1059 | *n*-C₄H₉O | *n*-C₅H₁₁ | | |
| 1060 | CH₃O | CH₃O | | |
| 1061 | C₂H₅O | C₂H₅O | | |
| 1062 | *n*-C₃H₇O | *n*-C₃H₇O | | |

### Ausgangsverbindungen 1063 bis 1093

Analog zu Ausgangsverbindung_841 werden hergestellt: 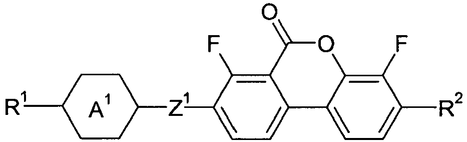 worin 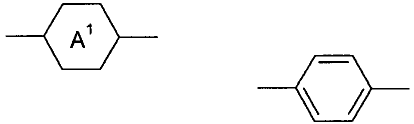 und
- Z¹: -CF₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1063 | CH₃ | CH₃ | | |
| 1064 | CH₃ | C₂H₅ | | |
| 1065 | CH₃ | *n*-C₃H₇ | | |
| 1066 | C₂H₅ | CH₃ | | |
| 1067 | C₂H₅ | C₂H₅ | | |
| 1068 | C₂H₅ | *n*-C₃H₇ | | |
| 1069 | *n*-C₃H₇ | CH₃ | | |
| 1070 | *n*-C₃H₇ | C₂H₅ | | |
| 1071 | *n*-C₃H₇ | *n*-C₃H₇ | | |
| 1072 | *n*-C₃H₇ | *n*-C₅H₁₁ | | |
| 1073 | *n*-C₅H₁₁ | *n*-C₃H₇ | | |
| 1074 | *n*-C₅H₁₁ | *n*-C₅H₁₁ | | |
| 1075 | CH₂=CH | CH₃ | | |
| 1076 | CH₂=CH | C₂H₅ | | |
| 1077 | CH₂=CH | *n*-C₃H₇ | | |
| 1078 | CH₂=CH | CH₂=CH | | |
| 1079 | CH₃ | CH₂=CH | | |
| 1080 | C₂H₅ | CH₂=CH | | |
| 1081 | *n*-C₃H₇ | CH₂=CH | | |
| 1082 | *E*-CH₃-CH=CH | CH₂=CH | | |
| 1083 | *E*-CH₃-CH=CH | *E*-CH₃-CH=CH | | |
| 1084 | CH₃ | CH₃O | | |
| 1085 | CH₃ | C₂H₅O | | |
| 1086 | CH₃ | *n*-C₃H₇O | | |
| 1087 | *n*-C₃H₇ | CH₃O | | |
| 1088 | *n*-C₃H₇ | C₂H₅O | | |
| 1089 | *n*-C₃H₇ | *n*-C₃H₇O | | |
| 1090 | *n*-C₄H₉O | *n*-C₅H₁₁ | | |
| 1091 | CH₃O | CH₃O | | |
| 1092 | C₂H₅O | C₂H₅O | | |
| 1093 | *n*-C₃H₇O | *n*-C₃H₇O | | |

### Ausgangsverbindungen 1094 bis 1125

Analog zu Ausgangsverbindung_841 werden hergestellt: 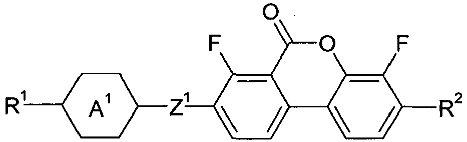 worin 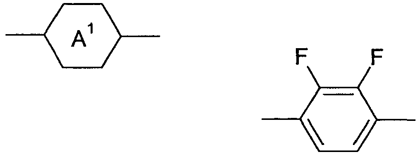 und
- Z¹: eine Einfachbindung
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1094 | CH₃ | CH₃ | | |
| 1095 | CH₃ | C₂H₅ | | |
| 1096 | CH₃ | *n*-C₃H₇ | | |
| 1097 | C₂H₅ | CH₃ | | |
| 1098 | C₂H₅ | C₂H₅ | | |
| 1099 | C₂H₅ | *n*-C₃H₇ | | |
| 1100 | *n*-C₃H₇ | CH₃ | | |
| 1101 | *n*-C₃H₇ | C₂H₅ | | |
| 1102 | *n*-C₃H₇ | *n*-C₃H₇ | | |
| 1103 | *n*-C₃H₇ | *n*-C₅H₁₁ | | |
| 1104 | *n*-C₅H₁₁ | *n*-C₃H₇ | | |
| 1105 | *n*-C₅H₁₁ | *n*-C₅H₁₁ | | |
| 1106 | CH₂=CH | CH₃ | | |
| 1107 | CH₂=CH | C₂H₅ | | |
| 1108 | CH₂=CH | *n*-C₃H₇ | | |
| 1109 | CH₂=CH | CH₂=CH | | |
| 1110 | CH₃ | CH₂=CH | | |
| 1111 | C₂H₅ | CH₂=CH | | |
| 1112 | *n*-C₃H₇ | CH₂=CH | | |
| 1113 | *E*-CH₃-CH=CH | CH₂=CH | | |
| 1114 | *E*-CH₃-CH=CH | *E*-CH₃-CH=CH | | |
| 1115 | CH₃ | CH₃O | | |
| 1116 | CH₃ | C₂H₅O | | |
| 1117 | CH₃ | *n*-C₃H₇O | | |
| 1118 | *n*-C₃H₇ | CH₃O | | |
| 1119 | *n*-C₃H₇ | C₂H₅O | | |
| 1120 | *n*-C₃H₇ | *n*-C₃H₇O | | |
| 1121 | *n*-C₅H₁₁ | *n*-C₄H₉O | | |
| 1122 | *n*-C₄H₉O | *n*-C₅H₁₁ | | |
| 1123 | CH₃O | CH₃O | | |
| 1124 | C₂H₅O | C₂H₅O | | |
| 1125 | *n*-C₃H₇O | *n*-C₃H₇O | | |

### Ausgangsverbindungen 1126 bis 1157

Analog zu Ausgangsverbindung 841 werden hergestellt: 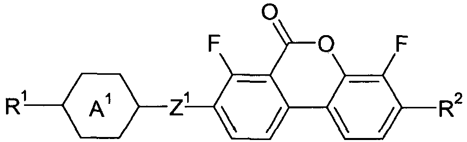 worin 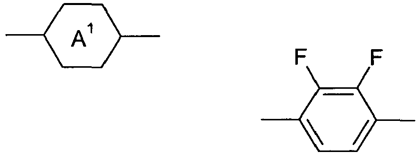 und
- Z¹: -CH₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1126 | CH₃ | CH₃ | | |
| 1127 | CH₃ | C₂H₅ | | |
| 1128 | CH₃ | *n*-C₃H₇ | | |
| 1129 | C₂H₅ | CH₃ | | |
| 1130 | C₂H₅ | C₂H₅ | | |
| 1131 | C₂H₅ | *n*-C₃H₇ | | |
| 1132 | *n*-C₃H₇ | CH₃ | | |
| 1133 | *n*-C₃H₇ | C₂H₅ | | |
| 1134 | *n*-C₃H₇ | *n*-C₃H₇ | | |
| 1135 | *n*-C₃H₇ | *n*-C₅H₁₁ | | |
| 1136 | *n*-C₅H₁₁ | *n*-C₃H₇ | | |
| 1137 | *n*-C₅H₁₁ | *n*-C₅H₁₁ | | |
| 1138 | CH₂=CH | CH₃ | | |
| 1139 | CH₂=CH | C₂H₅ | | |
| 1140 | CH₂=CH | *n*-C₃H₇ | | |
| 1141 | CH₂=CH | CH₂=CH | | |
| 1142 | CH₃ | CH₂=CH | | |
| 1143 | C₂H₅ | CH₂=CH | | |
| 1144 | *n*-C₃H₇ | CH₂=CH | | |
| 1145 | *E*-CH₃-CH=CH | CH₂=CH | | |
| 1146 | *E*-CH₃-CH=CH | *E*-CH₃-CH=CH | | |
| 1147 | CH₃ | CH₃O | | |
| 1148 | CH₃ | C₂H₅O | | |
| 1149 | CH₃ | *n*-C₃H₇O | | |
| 1150 | *n*-C₃H₇ | CH₃O | | |
| 1151 | *n*-C₃H₇ | C₂H₅O | | |
| 1152 | *n*-C₃H₇ | *n*-C₃H₇O | | |
| 1153 | *n*-C₅H₁₁ | *n*-C₄H₉O | | |
| 1154 | *n*-C₄H₉O | *n*-C₅H₁₁ | | |
| 1155 | CH₃O | CH₃O | | |
| 1156 | C₂H₅O | C₂H₅O | | |
| 1157 | *n*-C₃H₇O | *n*-C₃H₇O | | |

### Ausgangsverbindungen 1158 bis 1190

Analog zu Ausgangsverbindung 841 werden hergestellt: 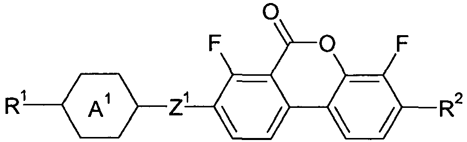 worin 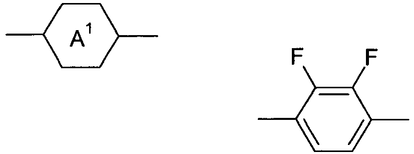 und
- Z¹: -CF₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1158 | CH₃ | CH₃ | | |
| 1159 | CH₃ | C₂H₅ | | |
| 1160 | CH₃ | *n*-C₃H₇ | | |
| 1161 | C₂H₅ | CH₃ | | |
| 1162 | C₂H₅ | C₂H₅ | | |
| 1163 | C₂H₅ | *n*-C₃H₇ | | |
| 1154 | *n*-C₃H₇ | CH₃ | | |
| 1165 | *n*-C₃H₇ | C₂H₅ | | |
| 1166 | *n*-C₃H₇ | *n*-C₃H₇ | | |
| 1167 | *n*-C₃H₇ | *n*-C₅H₁₁ | | |
| 1168 | *n*-C₅H₁₁ | *n*-C₃H₇ | | |
| 1159 | *n*-C₅H₁₁ | *n*-C₅H₁₁ | | |
| 1170 | CH₂=CH | CH₃ | | |
| 1171 | CH₂=CH | C₂H₅ | | |
| 1172 | CH₂=CH | *n*-C₃H₇ | | |
| 1173 | CH₂=CH | CH₂=CH | | |
| 1174 | CH₃ | CH₂=CH | | |
| 1175 | C₂H₅ | CH₂=CH | | |
| 1176 | *n*-C₃H₇ | CH₂=CH | | |
| 1177 | *E*-CH₃-CH=CH | CH₂=CH | | |
| 1178 | *E*-CH₃-CH=CH | *E*-CH₃-CH=CH | | |
| 1179 | CH₃ | CH₃O | | |
| 1180 | CH₃ | C₂H₅O | | |
| 1181 | CH₃ | *n*-C₃H₇O | | |
| 1182 | *n*-C₃H₇ | CH₃O | | |
| 1183 | *n*-C₃H₇ | C₂H₅O | | |
| 1184 | *n*-C₃H₇ | *n*-C₃H₇O | | |
| 1185 | *n*-C₅H₁₁ | *n*-C₄H₉O | | |
| 1186 | *n*-C₄H₉O | *n*-C₅H₁₁ | | |
| 1187 | CH₃O | CH₃O | | |
| 1188 | C₂H₅O | C₂H₅O | | |
| 1190 | *n*-C₃H₇O | *n*-C₃H₇O | | |

### Beispiele 1191 bis 1222

Analog zu Beispiel 241 und Beispiel 361a werden hergestellt: 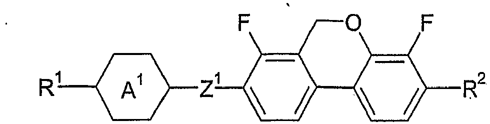 worin 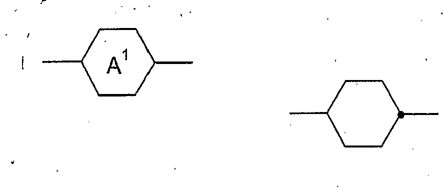 und
- Z¹: eine Einfachbindung
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1191 | CH₃ | CH₃ | | |
| 1192 | CH₃ | C₂H₅ | | |
| 1193 | CH₃ | *n*-C₃H₇ | | |
| 1194 | C₂H₅ | CH₃ | | |
| 1195 | C₂H₅ | C₂H₅ | | |
| 1196 | C₂H₅ | *n*-C₃H₇ | | |
| 1197 | *n*-C₃H₇ | CH₃ | | |
| 1198 | *n*-C₃H₇ | C₂H₅ | | |
| 1199 | *n*-C₃H₇ | *n*-C₃H₇ | | |
| 1200 | *n*-C₃H₇ | *n*-C₅H₁₁ | | |
| 1201 | *n*-C₅H₁₁ | *n*-C₃H₇ | | |
| 1202 | *n*-C₅H₁₁ | *n*-C₅H₁₁ | | |
| 1203 | CH₂=CH | CH₃ | | |
| 1204 | CH₂=CH | C₂H₅ | | |
| 1205 | CH₂=CH | *n*-C₃H₇ | | |
| 1206 | CH₂=CH | CH₂=CH | | |
| 1207 | CH₃ | CH₂=CH | | |
| 1208 | C₂H₅ | CH₂=CH | | |
| 1209 | *n*-C₃H₇ | CH₂=CH | | |
| 1200 | *E*-CH₃-CH=CH | CH₂=CH | | |
| 1211 | *E*-CH₃-CH=CH | *E*-CH₃-CH=CH | | |
| 1212 | CH₃ | CH₃O | | |
| 1213 | CH₃ | C₂H₅O | | |
| 1214 | CH₃ | *n*-C₃H₇O | | |
| 1215 | *n*-C₃H₇ | CH₃O | | |
| 1216 | *n*-C₃H₇ | C₂H₅O | | |
| 1217 | *n*-C₃H₇ | n-C₃H₇O | | |
| 1218 | *n*-C₅H₁₁ | *n*-C₄H₉O | | |
| 1219 | *n*-C₄H₉O | *n*-C₅H₁₁ | | |
| 1220 | CH₃O | CH₃O | | |
| 1221 | C₂H₅O | C₂H₅O | | |
| 1222 | *n*-C₃H₇O | *n*-C₃H₇O | | |

### Beispiele 1223 bis 1254

Analog zu Beispiel 241 und Beispiel 361a werden hergestellt: 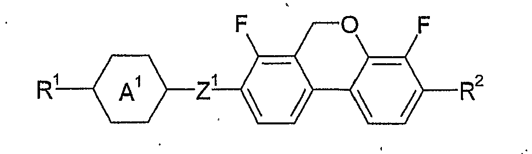 worin 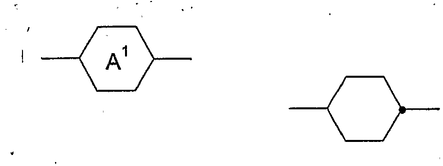 und
- Z¹: -CH₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1223 | CH₃ | CH₃ | | |
| 1224 | CH₃ | C₂H₅ | | |
| 1225 | CH₃ | *n*-C₃H₇ | | |
| 1226 | C₂H₅ | CH₃ | | |
| 1227 | C₂H₅ | C₂H₅ | | |
| 1228 | C₂H₅ | *n*-C₃H₇ | | |
| 1229 | *n*-C₃H₇ | CH₃ | | |
| 1230 | *n*-C₃H₇ | C₂H₅ | | |
| 1231 | *n*-C₃H₇ | *n*-C₃H₇ | | |
| 1232 | *n*-C₃H₇ | *n*-C₅H₁₁ | | |
| 1233 | n-C₅H₁₁ | *n*-C₃H₇ | | |
| 1234 | *n*-C₅H₁₁ | *n*-C₅H₁₁ | | |
| 1235 | CH₂=CH | CH₃ | | |
| 1236 | CH₂=CH | C₂H₅ | | |
| 1237 | CH₂=CH | *n*-C₃H₇ | | |
| 1238 | CH₂=CH | CH₂=CH | | |
| 1239 | CH₃ | CH₂=CH | | |
| 1240 | C₂H₅ | CH₂=CH | | |
| 1241 | *n*-C₃H₇ | CH₂=CH | | |
| 1242 | *E*-CH₃-CH=CH | CH₂=CH | | |
| 1243 | *E*-CH₃-CH=CH | *E*-CH₃-CH=CH | | |
| 1244 | CH₃ | CH₃O | | |
| 1245 | CH₃ | C₂H₅O | | |
| 1246 | CH₃ | *n*-C₃H₇O | | |
| 1247 | *n*-C₃H₇ | CH₃O | | |
| 1248 | *n*-C₃H₇ | C₂H₅O | | |
| 1249 | *n*-C₃H₇ | *n*-C₃H₇O | | |
| 1250 | *n*-C₅H₁₁ | *n*-C₄H₉O | | |
| 1251 | *n*-C₄H₉O | *n*-C₅H₁₁ | | |
| 1252 | CH₃O | CH₃O | | |
| 1253 | C₂H₅O | C₂H₅O | | |
| 1254 | *n*-C₃H₇O | *n*-C₃H₇O | | |

### Beispiele 1255 bis 1286

Analog zu Beispiel 241 und Beispiel 361a werden hergestellt: 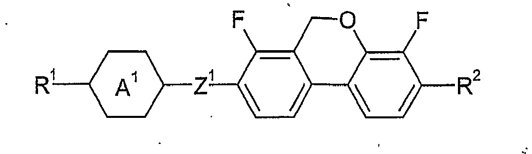 worin 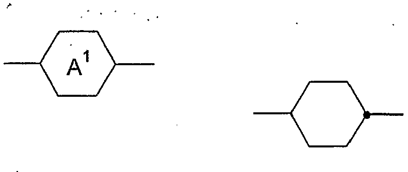 und
- Z¹: -CF₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1255 | CH₃ | CH₃ | | |
| 1256 | CH₃ | C₂H₅ | | |
| 1257 | CH₃ | *n*-C₃H₇ | | |
| 1258 | C₂H₅ | CH₃ | | |
| 1259 | C₂H₅ | C₂H₅ | | |
| 1260 | C₂H₅ | *n*-C₃H₇ | | |
| 1261 | *n*-C₃H₇ | CH₃ | | |
| 1262 | *n*-C₃H₇ | C₂H₅ | | |
| 1263 | *n*-C₃H₇ | *n*-C₃H₇ | | |
| 1264 | *n*-C₃H₇ | *n*-C₅H₁₁ | | |
| 1265. | *n*-C₅H₁₁ | *n*-C₃H₇ | | |
| 1266 | *n*-C₅H₁₁ | *n*-C₅H₁₁ | | |
| 1267 | CH₂=CH | CH₃ | | |
| 1268 | CH₂=CH | C₂H₅ | | |
| 1269 | CH₂=CH | *n*-C₃H₇ | | |
| 1270 | CH₂=CH | CH₂=CH | | |
| 1271 | CH₃ | CH₂=CH | | |
| 1272 | C₂H₅ | CH₂=CH | | |
| 1273 | *n*-C₃H₇ | CH₂=CH | | |
| 1274 | *E*-CH₃-CH=CH | CH₂=CH | | |
| 1275 | *E*-CH₃-CH=CH | *E*-CH₃-CH=CH | | |
| 1276 | CH₃ | CH₃O | | |
| 1277 | CH₃ | C₂H₅O | | |
| 1278 | CH₃ | *n*-C₃H₇O | | |
| 1279 | *n*-C₃H₇ | CH₃O | | |
| 1280 | *n*-C₃H₇ | C₂H₅O | | |
| 1281 | *n*-C₃H₇ | *n*-C₃H₇O | | |
| 1252 | *n*-C₅H₁₁ | *n*-C₄H₉O | | |
| 1283 | *n*-C₄H₉O | *n*-C₅H₁₁ | | |
| 1284 | CH₃O | CH₃O | | |
| 1285 | C₂H₅O | C₂H₅O | | |
| 1286 | *n*-C₃H₇O | *n*-C₃H₇O | | |

### Beispiele 1287 bis 1318

Analog zu Beispiel 241 und Beispiel 361a werden hergestellt: 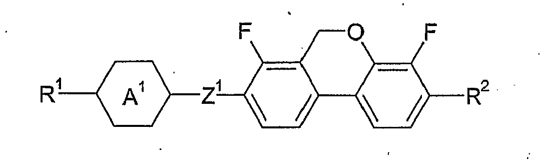 worin 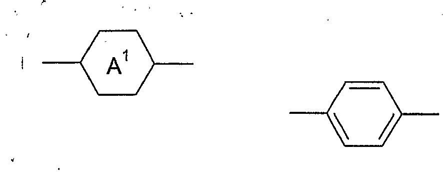 und
- Z¹: eine Einfachbindung
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1287 | CH₃ | CH₃ | | |
| 1288 | CH₃ | C₂H₅ | | |
| 1289 | CH₃ | *n*-C₃H₇ | | |
| 1290 | C₂H₅ | CH₃ | | |
| 1291 | C₂H₅ | C₂H₅ | | |
| 1292 | C₂H₅ | *n*-C₃H₇ | | |
| 1293 | *n*-C₃H₇ | CH₃ | | |
| 1294 | *n*-C₃H₇ | C₂H₅ | | |
| 1295 | *n*-C₃H₇ | *n*-C₃H₇ | | |
| 1296 | *n*-C₃H₇ | *n*-C₅H₁₁ | | |
| 1297 | *n*-C₅H₁₁ | *n*-C₃H₇ | | |
| 1298 | *n*-C₅H₁₁ | *n*-C₅H₁₁ | | |
| 1299 | CH₂=CH | CH₃ | | |
| 1300 | CH₂=CH | C₂H₅ | | |
| 1301 | CH₂=CH | *n*-C₃H₇ | | |
| 1302 | CH₂=CH | CH₂=CH | | |
| 1303 | CH₃ | CH₂=CH | | |
| 1304 | C₂H₅ | CH₂=CH | | |
| 1305 | *n*-C₃H₇ | CH₂=CH | | |
| 1306 | *E*-CH₃-CH=CH | CH₂=CH | | |
| 1307 | *E*-CH₃-CH=CH | *E*-CH₃-CH=CH | | |
| 1308 | CH₃ | CH₃O | | |
| 1309 | CH₃ | C₂H₅O | | |
| 1310 | CH₃ | *n*-C₃H₇O | | |
| 1311 | *n*-C₃H₇ | CH₃O | | |
| 1312 | *n*-C₃H₇ | C₂H₅O | | |
| 1313 | *n*-C₃H₇ | *n*-C₃H₇O | | |
| 1314 | *n*-C₅H₁₁ | *n*-C₄H₉O | | |
| 1315 | *n*-C₄H₉O | *n*-C₅H₁₁ | | |
| 1316 | CH₃O | CH₃O | | |
| 1317 | C₂H₅O | C₂H₅O | | |
| 1318 | *n*-C₃H₇O | *n*-C₃H₇O | | |

### Beispiele 1319 bis 1350

Analog zu Beispiel 241 und Beispiel 361a werden hergestellt: 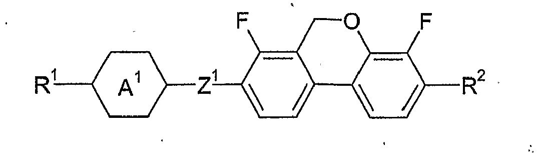 worin 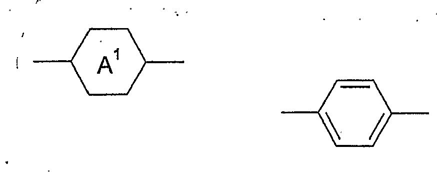 und
- z¹: -CH₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1319 | CH₃ | CH₃ | | |
| 1320 | CH₃ | C₂H₅ | | |
| 1321 | CH₃ | *n*-C₃H₇ | | |
| 1322 | C₂H₅ | CH₃ | | |
| 1323 | C₂H₅ | C₂H₅ | | |
| 1324 | C₂H₅ | *n*-C₃H₇ | | |
| 1325 | *n*-C₃H₇ | CH₃ | | |
| 1326 | *n*-C₃H₇ | C₂H₅ | | |
| 1327 | *n*-C₃H₇ | *n*-C₃H₇ | | |
| 1328 | *n*-C₃H₇ | *n*-C₅H₁₁ | | |
| 1329 | *n*-C₅H₁₁ | *n*-C₃H₇ | | |
| 1330 | *n*-C₅H₁₁ | *n*-C₅H₁₁ | | |
| 1331 | CH₂=CH | CH₃ | | |
| 1332 | CH₂=CH | C₂H₅ | | |
| 1333 | CH₂=CH | *n*-C₃H₇ | | |
| 1334 | CH₂=CH | CH₂=CH | | |
| 1335 | CH₃ | CH₂=CH | | |
| 1336 | C₂H₅ | CH₂=CH | | |
| 1337 | *n*-C₃H₇ | CH₂=CH | | |
| 1338 | *E*-CH₃-CH=CH | CH₂=CH | | |
| 1339 | *E*-CH₃-CH=CH | *E*-CH₃-CH=CH | | |
| 1340 | CH₃ | CH₃O | | |
| 1341 | CH₃ | C₂H₅O | | |
| 1342 | CH₃ | *n*-C₃H₇O | | |
| 1343 | *n*-C₃H₇ | CH₃O | | |
| 1344 | *n*-C₃H₇ | C₂H₅O | | |
| 1345 | *n*-C₃H₇ | *n*-C₃H₇O | | |
| 1346 | *n*-C₅H₁₁ | *n*-C₄H₉O | C 112 S_{A} 149 l | -8,1 |
| 1347 | *n*-C₄H₉O | *n*-C₅H₁₁ | | |
| 1348 | CH₃O | CH₃O | | |
| 1349 | C₂H₅O | C₂H₅O | | |
| 1350 | *n*-C₃H₇O | *n*-C₃H₇O | | |

### Beispiele 1351 bis 1382

Analog zu Beispiel 241 und Beispiel 361a werden hergestellt: 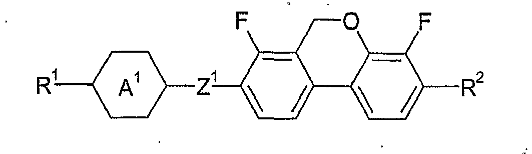 worin 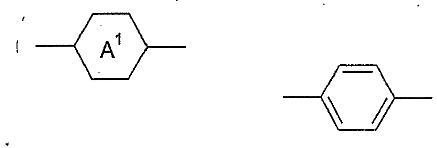 und
- Z¹: -CF₂-O-
bedeutet.

Bemerkung: *10%-iger Lösung in ZLI-4792, bzw. ZLI-2857 (Δε) extrapolierte Werte.

| N r. | R¹ | R² | Phasensequenz T/°C | Δε* |
|---|---|---|---|---|
| 1351 | CH₃ | CH₃. | | |
| 1352 | CH₃ | C₂H₅ | | |
| 1353 | CH₃ | *n*-C₃H₇ | | |
| 1354 | C₂H₅ | CH₃ | | |
| 1355 | C₂H₅ | C₂H₅ | | |
| 1356 | C₂H₅ | *n*-C₃H₇ | | |
| 1357 | *n*-C₃H₇ | CH₃ | | |
| 1358 | *n*-C₃H₇ | C₂H₅ | | |
| 1359 | *n*-C₃H₇ | *n*-C₃H₇ | | |
| 1360 | *n*-C₃H₇ | *n*-C₅H₁₁ | | |
| 1361 | *n*-C₅H₁₁ | *n*-C₃H₇ | | |
| 1362 | *n*-C₅H₁₁ | *n*-C₆H₁₁ | | |
| 1363 | CH₂=CH | CH₃ | | |
| 1364 | CH₂=CH | C₂H₅ | | |
| 1365 | CH₂=CH | *n*-C₃H₇ | | |
| 1366 | CH₂=CH | CH₂=CH | | |
| 1367 | CH₃ | CH₂=CH | | |
| 1368 | C₂H₅ | CH₂=CH | | |
| 1369 | *n*-C₃H₇ | CH₂=CH | | |
| 1370 | *E*-CH₃-CH=CH | CH₂=CH | | |
| 1371 | *E*-CH₃-CH=CH | *E*-CH₃-CH=CH | | |
| 1372 | CH₃ | CH₃O | | |
| 1373 | CH₃ | C₂H₅O | | |
| 1374 | CH₃ | *n*-C₃H₇O | | |
| 1375 | *n*-C₃H₇ | CH₃O | | |
| 1376 | *n*-C₃H₇ | C₂H₅O | | |
| 1377 | *n*-C₃H₇ | *n*-C₃H₇O | | |
| 1378 | *n*-C₅H₁₁ | *n*-C₄H₉O | | |
| 1379 | *n*-C₄H₉O | *n*-C₅H₁₁ | | |
| 1380 | CH₃O | CH₃O | | |
| 1381 | C₂H₅O | C₂H₅O | | |
| 1382 | *n*-C₃H₇O | *n*-C₃H₇O | | |

### Beispiele 1383 bis 1385

Analog zu Beispiel 241 und Beispiel 361a werden hergestellt: 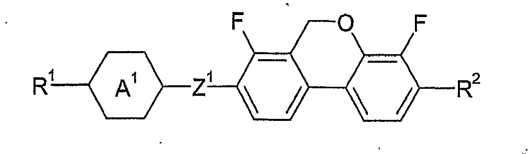 worin 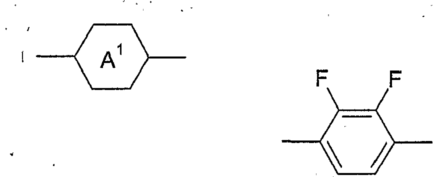 und
- Z¹: eine Einfachbindung
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1383 | CH₃ | CH₃ | | |
| 1384 | CH₃ | C₂H₅ | | |
| 1385 | CH₃ | *n*-C₃H₇ | | |
| 1386 | C₂H₅ | CH₃ | | |
| 1387 | C₂H₅ | C₂H₅ | | |
| 1388 | C₂H₅ | *n*-C₃H₇ | | |
| 1389 | *n*-C₃H₇ | CH₃ | | |
| 1390 | *n*-C₃H₇ | C₂H₅ | | |
| 1391 | *n*-C₃H₇ | *n*-C₃H₇ | | |
| 1392 | *n*-C₃H₇ | *n*-C₅H₁₁ | | |
| 1393 | *n*-C₅H₁₁ | *n*-C₃H₇ | | |
| 1394 | *n*-C₅H₁₁ | *n*-C₅H₁₁ | | |
| 1395 | CH₂=CH | CH₃ | | |
| 1396 | CH₂=CH | C₂H₅ | | |
| 1397 | CH₂=CH | *n*-C₃H₇ | | |
| 1398 | CH₂=CH | CH₂=CH | | |
| 1399 | CH₃ | CH₂=CH | | |
| 1400 | C₂H₅ | CH₂=CH | | |
| 1401 | *n*-C₃H₇ | CH₂=CH | | |
| 1402 | *E*-CH₃-CH=CH | CH₂=CH | | |
| 1403 | *E*-CH₃-CH=CH | *E*-CH₃-CH=CH | | |
| 1404 | CH₃ | CH₃O | | |
| 1405 | CH₃ | C₂H₅O | | |
| 1406 | CH₃ | *n*-C₃H₇O | | |
| 1407 | *n*-C₃H₇ | CH₃O | | |
| 1408 | *n*-C₃H₇ | C₂H₅O | | |
| 1409 | *n*-C₃H₇ | *n*-C₃H₇O | | |
| 1410 | *n*-C₅H₁₁ | *n*-C₄H₉O | | |
| 1411 | *n*-C₄H₉O | *n*-C₅H₁₁ | | |
| 1412 | CH₃O | CH₃O | | |
| 1413 | C₂H₅O | C₂H₅O | | |
| 1414 | *n*-C₃H₇O | *n*-C₃H₇O | | |

### Beispiele 1415 bis 1446

Analog zu Beispiel 241 und Beispiel 361a werden hergestellt: 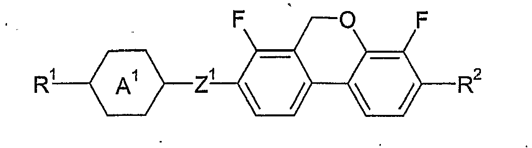 worin 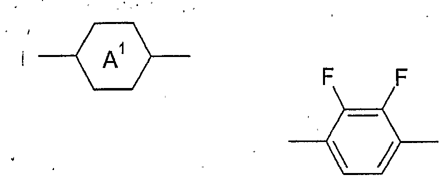 und
- Z¹: -CH₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1415 | CH₃ | CH₃ | | |
| 1416 | CH₃ | C₂H₅ | | |
| 1417 | CH₃ | *n*-C₃H₇ | | |
| 1418 | C₂H₅ | CH₃ | | |
| 1419 | C₂H₅ | C₂H₅ | | |
| 1420 | C₂H₅ | *n*-C₃H₇ | | |
| 1421 | *n*-C₃H₇ | CH₃ | | |
| 1422 | *n*-C₃H₇ | C₂H₅ | | |
| 1423 | *n*-C₃H₇ | *n*-C₃H₇ | | |
| 1424 | *n*-C₃H₇ | *n*-C₅H₁₁ | | |
| 1425 | *n*-C₅H₁₁ | *n*-C₃H₇ | | |
| 1426 | *n*-C₅H₁₁ | *n*-C₅H₁₁ | | |
| 1427 | CH₂=CH | CH₃ | | |
| 1428 | CH₂=CH | C₂H₅ | | |
| 1429 | CH₂=CH | *n*-C₃H₇ | | |
| 1430 | CH₂=CH | CH₂=CH | | |
| 1431 | CH₃ | CH₂=CH | | |
| 1432 | C₂H₅ | CH₂=CH | | |
| 1433 | *n*-C₃H₇ | CH₂=CH | | |
| 1434 | *E*-CH₃-CH=CH | CH₂=CH | | |
| 1435 | *E*-CH₃-CH=CH | *E*-CH₃-CH=CH | | |
| 1436 | CH₃ | CH₃O | | |
| 1437 | CH₃ | C₂H₅O | | |
| 1438 | CH₃ | *n*-C₃H₇O | | |
| 1439 | *n*-C₃H₇ | CH₃O | | |
| 1440 | *n*-C₃H₇ | C₂H₅O | | |
| 1441 | *n*-C₃H₇ | *n*-C₃H₇O | | |
| 1442 | *n*-C₅H₁₁ | *n*-C₄H₉O | | |
| 1443 | *n*-C₄H₉O | *n*-C₅H₁₁ | | |
| 1444 | CH₃O | CH₃O | | |
| 1445 | C₂H₅O | C₂H₅O | | |
| 1446 | *n*-C₃H₇O | *n*-C₃H₇O | | |

### Beispiele 1447 bis 1478

Analog zu Beispiel 241 und Beispiel 361a werden hergestellt: 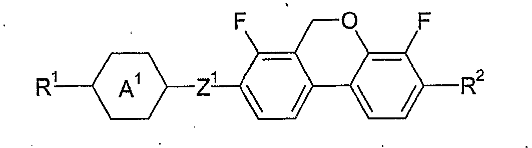 worin 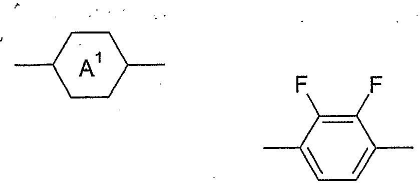 und
- Z¹: -CF₂-O-
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 1447 | CH₃ | CH₃ | | |
| 1448 | CH₃ | C₂H₅ | | |
| 1449 | CH₃ | *n*-C₃H₇ | | |
| 1450 | C₂H₅ | CH₃ | | |
| 1451 | C₂H₅ | C₂H₅ | | |
| 1452 | C₂H₅ | *n*-C₃H₇ | | |
| 1453 | *n*-C₃H₇ | CH₃ | | |
| 1454 | *n*-C₃H₇ | C₂H₅ | | |
| 1455 | *n*-C₃H₇ | *n*-C₃H₇ | | |
| 1456 | *n*-C₃H₇ | *n*-C₅H₁₁ | | |
| 1457 | *n*-C₅H₁₁ | *n*-C₃H₇ | | |
| 1458 | *n*-G₅H₁₁ | *n*-C₅H₁₁ | | |
| 1459 | CH₂=CH | CH₃ | | |
| 1460 | CH₂=CH | C₂H₅ | | |
| 1461 | CH₂=CH | *n*-C₃H₇ | | |
| 1462 | CH₂=CH | CH₂=CH | | |
| 1463 | CH₃ | CH₂=CH | | |
| 1464 | C₂H₅ | CH₂=CH | | |
| 1465 | *n*-C₃H₇ | CH₂=CH | | |
| 1466 | *E*-CH₃-CH=CH | CH₂=CH | | |
| 1467 | *E*-CH₃-CH=CH | *E*-CH₃-CH=CH | | |
| 1468 | CH₃ | CH₃O | | |
| 1469 | CH₃ | C₂H₅O | | |
| 1470 | CH₃ | *n*-C₃H₇O | | |
| 1471 | *n*-C₃H₇ | CH₃O | | |
| 1472 | *n*-C₃H₇ | C₂H₅O | | |
| 1473 | *n*-C₃H₇ | *n*-C₃H₇O | | |
| 1474 | *n*-C₅H₁₁ | *n*-C₄H₉O | | |
| 1475 | *n*-C₄H₉O | *n*-C₅H₁₁ | | |
| 1476 | CH₃O | CH₃O | | |
| 1477 | C₂H₅O | C₂H₅O | | |
| 1478 | *n*-C₃H₇O | *n*-C₃H₇O | | |

### Mischungsbeispiele

Es werden Flüssigkristalline Gemische hergestellt und auf ihre anwendungstechnischen Eigenschaften untersucht.

### Vergleichsbeispiel VM-1

Es wurde eine Flüssigkristallmischung mit der in der folgenden Tabelle angegbenen Zusammensetzung hergestellt und untersucht. Sie hat die ebenfalls in der Tabelle gezeigten Eigenschaften.

| Zusammensetzung | | | Physikalische Eigenschaften | | |
|---|---|---|---|---|---|
| Verbindung | | Konz. | T(N,I) | = | 65,9 °C |
| # | Abkürzung | /Massen-% | | | |
| 1 | PCH-301 | 9,0 | ε⊥(20°C, 1 kHz) | > | 6,1 |
| 2 | PCH-302 | 9,0 | Δε(20°C, 1 kHz) | > | -2,3 |
| 3 | CCH-301 | 29,7 | | | |
| 4 | CCN-47 | 9,9 | | | |
| 5 | CCN-55 | 9,0 | | | |
| 6 | CBC-33F | 4,5 | | | |
| 7 | CBC-53F | 4,5 | | | |
| 8 | CBC-55F | 4,5 | | | |
| 9 | CBC-33 | 4,5 | | | |
| 10 | CBC-53 | 5,4 | | | |
| 11 | BFFO-3-5FF | 10.0 | | | |
| ∑ | | 100,0 | | | |

Das Flüssigkristallmedium hat sehr gute anwendungstechnische Eigenschaften.

### Beispiel M-1

Es wurde eine Flüssigkristallmischung mit der in der folgenden Tabelle angegbenen Zusammensetzung hergestellt und untersucht. Sie hat die ebenfalls in der Tabelle gezeigten Eigenschaften.

| Zusammensetzung | | | Physikalische Eigenschaften | | |
|---|---|---|---|---|---|
| Verbindung | | Konz. | T(N,I) | = | 73,9 °C |
| # | Abkürzung | /Massen-% | | | |
| 1 | PCH-301 | 9,0 | ε_{⊥}(20°C, 1 kHz) | > | 5,4 |
| 2 | PCH-302 | 9,0 | Δε(20°C, 1 kHz) | > | -1,9 |
| 3 | CCH-301 | 29,7 | | | |
| 4 | CCN-47 | 9,9 | | | |
| 5 | CCN-55 | 9,0 | | | |
| 6 | CBC-33F | 4,5 | | | |
| 7 | CBC-53F | 4,5 | | | |
| 8 | CBC-55F | 4,5 | | | |
| 9 | CBC-33 | 4,5 | | | |
| 10 | CBC-53 | 5,4 | | | |
| 11 | BHHO-3O-5FF | 10,0 | | | |
| ∑ | | 100,0 | | | |

Das Flüssigkristallmedium hat sehr gute anwendungstechnische Eigenschaften.

### Beispiel M-2

Es wird die in der folgenden Tabelle gezeigte Mischung hergestellt und untersucht.

| Zusammensetzung | | | Physikalische Eigenschaften | | |
|---|---|---|---|---|---|
| Verbindung # Abkürzung | | Konz. /Massen-% | T(N,I) | = | 70 °C |
| 1 | PCH-304FF | 16,0 | Δn(20°C,589nm) | = | 0,100 |
| 2 | PCH-502FF | 8,0 | Δε(20°C, 1 kHz) | > | -4,5 |
| 3 | PCH-504FF | 14,0 | | | |
| 4 | CCP-302FF | 14,0 | | | |
| 5 | CCP-502FF | 12,0 | | | |
| 6 | CCH-35 | 6,0 | | | |
| 7 | CC-3-V1 | 8,0 | | | |
| 8 | CCP-V-1 | 8,0 | | | |
| 9 | BCH-32 | 8,0 | | | |
| 10 | BFFO-3-5FF | 3,0 | | | |
| 11 | BHHO-20-5FF | 3,0 | | | |
| ∑ | | 100,0 | | | |

Das Flüssigkristallmedium hat hervorragende anwendungstechnische Eigenschaften.

### Beispiel M-3

Es wird die in der folgenden Tabelle gezeigte Mischung hergestellt und untersucht.

| Zusammensetzung | | | Physikalische Eigenschaften | | |
|---|---|---|---|---|---|
| Verbindung | | Konz. | T(N,I) | = | 95,5 °C |
| # | Abkürzung | /Massen-% | | | |
| 1 | PCH-304FF | 7,0 | Δn(20°C,589nm) | = | 0,128 |
| 2 | CCP-402FF | 2,0 | Δε(20°C, 1 kHz) | > | -3,2 |
| 3 | CCP-303FF | 7,0 | k₁(20°C) | = | 16,0 pN |
| 4 | BCH-202FF | 11,0 | k₁/k₃(20°C) | = | 0,94 |
| 5 | BCH-302FF | 11,0 | γ₁(20°C) | = | 154 mPa·s |
| 6 | PYP-2-3 | 10,0 | | | |
| 7 | PYP-2-4 | 10,0 | t_{stor.}(-20°C) | > | 1.000 h |
| 8 | CC-4-V | 10,0 | | | |
| 9 | CC-5-V | 10,0 | V₀(20°C) | = | 2,28 V |
| 10 | CC-3-V1 | 10,0 | | | |
| 11 | CCP-V-1 | 2,0 | | | |
| 12 | CCH-34 | 5,0 | | | |
| 13 | C-BHHO-5-04FF | 3,0 | | | |
| ∑ | | 100,0 | | | |

Das Flüssigkristallmedium hat hervorragende anwendungstechnische Eigenschaften, wie z.B. im Vergleich mit dem folgenden Vergleichsbeispiel (VM-2) zu sehen ist.

### Vergleichsbeispiel VM-2

Es wird die in der folgenden Tabelle gezeigte Mischung, die keine erfindungsgemäße Verbindung enthält, hergestellt und untersucht.

| Zusammensetzung | | | Physikalische Eigenschaften | | |
|---|---|---|---|---|---|
| Verbindung | | Konz. | T(N,I) | = | 96 °C |
| # | Abkürzung | /Massen-% | | | |
| 1 | PCH-304FF | 12,0 | Δn(20°C,589nm) | = | 0,126 |
| 2 | PCH-502FF | 10,0 | Δε(20°C, 1 kHz) | > | -3,1 |
| 3 | BCH-202FF | 12,0 | k₁(20°C) | = | 15,0 pN |
| 4 | BCH-302FF | 13,0 | k₁/k₃(20°C) | = | 1,09 |
| 5 | PYP-2-3 | 8,0 | γ₁(20°C) | = | 169 mPa·s |
| 6 | PYP-2-4 | 8,0 | | | |
| 7 | CC-4-V | 13,0 | t_{stor.}(-20°C) | > | 1.000 h |
| 8 | CC-3-V1 | 9,0 | | | |
| 9 | CCP-V-1 | 10,0 | V₀(20°C) | = | 2,42 V |
| 10 | CCPC-33 | 3,0 | | | |
| 11 | CCPC-34 | 3,0 | | | |
| ∑ | | 100,0 | | | |

Das Flüssigkristallmedium hat ähnliche Werte für den Klärpunkt, die Doppelbrechung und die dielektrische Anisotropie wie das Medium des Beispiels 3. Es hat jedoch eine deutlich höhere Rotationsviskosität und gleichzeitig eine höhere Schwellenspannung und ist dadurch anwendungstechnisch deutlich unterlegen.

### Beispiel M-4

Es wird die in der folgenden Tabelle gezeigte Mischung hergestellt und untersucht.

| Zusammensetzung | | | Physikalische Eigenschaften | | |
|---|---|---|---|---|---|
| Verbindung # Abkürzung | | Konz. /Massen-% | T(N,I) | = | 95,5 °C |
| 1 | PCH-304FF | 5,0 | Δn(20°C,589nm) | = | 0,129 |
| 2 | PCH-502FF | 3,0 | Δε(20°C, 1 kHz) | > | -3,8 |
| 3 | CCP-303FF | 10,0 | k₁(20°C) | = | 15,7 pN |
| 4 | BCH-202FF | 11,0 | k₁/k₃(20°C) | = | 0,97 |
| 5 | BCH-302FF | 11,0 | γ₁(20°C) | = | 173 mPa·s |
| 6 | PYP-2-3 | 6,0 | | | |
| 7 | PYP-2-4 | 14,0 | t_{stor.}(-20°C) | > | 1.000 h |
| 8 | CC-4-V | 15,0 | | | |
| 9 | CC-3-V1 | 12,0 | V₀(20°C) | = | 2,10 V |
| 10 | CCH-34 | 6,0 | | | |
| 11 | C-BHHO-5-04FF | 7.0 | | | |
| ∑ | | 100,0 | | | |

Das Flüssigkristallmedium hat hervorragende anwendungstechnische Eigenschaften, wie z.B. im Vergleich mit dem folgenden Vergleichsbeispiel (VM-2) zu sehen ist.

### Vergleichsbeispiel VM-3

Es wird die in der folgenden Tabelle gezeigte Mischung, die keine erfindungsgemäße Verbindung enthhält, hergestellt und untersucht.

| Zusammensetzung | | | Physikalische Eigenschaften | | |
|---|---|---|---|---|---|
| Verbindung # Abkürzung | | Konz. /Massen-% | T(N,I) | = | 96 °C |
| 1 | PCH-304FF | 9,0 | Δn(20°C,589nm) | = | 0,1127 |
| 2 | PCH-502FF | 5,0 | Δε(20°C, 1 kHz) | > | -3,7 |
| 3 | CCP-302FF | 8,0 | k₁(20°C) | = | 16,3 pN |
| 4 | CCP-402FF | 9,0 | k₁/k₃(20°C) | = | 0,97 |
| 5 | BCH-2-02FF | 12,0 | γ₁(20°C) | = | 179 mPa·s |
| 6 | BCH-3-02FF | 12,0 | | | |
| 7 | PYP-2-3 | 9,0 | t_{stor.}(-20°C) | = | 950 h |
| 8 | PYP-2-4 | 9,0 | | | |
| 9 | CC-4-V | 13,0 | V₀(20°C) | = | 2,19 V |
| 10 | CC-3-V1 | 11,0 | | | |
| 11 | CCH-35 | 2.0 | | | |
| ∑ | | 100,0 | | | |

Das Flüssigkristallmedium hat ähnliche Werte für den Klärpunkt, die Doppelbrechung und die dielektrische Anisotropie wie das Medium des Beispiels 4. Es hat jedoch eine signifikant höhere Rotationsviskosität und gleichzeitig eine höhere Schwellenspannung und ist dadurch anwendungstechnisch weniger gut geeignet.

## Patentansprüche

1. Verbindung der Formel I-2 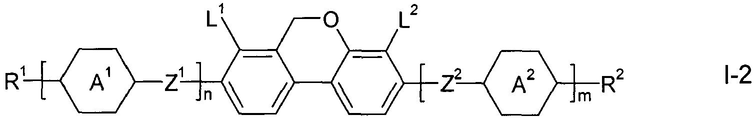 worin
L¹ und L² beide F, 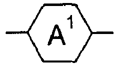
und 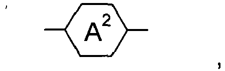 jeweils unabhängig voneinander, und wenn mehrfach vorhanden auch diese unabhängig voneinander,
(a) einen trans-1,4-Cyclohexylenrest, worin auch eine oder zwei nicht benachbarte CH₂-Gruppen durch -O- und/oder -S-ersetzt sein können,
(b) 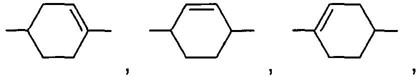
(c) einen 1,4-Phenylenrest, worin auch eine oder zwei nicht benachbarte CH- Gruppen durch N ersetzt sein können, 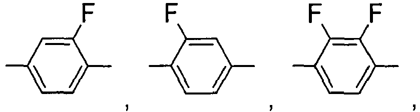 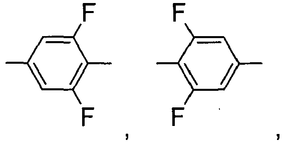 oder
(d) einen Rest ausgewählt aus der Gruppe 1,4-Bicyclo-[2,2,2]-octylen, 1,3-Bicyclo-[1,1,1]-pentylen, Spiro-[3,3]-heptan-2,4-diyl, Piperidin-1,4-diyl, Naphtalin-2,6-diyl, Decahydro-naphthalin-2,6-diyl und 1,2,3,4-Tetrahydronaphthalin-2,6-diyl,
R¹ und R² jeweils unabhängig voneinander, H, Halogen, -CN, -SCN, -SF₅, -CF₃, -CHF₂, -CH₂F, -OCF₃, -OCHF₂, eine einfach durch CN oder CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-, -CF=CH-, -CH=CF-, 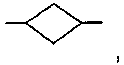 -CO-, -CO-O-, -O-CO- oder -O-CO-O- so ersetzt sein können, dass weder O- noch S-Atome direkt miteinander verknüpft sind, oder
einer von
R¹ und R² Alkyl und Alkoxy mit 1 bis 12 C-Atomen, Alkoxyalkyl, Alkenyl oder Alkenyloxy mit 2 bis 12 C-Atomen und der andere, unabhängig vom ersten, ebenfalls Alkyl und Alkoxy mit 1 bis 12 C-Atomen, Alkoxyalkyl, Alkenyl oder Alkenyloxy mit 2 bis 12 C-Atomen oder auch F, Cl, Br, -CN, -SCN, -SF₅, -CF₃, -CHF₂, -CH₂F, -OCF₃ oder -OCHF₂
Z¹ und Z² jeweils unabhängig voneinander, -CH₂-CH₂-, -CF₂-CF₂-, -CF₂-CH₂-,-CH₂-CF₂-, -CH=CH-, -CF=CF-, -CF=CH-, -CH=CF-, -C≡C-, -COO-, -OCO-, - OCH₂-, -CH₂O-, -CF₂O-, -OCF₂-, eine Kombination von zweien dieser Gruppen, wobei keine zwei O-Atome miteinander verbunden sind, oder eine Einfachbindung und
n und m jeweils 0, 1 oder 2, wobei
n + m 0, 1, 2 oder 3
bedeuten.

2. Verbindung nach Anspruch 1, **dadurch gekennzeichnet, dass** Z¹ und Z² beide eine Einfachbindung bedeuten.

3. Verbindung nach Anspruch 1 oder 2, **dadurch gekennzeichnet, dass** (n + m) 0, 1 oder 2 bedeutet.

4. Verbindung nach Anspruch 3, **dadurch gekennzeichnet, dass** (n + m) 0 oder 1 bedeutet.

5. Verbindung nach Anspruch 4, **dadurch gekennzeichnet, dass** n und m beide 0 bedeuten.

6. Flüssigkristallmedium, **dadurch gekennzeichnet, dass** es eine oder mehrere Verbindungen der Formel I, wie in mindestens einem oder mehreren der Ansprüche 1 bis 5 definiert, enthält.

7. Flüssigkristallmedium nach Anspruch 6, **dadurch gekennzeichnet, dass** es eine nematische Phase aufweist.

8. Flüssigkristallmedium nach mindestens einem der Ansprüche 6 und 7, **dadurch gekennzeichnet, dass** es
eine oder mehrere dielektrisch negative Verbindung(en) der Formel II 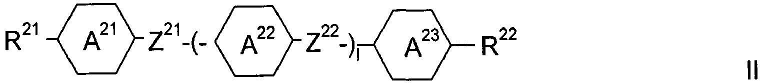 worin
R²¹ und R²² jeweils unabhängig voneinander die in Anspruch 1 für R¹ gegebene Bedeutung haben,
Z²¹ und Z²² jeweils unabhängig voneinander die die in Anspruch 1 für Z¹ gegebene Bedeutung haben,
mindestens einer der vorhandenen Ringe 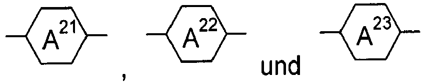 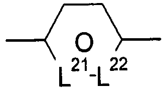 und die anderen, jeweils unabhängig voneinander, 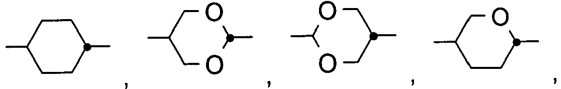 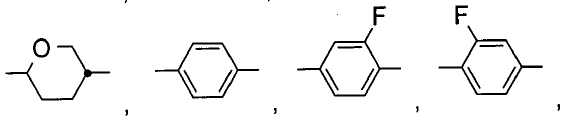 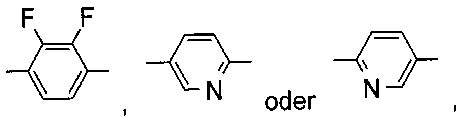
L²¹ und L²¹ beide C-F oder einer von beiden N und der andere C-F und
I 0, 1 oder 2
bedeuten, enthält.

9. Flüssigkristallmedium nach einem oder mehreren der Ansprüche 6 bis 8 **dadurch gekennzeichnet, daß** es eine oder mehrere Verbindung(en) der Formel II-1 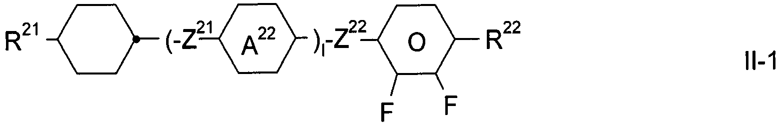 worin R²¹ , R²², Z²¹ , Z²², 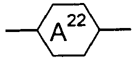 und I die in Anspruch 8 gegebene Bedeutung haben, enthält.

10. Verwendung eines Flüssigkristallmediums nach einem oder mehreren der Ansprüche 6 bis 9 in einer elektrooptischen Anzeige.

11. Elektrooptische Anzeige enthaltend ein Flüssigkristallmedium nach einem oder mehreren der Ansprüche 6 bis 9.

12. Anzeige nach Anspruch 11, **dadurch gekennzeichnet, daß** es sich um ein VAN LCD handelt.

## Claims

1. Compound of the formula I-2 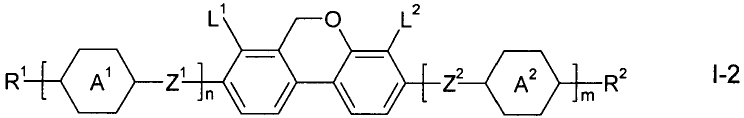 in which
L¹ and L² both denote F, 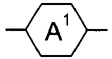
and 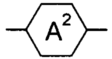 each, independently of one another, and, if present more than once, also independently of one another, denote
(a) a trans-1,4-cyclohexylene radical, in which, in addition, one or two non-adjacent CH₂ groups may be replaced by -O- and/or -S-,
(b) 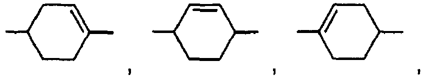
(c) a 1,4-phenylene radical, in which, in addition, one or two non-adjacent CH groups may be replaced by N, 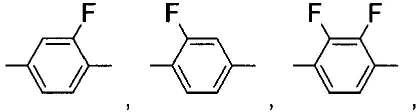 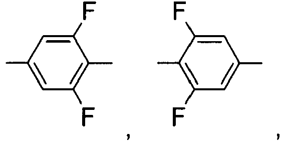 or
(d) a radical selected from the group 1,4-bicyclo[2.2.2]octylene, 1,3-bicyclo[1.1.1]pentylene, spiro[3.3]heptane-2,4-diyl, piperidine-1,4-diyl, naphthalene-2,6-diyl, decahydronaphthalene-2,6-diyl and 1,2,3,4-tetrahydronaphthalene-2,6-diyl,
R¹ and R² each, independently of one another, denote H, halogen, -CN, -SCN, -SF₅, -CF₃, -CHF₂, -CH₂F, -OCF₃, -OCHF₂, an alkyl group having 1 to 15 C atoms which is monosubstituted by CN or CF₃ or at least monosubstituted by halogen, where, in addition, one or more CH₂ groups may each, independently of one another, be replaced by -O-, -S-, -CH=CH-,
-CF=CF-, -CF=CH-, -CH=CF-, 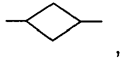 -CO-, -CO-O-, -O-CO- or -O-CO-O- in such a way that neither O nor S atoms are linked directly to one another, or
one of
R¹ and R² denotes alkyl or alkoxy having 1 to 12 C atoms, alkoxyalkyl, alkenyl or alkenyloxy having 2 to 12 C atoms and the other, independently of the first, like-wise denotes alkyl or alkoxy having 1 to 12 C atoms, alkoxyalkyl, alkenyl or alkenyloxy having 2 to 12 C atoms or alternatively F, Cl, Br, -CN, -SCN, -SF₅, -CF₃, -CHF₂, -CH₂F, -OCF₃ or -OCHF₂,
Z¹ and Z² each, independently of one another, denote -CH2-CH2-, -CF₂-CF₂-, -CF2-CH2-, -CH₂-CF₂-, -CH=CH-, -CF=CF-, -CF=CH-, -CH=CF-, -C≡C-, -COO-, -OCO-, -OCH₂-, -CH₂O-, -CF₂O-, -OCF₂-, or a combination of two of these groups, where no two O atoms are bonded to one another, or a single bond and
n and m each denote 0, 1 or 2, where
n + m denotes 0, 1, 2 or 3.

2. Compound according to Claim 1, **characterised in that** Z¹ and Z² both denote a single bond.

3. Compound according to Claim 1 or 2, **characterised in that** (n + m) denotes 0, 1 or 2.

4. Compound according to Claim 3, **characterised in that** (n + m) denotes 0 or 1.

5. Compound according to Claim 4, **characterised in that** n and m both denote 0.

6. Liquid-crystal medium, **characterised in that** it comprises one or more compounds of the formula I as defined in at least one or more of Claims 1 to 5.

7. Liquid-crystal medium according to Claim 6, **characterised in that** it has a nematic phase.

8. Liquid-crystal medium according to at least one of Claims 6 and 7, **characterised in that** it comprises
one or more dielectrically negative compound(s) of the formula II 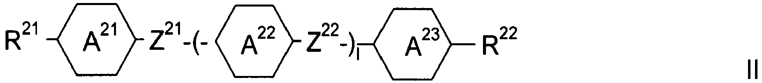 in which
R²¹ and R²² each, independently of one another, have the meaning given for R¹ in Claim 1,
Z²¹ and Z²² each, independently of one another, have the meaning given for Z¹ in Claim 1,
at least one of the rings 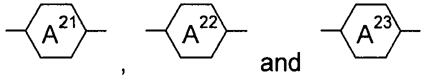 present denotes 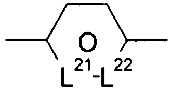 and the others, in each case independently of one another, denote 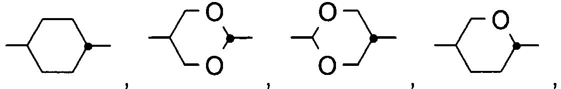 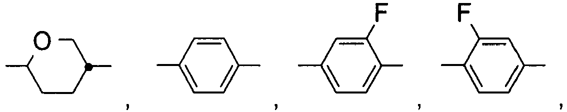 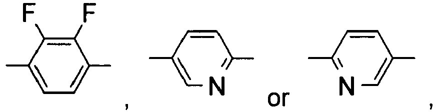
L¹ and L² both denote C-F or one of the two denotes N and the other denotes C-F, and
I denotes 0, 1 or 2.

9. Liquid-crystal medium according to one or more of Claims 6 to 8, **characterised in that** it comprises one or more compound(s) of the formula II-1 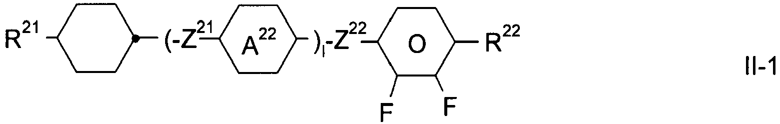 in which R²¹, R²², Z²¹, Z²², 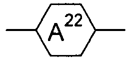 and I have the meaning given in Claim 8.

10. Use of a liquid-crystal medium according to one or more of Claims 6 to 9 in an electro-optical display.

11. Electro-optical display containing a liquid-crystal medium according to one or more of Claims 6 to 9.

12. Display according to Claim 11, **characterised in that** it is a VAN LCD.

## Revendications

1. Composé de la formule I-2 : 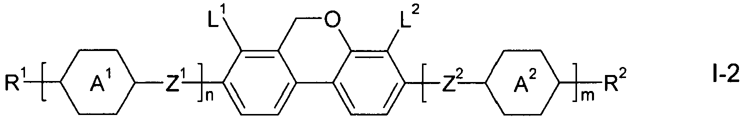 formule dans laquelle :
L¹ et L² représentent tous deux F, 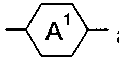
et 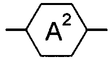 représentent chacun, indépendamment l'un de l'autre, et, s'ils sont présents plus d'une fois, également indépendamment les uns des autres :
(a) un radical trans-1,4-cyclohexylène, où, en outre, un ou deux groupe(s) CH₂ non adjacents peut/peuvent être remplacé(s) par -O- et/ou -S-,
(b) 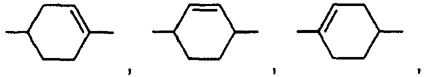
(c) un radical 1,4-phénylène, où, en outre, un ou deux groupe(s) CH non adjacents peut/peuvent être remplacé(s) par N, 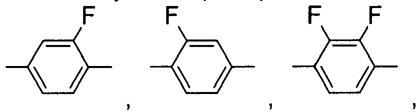 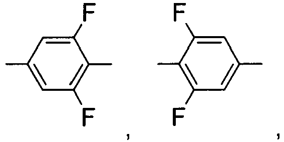 ou
(d) un radical sélectionné parmi le groupe 1,4-bicyclo[2.2.2]-octylène, 1,3-bicyclo[1.1.1]pentylène, spiro[3.3]heptane-2,4-diyle, pipéridine-1,4-diyle, naphtalène-2,6-diyle, décahydro-naphtalène-2,6-diyle et 1,2,3,4-tétrahydronaphtalène-2,6-diyle,
R¹ et R² représentent chacun, indépendamment l'un de l'autre, H, halogène, -CN, -SCN, -SF₅, -CF₃, -CHF₂, -CH₂F, -OCF₃, -OCHF₂, un groupe alkyle qui comporte 1 à 15 atome(s) de C, lequel est monosubstitué par CN ou CF₃ ou au moins monosubstitué par halogène, où, en outre, un ou plusieurs groupe(s) CH₂ peut/peuvent chacun, indépendamment les uns des autres, être remplacé(s) par -O-, -S-, -CH=CH-,
-CF=CF-, -CF=CH-, -CH=CF-, 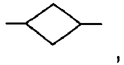 -CO-,
-CO-O-, -O-CO- ou -O-CO-O- de telle sorte que ni des atomes de O, ni des atomes de S ne soient liés directement les uns aux autres, ou
l'un de
R¹ et R² représente alkyle ou alcoxy qui comporte 1 à 12 atome(s) de C, alcoxyalkyle, alkényle ou alkényloxy qui comporte 2 à 12 atomes de C et l'autre, indépendamment du premier, représente pareillement alkyle ou alcoxy qui comporte 1 à 12 atome(s) de C, alcoxyalkyle, alkényle ou alkényloxy qui comporte 2 à 12 atomes de C ou, à titre d'alternative, F, Cl, Br, -CN, -SCN, -SF₅, -CF₃, -CHF₂, -CH₂F, -OCF₃ ou -OCHF₂,
Z¹ et Z² représentent chacun, indépendamment l'un de l'autre, -CH₂-CH₂-, -CF₂-CF₂-, -CF₂-CH₂-, -CH₂-CF₂-, -CH=CH-, -CF=CF-, -CF=CH-, -CH=CF-, -C≡C-, -COO-, -OCO-, -OCH₂-, -CH₂O-, -CF₂O-, -OCF₂-, ou une combinaison de deux de ces groupes, où deux
atomes de O ne sont pas liés l'un à l'autre, ou une liaison simple et
n et m représentent chacun 0, 1 ou 2, où
n + m représente 0, 1, 2 ou 3.

2. Composé selon la revendication 1, **caractérisé en ce que**, Z¹ et Z² représentent tous deux une liaison simple.

3. Composé selon la revendication 1 ou 2, **caractérisé en ce que** (n + m) représente 0, 1 ou 2.

4. Composé selon la revendication 3, **caractérisé en ce que** (n + m) représente 0 ou 1.

5. Composé selon la revendication 4, **caractérisé en ce que** n et m représentent tous deux 0.

6. Milieu cristallin liquide, **caractérisé en ce qu'**il comprend un ou plusieurs composé(s) de la formule I comme défini selon au moins une ou plusieurs des revendications 1 à 5.

7. Milieu cristallin liquide selon la revendication 6, **caractérisé en ce qu'**il présente une phase nématique.

8. Milieu cristallin liquide selon au moins l'une des revendications 6 et 7, **caractérisé en ce qu'**il comprend :
un ou plusieurs composé(s) diélectriquement négatif(s) de la formule II : 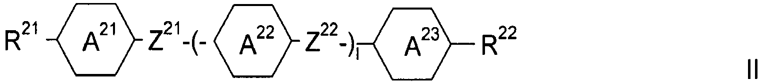 formule dans laquelle :
R²¹ et R²² présentent chacun, indépendamment l'un de l'autre, la signification qui a été donnée pour R¹ selon la revendication 1,
Z²¹ et Z²² présentent chacun, indépendamment l'un de l'autre, la signification qui a été donnée pour Z¹ selon la revendication 1,
au moins l'un des cycles : 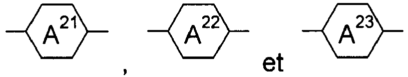 qui sont présents représente 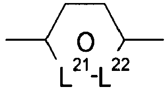 et les autres, dans chaque cas indépendamment les uns des autres, représentent 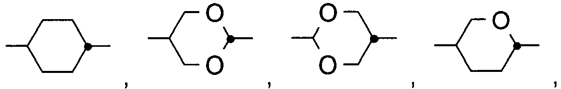 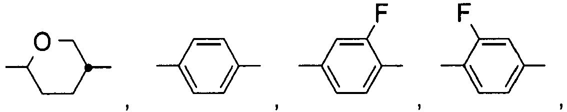 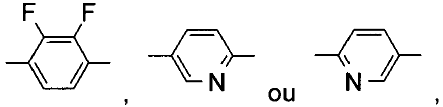
L¹ et L² représentent tous deux C-F ou l'un des deux représente N et l'autre représente C-F, et
I représente 0, 1 ou 2.

9. Milieu cristallin liquide selon une ou plusieurs des revendications 6 à 8, **caractérisé en ce qu'**il comprend un ou plusieurs composé(s) de la formule II-1 : 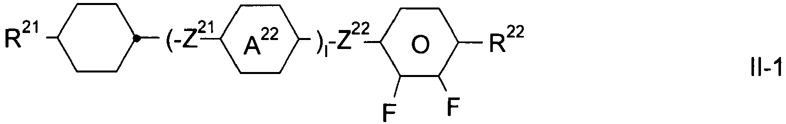 formule dans laquelle R²¹, R²², Z²¹, Z²², 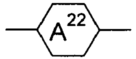 et I présentent la signification qui a été donnée selon la revendication 8.

10. Utilisation d'un milieu cristallin liquide selon une ou plusieurs des revendications 6 à 9 dans un affichage électro-optique.

11. Affichage électro-optique contenant un milieu cristallin liquide selon une ou plusieurs des revendications 6 à 9.

12. Affichage selon la revendication 11, **caractérisé en ce qu'**il s'agit d'un affichage à cristaux liquides/LCD VAN.
